# EUROPEAN PATENT APPLICATION

(11) **EP 1 439 192 A1**
(43) Date of publication of application: **21.07.2004**
(21) Application number: 03000615.9
(22) Date of filing: 15.01.2003
(51) Int. Cl.: C07K 16/30, C07K 16/28, C12N 15/11, C12N 15/13, A61K 51/10

(54) **Neuropilin-1 inhibitors**

(71) Applicant: Xerion Pharmaceuticals AG, 81377 München (DE); Tufts University, Boston, MA 02111 (US)
(72) Inventor: Unger, Christine M., 80469 Munich (DE); Beste, Gerald, 80687 Munich (DE); Zehetmeier, Carolin, 81541 Munich (DE); Lain, Blanca, 82061 Neuried (DE); Torella, Claudia, 82061 Neuried (DE); Niewöhner, Jens, 81477 Munich (DE); Jay, Daniel G., Brighton, MA 02111 (US); Eustace, Brenda K., Brookline, MA 02446 (US)
(74) Representative: Fleuchaus, Andrea, Dr.

(57) **Abstract**

The invention relates to the use of molecules interfering with the function of neuropilin-1 in tumor cells for the treatment of specific cancers. Polypeptides, compositions and methods are provided that are useful for reducing or inhibiting the invasiveness and/or the metastatic potential of specific tumor cells. Furthermore, a method is provided that allows to determine whether a naturally occurring tumor cell depends on functional neuropilin-1 for its invasiveness and/or metastatic potential.

SEQUENCE LISTING

<110>  Xerion Pharmaceuticals AG

<120>  Neuropilin-1 Inhibitors

<130>  X38922EP

<140>  EP 03000615.9
<141>  2003-01-15

<160>  10

<170>  PatentIn version 3.1

<210>  1
<211>  269
<212>  PRT
<213>  artificial sequence

<220>
<223>  scFv1 polypeptide

<400>  1

Glu Val Gln Leu Gln Gln Ser Gly Pro Glu Leu Val Lys Pro Gly Ala
1               5                   10                  15

Leu Val Lys Ile Ser Cys Lys Ala Ser Gly Tyr Thr Val Thr Ser Tyr
            20                  25                  30

Asp Ile Asn Trp Val Lys Gln Arg Pro Gly Gln Gly Leu Glu Trp Ile
        35                  40                  45

Gly Trp Ile Tyr Pro Gly Asp Gly Ser Thr Lys Tyr Asn Glu Lys Phe
    50                  55                  60

Lys Gly Lys Ala Thr Leu Thr Val Asp Lys Ser Ser Thr Thr Val Tyr
65                  70                  75                  80

Met Gln Leu Ser Ser Leu Thr Ser Glu Asn Ser Ala Val Tyr Phe Cys
                85                  90                  95

Ala Arg Gly Gly Lys Tyr Phe Asp Tyr Trp Gly Gln Gly Thr Thr Leu
            100                 105                 110

Thr Val Ser Thr Gly Gly Gly Gly Ser Gly Gly Gly Gly Ser Gly Gly
        115                 120                 125

Gly Gly Ser Ala Leu Asp Ile Val Met Thr Gln Ser Pro Lys Phe Met
    130                 135                 140

Ser Thr Ser Val Gly Asp Arg Val Ser Val Thr Cys Lys Ala Ser Gln
145                 150                 155                 160

Asn Val Ala Thr Asn Val Ala Trp Tyr Gln Gln Lys Pro Gly Gln Ser
                165                 170                 175

Pro Lys Pro Leu Thr Tyr Ser Ala Ser Phe Arg Ser Ser Gly Val Pro
            180                 185                 190

Asp Arg Phe Thr Gly Ser Gly Ser Gly Thr Asp Phe Thr Leu Thr Ile
        195                 200                 205

Ser Asn Val Gln Ser Glu Asp Leu Ala Glu Tyr Phe Cys Gln Gln Tyr
    210                 215                 220

Asn Ser Tyr Pro Tyr Thr Phe Gly Gly Gly Thr Lys Leu Glu Ile Lys
225                 230                 235                 240

Ala Ala Ala Gly Ala Pro Val Pro Tyr Pro Asp Pro Leu Glu Pro Arg
                245                 250                 255

Gly Ala Ala Ser Ala Trp Ser His Pro Gln Phe Glu Lys
            260                 265

<210>  2
<211>  288
<212>  PRT
<213>  artificial sequence

<220>
<223>  scFv2 polypeptide

<400>  2

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Ser Gly Leu Gln Gln Gly Pro Arg Arg Arg Gly Ala Arg
            100                 105                 110

Val Asn Phe Ser Tyr Tyr Gly Leu Asp Val Trp Gly Arg Gly Thr Thr
        115                 120                 125

Val Thr Val Ser Ser Gly Gly Gly Gly Ser Gly Gly Gly Gly Ser Gly
    130                 135                 140

Gly Gly Gly Ser Ala Gln Ala Val Leu Thr Gln Pro Ser Ser Ala Ser
145                 150                 155                 160

Gly Thr Pro Gly Gln Arg Val Thr Ile Ser Cys Ser Gly Ser Asn Ser
                165                 170                 175

Asn Ile Gly Arg Asn Tyr Val Phe Trp Tyr Gln Gln Phe Pro Gly Thr
            180                 185                 190

Ala Pro Lys Ile Leu Ile Tyr Arg Asn Asn Gln Arg Pro Ser Gly Val
        195                 200                 205

Pro Asp Arg Phe Ser Gly Ser Lys Ser Gly Thr Ser Ala Ser Leu Ala
    210                 215                 220

Ile Ser Gly Leu Arg Ser Glu Asp Glu Ala Asp Tyr Tyr Cys Ala Ser
225                 230                 235                 240

Trp Asp Asp Ser Leu Thr Trp Val Phe Gly Gly Gly Thr Lys Val Thr
                245                 250                 255

Val Leu Gly Ala Ala Ala Gly Ala Pro Val Pro Tyr Pro Asp Pro Leu
            260                 265                 270

Glu Pro Arg Gly Ala Ala Ser Ala Trp Ser His Pro Gln Phe Glu Lys
        275                 280                 285

<210>  3
<211>  810
<212>  DNA
<213>  artificial sequence

<220>
<223>  DNA coding for the polypeptide scFv 1

<400>  3
gaggtccagc tgcaacagtc tggacctgag ctggtgaagc ctggggcttt agtgaagata      60

tcctgcaagg cctcgggata caccgtcaca agctacgata taaactgggt gaagcagagg     120

cctggacagg gacttgagtg gattggatgg atttatcctg gagatggtag tactaagtac     180

aatgagaaat tcaagggcaa ggccacactg actgtagaca aatcctccac cacagtctac 240

atgcagctca gcagcctgac ttctgagaac tctgcagtct atttctgtgc aagaggtggt 300

aaatactttg actactgggg ccaaggcacc actctcacag tgtcgacagg tggaggcggt 360

tcaggcggag gtggctctgg cggtggcgga agtgcactcg acattgtgat gacacagtct 420

ccaaaattca tgtccacatc agtaggagac agggtcagcg tcacctgcaa ggccagtcag 480

aatgtggcta ctaatgtagc ctggtatcaa cagaaaccag ggcaatctcc taaaccactg 540

acttactcgg catccttccg gtccagtgga gtccctgatc gcttcacagg cagtggatct 600

gggacagatt tcactctcac catcagcaat gtgcagtctg aagacttggc agagtatttc 660

tgtcagcaat ataacagcta tccgtacacg ttcggagggg ggaccaagct ggaaataaaa 720

gcggccgcag gtgcgccggt gccgtatcca gatccgctgg aaccgcgtgg ggccgcaagc 780

gcttggagcc acccgcagtt cgaaaaataa 810

<210> 4
<211> 867
<212> DNA
<213> artificial sequence

<220>
<223> DNA coding for the polypeptide scFv 2

<400> 4
gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcaaatga acagcctgag agccgaggac acggccgtgt attactgtgc gcgagactcg 300

gggctacagc agggaccccg ccgaagaggg gcccgagtaa atttctccta ctacggtctg 360

gacgtctggg ggcgggggac cacggtcacc gtctcgagtg gaggcggcgg ttcaggcgga 420

ggtggctctg gcggtggcgg aagtgcacag gctgtgctga ctcagccgtc ctcagcgtct 480

gggacccccg ggcagagggt caccatctct tgttctggaa gcaactccaa catcggacgc 540

aattatgtat tctggtacca gcagttccca ggaacggccc ccaaaatcct catctacagg 600

aacaatcagc ggccctcagg ggtccctgac cgattctctg gctccaagtc tggcacatca 660

gcctccctgg ccatcagtgg gctccggtcc gaggatgagg ctgattatta ctgtgcatca 720

tgggatgaca gcctgacttg ggtgttcggc ggagggacca aggtcaccgt cctaggtgcg 780

gccgcaggtg cgccggtgcc gtatccagat ccgctggaac cgcgtggggc cgcaagcgct 840

tggagccacc cgcagttcga aaaataa 867

<210> 5
<211> 21
<212> DNA
<213> artificial sequence

<220>
<223> oligonucleotide for RNAi, used together with SEQ ID NO. 6

<400> 5
cagcaccata caatcagagt t 21

<210> 6
<211> 21
<212> DNA
<213> artificial sequence

<220>
<223> oligonucleotide for RNAi, used together with SEQ ID NO. 5

<400> 6
ctctgattgt atggtgctgt t 21

<210> 7
<211> 21
<212> DNA
<213> artificial sequence

<220>
<223> oligonucleotide for RNAi, used together with SEQ ID NO. 8

<400> 7
tgggatgtca gaaagaaact t 21

<210> 8
<211> 21
<212> DNA
<213> artificial sequence

<220>
<223> oligonucleotide for RNAi, used together with SEQ ID NO. 7

<400> 8
gtttctttct gacatcccat t 21

<210> 9
<211> 7836
<212> DNA
<213> artificial sequence

<220>
<223> sequence of the scFv display vector pXP10

<400> 9
   gacgaaaggg cctcgtgata cgcctatttt tataggttaa tgtcatgata ctgctttccc 60
   ggagcactat gcggataaaa atatccaatt acagtactat ataatggttt cttagacgtc 120
   aggtggcact tttcggggaa atgtgcgcgg tattaccaaa gaatctgcag tccaccgtga 180
   aaagcccctt tacacgcgcc aacccctatt tgtttatttt tctaaataca ttcaaatatg 240
   tatccgctca ttggggataa acaaataaaa agatttatgt aagtttatac ataggcgagt 300
   tgagacaata accctgataa atgcttcaat aatattgaaa aaggaagagt actctgttat 360
   tgggactatt tacgaagtta ttataacttt ttccttctca atgagtattc aacatttccg 420
   tgtcgccctt attccctttt ttgcggcatt tactcataag ttgtaaaggc acagcgggaa 480
   taagggaaaa aacgccgtaa ttgccttcct gtttttgctc acccagaaac gctggtgaaa 540
   gtaaaagatg aacggaagga caaaaacgag tgggtctttg cgaccacttt cattttctac 600
   ctgaagatca gttgggtgct cgagtgggtt acatcgaact ggatctcaac gacttctagt 660
   caacccacga gctcacccaa tgtagcttga cctagagttg agcggtaaga tccttgagag 720
   ttttcgcccc gaagaacgtt ttccaatgat tcgccattct aggaactctc aaaagcgggg 780
   cttcttgcaa aaggttacta gagcactttt aaagttctgc tatgtggcgc ggtattatcc 840
   cgtattgacg ctcgtgaaaa tttcaagacg atacaccgcg ccataatagg gcataactgc 900
   ccgggcaaga gcaactcggt cgccgcatac actattctca gaatgacttg ggcccgttct 960
   cgttgagcca gcggcgtatg tgataagagt cttactgaac gttgagtact caccagtcac 1020
   agaaaagcat cttacggatg gcatgacagt caactcatga gtggtcagtg tcttttcgta 1080
   gaatgcctac cgtactgtca aagagaatta tgcagtgctg ccataaccat gagtgataac 1140
   actgcggcca ttctcttaat acgtcacgac ggtattggta ctcactattg tgacgccggt 1200
   acttacttct gacaacgatc ggaggaccga aggagctaac cgcttttttg tgaatgaaga 1260
   ctgttgctag cctcctggct tcctcgattg gcgaaaaaac cacaacatgg gggatcatgt 1320
   aactcgcctt gatcgttggg aaccggagct gtgttgtacc ccctagtaca ttgagcggaa 1380
   ctagcaaccc ttggcctcga gaatgaagcc ataccaaacg acgagcgtga caccacgatg 1440
   cctgtagcaa cttacttcgg tatggtttgc tgctcgcact gtggtgctac ggacatcgtt 1500
   tggcaacaac gttgcgcaaa ctattaactg gcgaactact tactctagct accgttgttg 1560
   caacgcgttt gataattgac cgcttgatga atgagatcga tcccggcaac aattaataga 1620
   ctggatggag gcggataaag ttgcaggacc agggccgttg ttaattatct gacctacctc 1680
   cgcctatttc aacgtcctgg acttctgcgc tcggcccttc cggctggctg gtttattgct 1740
   gataaatctg tgaagacgcg agccgggaag gccgaccgac caaataacga ctatttagac 1800
   gagccggtga gcgtgggtct cgcggtatca ttgcagcact ggggccagat ctcggccact 1860
   cgcacccaga gcgccatagt aacgtcgtga ccccggtcta ggtaagccct cccgtatcgt 1920
   agttatctac acgacgggga gtcaggcaac ccattcggga gggcatagca tcaatagatg 1980
   tgctgcccct cagtccgttg tatggatgaa cgaaatagac agatcgctga gataggtgcc 2040
   tcactgatta atacctactt gctttatctg tctagcgact ctatccacgg agtgactaat 2100
   agcattggta actgtcagac caagtttact catatatact ttagattgat tcgtaaccat 2160
   tgacagtctg gttcaaatga gtatatatga aatctaacta ttaaaacttc atttttaatt 2220
   taaaaggatc taggtgaaga tcctttttga aattttgaag taaaaattaa attttcctag 2280
   atccacttct aggaaaaact taatctcatg accaaaatcc cttaacgtga gttttcgttc 2340
   cactgagcgt attagagtac tggttttagg gaattgcact caaaagcaag gtgactcgca 2400
   cagaccccgt agaaaagatc aaaggatctt cttgagatcc tttttttctg gtctggggca 2460
   tcttttctag tttcctagaa gaactctagg aaaaaaagac cgcgtaatct gctgcttgca 2520
   aacaaaaaaa ccaccgctac cagcggtggt gcgcattaga cgacgaacgt ttgttttttt 2580
   ggtggcgatg gtcgccacca ttgtttgccg gatcaagagc taccaactct ttttccgaag 2640
   gtaactggct aacaaacggc ctagttctcg atggttgaga aaaaggcttc cattgaccga 2700
   tcagcagagc gcagatacca aatactgtcc ttctagtgta gccgtagtta agtcgtctcg 2760
   cgtctatggt ttatgacagg aagatcacat cggcatcaat ggccaccact tcaagaactc 2820
   tgtagcaccg cctacatacc tcgctctgct ccggtggtga agttcttgag acatcgtggc 2880
   ggatgtatgg agcgagacga aatcctgtta ccagtggctg ctgccagtgg cgataagtcg 2940
   tgtcttaccg ttaggacaat ggtcaccgac gacggtcacc gctattcagc acagaatggc 3000
   ggttggactc aagacgatag ttaccggata aggcgcagcg gtcgggctga ccaacctgag 3060
   ttctgctatc aatggcctat tccgcgtcgc cagcccgact acggggggtt cgtgcataca 3120
   gcccagcttg gagcgaacga cctacaccga tgccccccaa gcacgtatgt cgggtcgaac 3180
   ctcgcttgct ggatgtggct actgagatac ctacagcgtg agctatgaga aagcgccacg 3240
   cttcccgaag tgactctatg gatgtcgcac tcgatactct ttcgcggtgc gaagggcttc 3300
   ggagaaaggc ggacaggtat ccggtaagcg gcagggtcgg aacaggagag cctctttccg 3360
   cctgtccata ggccattcgc cgtcccagcc ttgtcctctc cgcacgaggg agcttccagg 3420
   gggaaacgcc tggtatcttt atagtcctgt gcgtgctccc tcgaaggtcc ccctttgcgg 3480
   accatagaaa tatcaggaca cgggtttcgc cacctctgac ttgagcgtcg atttttgtga 3540
   tgctcgtcag gcccaaagcg gtggagactg aactcgcagc taaaaacact acgagcagtc 3600
   gggggcggag cctatggaaa aacgccagca acgcggcctt tttacggttc cccccgcctc 3660
   ggataccttt ttgcggtcgt tgcgccggaa aaatgccaag ctggcctttt gctggccttt 3720
   tgctcacatg ttctttcctg cgttatcccc gaccggaaaa cgaccggaaa acgagtgtac 3780
   aagaaaggac gcaatagggg tgattctgtg gataaccgta ttaccgcctt tgagtgagct 3840
   gataccgctc actaagacac ctattggcat aatggcggaa actcactcga ctatggcgag 3900
   gccgcagccg aacgaccgag cgcagcgagt cagtgagcga ggaagcggaa cggcgtcggc 3960
   ttgctggctc gcgtcgctca gtcactcgct ccttcgcctt gagcgcccaa tacgcaaacc 4020
   gcctctcccc gcgcgttggc cgattcatta ctcgcgggtt atgcgtttgg cggagagggg 4080
   cgcgcaaccg gctaagtaat atgcagctgg cacgacaggt ttcccgactg gaaagcgggc 4140
   agtgagcgca tacgtcgacc gtgctgtcca aagggctgac ctttcgcccg tcactcgcgt 4200
   acgcaattaa tgtgagttag ctcactcatt aggcacccca ggctttacac tgcgttaatt 4260
   acactcaatc gagtgagtaa tccgtggggt ccgaaatgtg tttatgcttc cggctcgtat 4320
   gttgtgtgga attgtgagcg gataacaatt aaatacgaag gccgagcata caacacacct 4380
   taacactcgc ctattgttaa tcacacagga aacagctatg accatgatta cgccaagctt 4440
   tggagccttt agtgtgtcct ttgtcgatac tggtactaat gcggttcgaa acctcggaaa 4500
   tttttggaga ttttcaacgt gaaaaaatta ttattcgcaa ttcctttagt aaaaacctct 4560
   aaaagttgca cttttttaat aataagcgtt aaggaaatca tgttcctttc tatgcggccc 4620
   agccggccat ggcccaggtc cagtcgacag acaaggaaag atacgccggg tcggccggta 4680
   ccgggtccag gtcagctgtc gtggaggcgg ttcaggcgga ggtggctctg gcggtggcgg 4740
   aagtgcactc cacctccgcc aagtccgcct ccaccgagac cgccaccgcc ttcacgtgag 4800
   atcaaacggc ggccgcaggt gcgccggtgc cgtatccgga tccgctggaa tagtttgccg 4860
   ccggcgtcca cgcggccacg gcataggcct aggcgacctt ccgcgtgccg cataggctgg 4920
   cggcggctct ggtggtggtt ctggtggcgg ggcgcacggc gtatccgacc gccgccgaga 4980
   ccaccaccaa gaccaccgcc ctctgagggt ggcggctctg agggtggcgg ttctgagggt 5040
   ggcggctctg gagactccca ccgccgagac tcccaccgcc aagactccca ccgccgagac 5100
   agggtggcgg ttccggtggc ggctccggtt ccggtgattt tgattatgaa tcccaccgcc 5160
   aaggccaccg ccgaggccaa ggccactaaa actaatactt aaaatggcaa acgctaataa 5220
   gggggctatg accgaaaatg ccgatgaaaa ttttaccgtt tgcgattatt cccccgatac 5280
   tggcttttac ggctactttt cgcgctacag tctgacgcta aaggcaaact tgattctgtc 5340
   gctactgatt gcgcgatgtc agactgcgat ttccgtttga actaagacag cgatgactaa 5400
   acggtgctgc tatcgatggt ttcattggtg acgtttccgg ccttgctaat tgccacgacg 5460
   atagctacca aagtaaccac tgcaaaggcc ggaacgatta ggtaatggtg ctactggtga 5520
   ttttgctggc tctaattccc aaatggctca ccattaccac gatgaccact aaaacgaccg 5580
   agattaaggg tttaccgagt agtcggtgac ggtgataatt cacctttaat gaataatttc 5640
   cgtcaatatt tcagccactg ccactattaa gtggaaatta cttattaaag gcagttataa 5700
   taccttcttt gcctcagtcg gttgaatgtc gcccttatgt ctttggcgct atggaagaaa 5760
   cggagtcagc caacttacag cgggaataca gaaaccgcga ggtaaaccat atgaattttc 5820
   tattgattgt gacaaaataa acttattccg ccatttggta tacttaaaag ataactaaca 5880
   ctgttttatt tgaataaggc tggtgtcttt gcgtttcttt tatatgttgc cacctttatg 5940
   tatgtatttt accacagaaa cgcaaagaaa atatacaacg gtggaaatac atacataaaa 6000
   cgacgtttgc taacatactg cgtaataagg agtcttaata agaattcact gctgcaaacg 6060
   attgtatgac gcattattcc tcagaattat tcttaagtga ggccgtcgtt ttacaacgtc 6120
   gtgactggga aaaccctggc gttacccaac ccggcagcaa aatgttgcag cactgaccct 6180
   tttgggaccg caatgggttg ttaatcgcct tgcagcacat ccccctttcg ccagctggcg 6240
   taatagcgaa aattagcgga acgtcgtgta gggggaaagc ggtcgaccgc attatcgctt 6300
   gaggcccgca ccgatcgccc ttcccaacag ttgcgcagcc tgaatggcga ctccgggcgt 6360
   ggctagcggg aagggttgtc aacgcgtcgg acttaccgct atggcgcctg atgcggtatt 6420
   ttctccttac gcatctgtgc ggtatttcac taccgcggac tacgccataa aagaggaatg 6480
   cgtagacacg ccataaagtg accgcatacg tcaaagcaac catagtacgc gccctgtagc 6540
   ggcgcattaa tggcgtatgc agtttcgttg gtatcatgcg cgggacatcg ccgcgtaatt 6600
   gcccggcggg tgtggtggtt acgcgcagcg tgaccgctac acttgccagc cgggccgccc 6660
   acaccaccaa tgcgcgtcgc actggcgatg tgaacggtcg gccctagccc ccgctccttt 6720
   cgctttcttc ccttcctttc tcgccacgtt cgggatcggg ggcgaggaaa gcgaaagaag 6780
   ggaaggaaag agcggtgcaa cgccggcttt ccccgtcaag ctctaaatcg ggggctccct 6840
   ttagggttcc gcggccgaaa ggggcagttc gagatttagc cccgagggga aatcccaagg 6900
   gatttagtgc tttacggcac ctcgacccca aaaaacttga tttgggtgat ctaaatcacg 6960
   aaatgccgtg gagctggggt tttttgaact aaacccacta ggttcacgta gtgggccatc 7020
   gccctgatag acggtttttc gtcctttgac ccaagtgcat cacccggtag cgggactatc 7080
   tgccaaaaag caggaaactg gttcgagtcc acgttcttta atagtggact cttgttccaa 7140
   actggaacaa caagctcagg tgcaagaaat tatcacctga gaacaaggtt tgaccttgtt 7200
   tactcaaccc tatctcgggc tattcttttg atttataagg gattttgccg atgagttggg 7260
   atagagcccg ataagaaaac taaatattcc ctaaaacggc atttcggcct attggttaaa 7320
   aaatgagctg atttaacaaa aatttaacgc taaagccgga taaccaattt tttactcgac 7380
   taaattgttt ttaaattgcg gaattttaac aaaatattaa cgtttacaat tttatggtgc 7440
   agtctcagta cttaaaattg ttttataatt gcaaatgtta aaataccacg tcagagtcat 7500
   caatctgctc tgatgccgca tagttaagcc agccccgaca cccgccaaca gttagacgag 7560
   actacggcgt atcaattcgg tcggggctgt gggcggttgt cccgctgacg cgccctgacg 7620
   ggcttgtctg ctcccggcat ccgcttacag gggcgactgc gcgggactgc ccgaacagac 7680
   gagggccgta ggcgaatgtc acaagctgtg accgtctccg ggagctgcat gtgtcagagg 7740
   ttttcaccgt tgttcgacac tggcagaggc cctcgacgta cacagtctcc aaaagtggca 7800
   catcaccgaa acgcgcgagt agtggctttg cgcgct 7836

<210> 10
<211> 6702
<212> DNA
<213> artificial sequence

<220>
<223> sequence of the scFv expression vector pXP14

<400> 10
gacgaaaggg cctcgtgata cgcctatttt tataggttaa tgtcatgata ctgctttccc 60

ggagcactat gcggataaaa atatccaatt acagtactat ataatggttt cttagacgtc 120

aggtggcact tttcggggaa atgtgcgcgg tattaccaaa gaatctgcag tccaccgtga 180

aaagcccctt tacacgcgcc aacccctatt tgtttatttt tctaaataca ttcaaatatg 240

tatccgctca ttggggataa acaaataaaa agatttatgt aagtttatac ataggcgagt 300

tgagacaata accctgataa atgcttcaat aatattgaaa aaggaagagt actctgttat 360

tgggactatt tacgaagtta ttataacttt ttccttctca atgagtattc aacatttccg 420

tgtcgccctt attccctttt ttgcggcatt tactcataag ttgtaaaggc acagcgggaa 480

taagggaaaa aacgccgtaa ttgccttcct gtttttgctc acccagaaac gctggtgaaa 540

gtaaaagatg aacggaagga caaaaacgag tgggtctttg cgaccacttt cattttctac 600

ctgaagatca gttgggtgct cgagtgggtt acatcgaact ggatctcaac gacttctagt 660

caacccacga gctcacccaa tgtagcttga cctagagttg agcggtaaga tccttgagag 720

ttttcgcccc gaagaacgtt ttccaatgat tcgccattct aggaactctc aaaagcgggg 780

cttcttgcaa aaggttacta gagcactttt aaagttctgc tatgtggcgc ggtattatcc 840

cgtattgacg ctcgtgaaaa tttcaagacg atacaccgcg ccataatagg gcataactgc 900

ccgggcaaga gcaactcggt cgccgcatac actattctca gaatgacttg ggcccgttct 960

cgttgagcca gcggcgtatg tgataagagt cttactgaac gttgagtact caccagtcac 1020

agaaaagcat cttacggatg gcatgacagt caactcatga gtggtcagtg tcttttcgta 1080

gaatgcctac cgtactgtca aagagaatta tgcagtgctg ccataaccat gagtgataac 1140

actgcggcca ttctcttaat acgtcacgac ggtattggta ctcactattg tgacgccggt 1200

acttacttct gacaacgatc ggaggaccga aggagctaac cgcttttttg tgaatgaaga 1260

ctgttgctag cctcctggct tcctcgattg gcgaaaaaac cacaacatgg gggatcatgt 1320

aactcgcctt gatcgttggg aaccggagct gtgttgtacc ccctagtaca ttgagcggaa 1380

ctagcaaccc ttggcctcga gaatgaagcc ataccaaacg acgagcgtga caccacgatg 1440

cctgtagcaa cttacttcgg tatggtttgc tgctcgcact gtggtgctac ggacatcgtt 1500

tggcaacaac gttgcgcaaa ctattaactg gcgaactact tactctagct accgttgttg 1560

caacgcgttt gataattgac cgcttgatga atgagatcga tcccggcaac aattaataga 1620

ctggatggag gcggataaag ttgcaggacc agggccgttg ttaattatct gacctacctc 1680

cgcctatttc aacgtcctgg acttctgcgc tcggcccttc cggctggctg gtttattgct 1740

gataaatctg tgaagacgcg agccgggaag gccgaccgac caaataacga ctatttagac 1800

gagccggtga gcgtgggtct cgcggtatca ttgcagcact ggggccagat ctcggccact 1860

cgcacccaga gcgccatagt aacgtcgtga ccccggtcta ggtaagccct cccgtatcgt 1920

agttatctac acgacgggga gtcaggcaac ccattcggga gggcatagca tcaatagatg 1980

tgctgcccct cagtccgttg tatggatgaa cgaaatagac agatcgctga gataggtgcc 2040

tcactgatta atacctactt gctttatctg tctagcgact ctatccacgg agtgactaat 2100

agcattggta actgtcagac caagtttact catatatact ttagattgat tcgtaaccat 2160

tgacagtctg gttcaaatga gtatatatga aatctaacta ttaaaacttc atttttaatt 2220

taaaaggatc taggtgaaga tcctttttga aattttgaag taaaaattaa attttcctag 2280

atccacttct aggaaaaact taatctcatg accaaaatcc cttaacgtga gttttcgttc 2340

cactgagcgt attagagtac tggttttagg gaattgcact caaaagcaag gtgactcgca 2400

cagaccccgt agaaaagatc aaaggatctt cttgagatcc tttttttctg gtctggggca 2460

tcttttctag tttcctagaa gaactctagg aaaaaaagac cgcgtaatct gctgcttgca 2520

aacaaaaaaa ccaccgctac cagcggtggt gcgcattaga cgacgaacgt ttgttttttt 2580

ggtggcgatg gtcgccacca ttgtttgccg gatcaagagc taccaactct ttttccgaag 2640

gtaactggct aacaaacggc ctagttctcg atggttgaga aaaaggcttc cattgaccga 2700

tcagcagagc gcagatacca aatactgtcc ttctagtgta gccgtagtta agtcgtctcg 2760

cgtctatggt ttatgacagg aagatcacat cggcatcaat ggccaccact tcaagaactc 2820

tgtagcaccg cctacatacc tcgctctgct ccggtggtga agttcttgag acatcgtggc 2880

ggatgtatgg agcgagacga aatcctgtta ccagtggctg ctgccagtgg cgataagtcg 2940

tgtcttaccg ttaggacaat ggtcaccgac gacggtcacc gctattcagc acagaatggc 3000

ggttggactc aagacgatag ttaccggata aggcgcagcg gtcgggctga ccaacctgag 3060

ttctgctatc aatggcctat tccgcgtcgc cagcccgact acggggggtt cgtgcataca 3120

gcccagcttg gagcgaacga cctacaccga tgccccccaa gcacgtatgt cgggtcgaac 3180

ctcgcttgct ggatgtggct actgagatac ctacagcgtg agctatgaga aagcgccacg 3240

cttcccgaag tgactctatg gatgtcgcac tcgatactct ttcgcggtgc gaagggcttc 3300

ggagaaaggc ggacaggtat ccggtaagcg gcagggtcgg aacaggagag cctctttccg 3360

cctgtccata ggccattcgc cgtcccagcc ttgtcctctc cgcacgaggg agcttccagg 3420

gggaaacgcc tggtatcttt atagtcctgt gcgtgctccc tcgaaggtcc ccctttgcgg 3480

accatagaaa tatcaggaca cgggtttcgc cacctctgac ttgagcgtcg atttttgtga 3540

tgctcgtcag gcccaaagcg gtggagactg aactcgcagc taaaaacact acgagcagtc 3600

gggggcggag cctatggaaa aacgccagca acgcggcctt tttacggttc cccccgcctc 3660

ggataccttt ttgcggtcgt tgcgccggaa aaatgccaag ctggcctttt gctggccttt 3720

tgctcacatg ttctttcctg cgttatcccc gaccggaaaa cgaccggaaa acgagtgtac 3780

aagaaaggac gcaatagggg tgattctgtg gataaccgta ttaccgcctt tgagtgagct 3840

gataccgctc actaagacac ctattggcat aatggcggaa actcactcga ctatggcgag 3900

gccgcagccg aacgaccgag cgcagcgagt cagtgagcga ggaagcggaa cggcgtcggc 3960

ttgctggctc gcgtcgctca gtcactcgct ccttcgcctt gagcgcccaa tacgcaaacc 4020

gcctctcccc gcgcgttggc cgattcatta ctcgcgggtt atgcgtttgg cggagagggg 4080

cgcgcaaccg gctaagtaat atgcagctgg cacgacaggt ttcccgactg gaaagcgggc 4140

agtgagcgca tacgtcgacc gtgctgtcca aagggctgac ctttcgcccg tcactcgcgt 4200

acgcaattaa tgtgagttag ctcactcatt aggcacccca ggctttacac tgcgttaatt 4260

acactcaatc gagtgagtaa tccgtggggt ccgaaatgtg tttatgcttc cggctcgtat 4320

gttgtgtgga attgtgagcg gataacaatt aaatacgaag gccgagcata caacacacct 4380

taacactcgc ctattgttaa tcacacagga aacagctatg accatgatta cgccaagctt 4440

gcatgcaaat agtgtgtcct ttgtcgatac tggtactaat gcggttcgaa cgtacgttta 4500

tctatttcaa ggagacagtc ataatgaaat acctattgcc tacggcagcc agataaagtt 4560

cctctgtcag tattacttta tggataacgg atgccgtcgg gctggattgt tattactcgc 4620

ggcccagccg gccatggccc aggtgcagct cgacctaaca ataatgagcg ccgggtcggc 4680

cggtaccggg tccacgtcga gcaggtcggc ctcgagatca aacgggcggc cgcaggtgcg 4740

ccggtgccgt cgtccagccg gagctctagt ttgcccgccg gcgtccacgc ggccacggca 4800

atccagatcc gctggaaccg cgtggggccg caagcgcttg gagccacccg taggtctagg 4860

cgaccttggc gcaccccggc gttcgcgaac ctcggtgggc cagttcgaaa aataataagg 4920

atccgaattc actggccgtc gttttacaac gtcaagcttt ttattattcc taggcttaag 4980

tgaccggcag caaaatgttg gtcgtgactg ggaaaaccct ggcgttaccc aacttaatcg 5040

ccttgcagca cagcactgac ccttttggga ccgcaatggg ttgaattagc ggaacgtcgt 5100

catccccctt tcgccagctg gcgtaatagc gaagaggccc gcaccgatcg gtagggggaa 5160

agcggtcgac cgcattatcg cttctccggg cgtggctagc cccttcccaa cagttgcgca 5220

gcctgaatgg cgaatggcgc ctgatgcggt gggaagggtt gtcaacgcgt cggacttacc 5280

gcttaccgcg gactacgcca attttctcct tacgcatctg tgcggtattt cacaccgcat 5340

acgtcaaagc taaaagagga atgcgtagac acgccataaa gtgtggcgta tgcagtttcg 5400

aaccatagta cgcgccctgt agcggcgcat taagcccggc gggtgtggtg ttggtatcat 5460

gcgcgggaca tcgccgcgta attcgggccg cccacaccac gttacgcgca gcgtgaccgc 5520

tacacttgcc agcgccctag cccccgctcc caatgcgcgt cgcactggcg atgtgaacgg 5580

tcgcgggatc gggggcgagg tttcgctttc ttcccttcct ttctcgccac gttcgccggc 5640

tttccccgtc aaagcgaaag aagggaagga aagagcggtg caagcggccg aaaggggcag 5700

aagctctaaa tcgggggctc cctttagggt tccgatttag tgctttacgg ttcgagattt 5760

agcccccgag ggaaatccca aggctaaatc acgaaatgcc cacctcgacc ccaaaaaact 5820

tgatttgggt gatggttcac gtagtgggcc gtggagctgg ggttttttga actaaaccca 5880

ctaccaagtg catcacccgg atcgccctga tagacggttt ttcgtccttt gacgttcgag 5940

tccacgttct tagcgggact atctgccaaa aagcaggaaa ctgcaagctc aggtgcaaga 6000

ttaatagtgg actcttgttc caaactggaa caatactcaa ccctatctcg aattatcacc 6060

tgagaacaag gtttgacctt gttatgagtt gggatagagc ggctattctt ttgatttata 6120

agggattttg ccgatttcgg cctattggtt ccgataagaa aactaaatat tccctaaaac 6180

ggctaaagcc ggataaccaa aaaaaatgag ctgatttaac aaaaatttaa cgcgaatttt 6240

aacaaaatat ttttttactc gactaaattg tttttaaatt gcgcttaaaa ttgttttata 6300

taacgtttac aattttatgg tgcagtctca gtacaatctg ctctgatgcc attgcaaatg 6360

ttaaaatacc acgtcagagt catgttagac gagactacgg gcatagttaa gccagccccg 6420

acacccgcca acacccgctg acgcgccctg cgtatcaatt cggtcggggc tgtgggcggt 6480

tgtgggcgac tgcgcgggac acgggcttgt ctgctcccgg catccgctta cagacaagct 6540

gtgaccgtct tgcccgaaca gacgagggcc gtaggcgaat gtctgttcga cactggcaga 6600

ccgggagctg catgtgtcag aggttttcac cgtcatcacc gaaacgcgcg ggccctcgac 6660

gtacacagtc tccaaaagtg gcagtagtgg ctttgcgcgc at 6702

## Description

The present invention relates to the use of molecules interfering with the function of neuropilin-1 in tumor cells for the treatment of specific cancers. Polypeptides, compositions and methods are provided that are useful for reducing or inhibiting the invasiveness and/or the metastatic potential of specific tumor cells. Furthermore, a method is provided that allows to determine whether a naturally occurring tumor cell depends on functional neuropilin-1 for its invasiveness and/or metastatic potential.

### BACKGROUND OF THE INVENTION

Malignant tumors shed cells, which migrate to new tissues and create secondary tumors. The process of generating secondary tumors is called metastasis and is a complex process in which tumor cells colonize sites distant from the primary tumor. Recently, research has been focused on identifying specific proteins involved in metastasis, which can be used as a basis for better diagnostic or improved therapeutic strategies. Cell adhesion molecules (CAM's), which mediate cell-cell or cell-matrix interactions, have been proposed to be involved in the process of metastasis. Cell adhesion in normal cells involves interactions between numerous cell surface proteins. Adhesive interactions are known to involve interactions between substances surrounding the cell (e.g., extracellular matrix molecules, for example fibronectin, vitronectin and laminin) and extracellular adhesion receptors. It has also become apparent that cell adhesion molecules fulfill much more complex functions, which may result in cells acquiring the ability to proliferate and invade host tissues.

Liotta (1986) Cancer Res. 46, 1-7 has proposed a three-step hypothesis for the process of metastasis: The first step is tumor cell attachment via cell surface receptors. The anchored tumor cell next secretes hydrolytic enzymes or induces host cells to secrete enzymes, which can degrade the matrix locally. Matrix lysis most likely takes place in a highly localized region close to the tumor cell surface. The third step is tumor cell locomotion into the region of the matrix modified by proteolysis. Thus, invasion of the matrix is not merely due to passive growth pressure but requires active biochemical mechanisms. Degradation of the surrounding normal tissue is a central feature of invasiveness of malignant tumors.

A protein involved in neuronal cell attraction and retraction is neuropilin-1. Neuropilin-1 is a transmembrane glycoprotein with a mass of approximately 130kDa. Neuropilin-1 is a multifunctional protein. Fujisawa et al. (1998) Cur. Opin. Neurobiol. 8, 587-592 describe that neuropilin-1 acts as a receptor for the semaphorin/collapsin family of neural guidance mediators. Further, neuropilin-1 has been associated with angiogenesis. Angiogenesis is the sprouting of new blood vessels, which is dependent on endothelial cell proliferation and migration. It occurs at specific times in development and growth, e.g. during development of the embryo or wound healing. (Folkman et al. (1987) Science 235, 442-447, Folkman (1991) J. Natl. Cancer Inst. 82, 4-6). Angiogenesis is also involved in the pathogenesis of disorders dependent on the growth of new blood vessels, the most relevant of which are tumor growth and growth of metastases (Hanahan et al. (1996) Cell 86, 353-364). Vascular endothelial growth factor (VEGF) herein is considered to be the prime regulator for both physiological and pathological angiogenesis, acting through VEGF receptors on endothelial cells and mediating angiogenic signals (Dvorak et al. (1999) Curr. Top. Microbiol. Immunol. 237, 97-132, Neufeld et al. (1999) FASEB J. 13, 9-22). Expression of neuropilin-1 in endothelial cells enhances the binding of VEGF₁₆₅ to VEGFR-2 and VEGF₁₆₅-induced chemotaxis. According to Gagnon et al. and WO 99/29729, neuropilin-1 acts as a co-receptor that enhances VEGFR-2 activity (Gagnon et al. (2000) PNAS 97, 2573-2578).

Neuropilin-1 is also expressed on certain tumor cells of ektodermal origin, like cells derived from prostate and breast carcinoma as well as from melanoma. In experiments with breast cancer cells VEGF₁₆₅ stimulated breast cancer cell motility in a dose-dependent manner (WO 99/29729). A comparison between two types of rat prostate carcinoma cells, AT2.1 cells and AT3.1 cells showed that the highly motile AT3.1 cells expressed more neuropilin-1 than the less motile AT2.1 cells. Collapsin-1, a ligand of neuropilin-1, inhibited the basal migration of PAE cells (porcine aortic endothelial cells) when cells were transfected with the neuropilin-1 cDNA to overexpress neuropilin-1. All this was interpreted by the authors of WO 99/29729 to suggest that neuropilin-1 expression was associated with a motile phenotype of tumor cells. However, it is dangerous and speculative to assign a physiological role for a protein based on a study in which it has been overexpressed, and it is an accepted standard in science to interpret results of overexpression studies with great care. To give only one example, the peroxisomal protein PEX 11 had been proposed, based on overexpression studies, to play a role in fatty acid oxidation. The careful studies of Li and Gould (2002) J. Cell Biol. 156, 643-651 showed that PEX 11 had no such role and instead acts on peroxisome division.

The determination of the physiological role of a protein is a prerequisite for deciding whether interference with this protein's function might be a possible avenue for the treatment of disease or not. It must be kept in mind that in a physiological setting, that is to say in a naturally occurring tumor cell of a patient, neuropilin-1 is overexpressed together with other proteins which can modulate and change the function of neuropilin-1. It is the functional interplay between neuropilin-1 and interacting proteins that determines its physiological role.

As discussed in WO99/29729 as well as in Soker et al., Cell (1998) 92, 735-745, neuropilin-1 can act as a co-receptor of VEGFR-2 for VEGF₁₆₅ to mediate the effects of this regulator for both physiological and pathological angiogenesis. VEGFR-2 is expressed in endothelial and haematopoietic precursors, endothelial cells, nascent haematopoietic stem cells and the umbilical cord stroma only (for review see Robinson and Stringer J. Cell Sci. (2001) 114, 853-865). If the cancer-related function of neuropilin-1 was dependent on its role in VEGF-signaling - as suggested by, e.g., WO 99/29729 - then this cancer-related function of neuropilin-1 should only be relevant for tissues which express neuropilin-1 and VEGFR-2 together in the presence of VEGF. The expression of VEGFR-2 is, however, limited to certain tissues. It is therefore unclear whether neuropilin-1 plays any cancer-related role in those tissues, which do not express VEGFR-2. The authors of WO 99/29729 showed that VEGF₁₆₅ stimulated 231 breast cancer cell motility in a dose dependent-manner. They postulated that tumor cells, which do not express the VEGF receptors KDR or Flt-1, are responsive to VEGF₁₆₅ via the neuropilin-1.

We now show that neuropilin-1-dependent invasion and/or adhesion can be inhibited by using the molecules of this invention. Surprisingly, we have found that stimulation of HT1080 cells with VEGF₁₆₅ has no influence on the invasiveness of cells. Invasiveness of HT1080 cells could only be stimulated in the presence of FCS. Therefore, we believe, while not wishing to be bound by theory, that neuropilin-1 mediated invasion and/or adhesion is independent on the stimulation of VEGF₁₆₅.

The development of drugs, which inhibit metastasis of such naturally occurring tumor cells - and especially of metastatic sarcomas, which are hardly curable with current cancer treatments - wherein the metastatic potential depends on neuropilin-1-related invasion and/or adhesion, would therefore be of highest interest.

We have now surprisingly discovered that neuropilin-1 acts as an active mediator of the invasion and/or adhesion of metastatic tumor cells.

### SUMMARY OF THE INVENTION

In an unbiased screen for molecules that can inhibit invasiveness and/or adhesiveness of naturally occurring tumor cells surprisingly an antibody fragment binding to the extracellular domain of neuropilin-1 has been identified as such an inhibitor.

The present invention relates to polypeptides, which can specifically bind to the extracellular domain of neuropilin-1 and inhibit neuropilin-1 function.

Furthermore, the polypeptides of the invention can be labeled with detectable groups, if desired, or can be part of a bioconjugate.

The invention further relates to pharmaceutical compositions comprising a bioconjugate of the invention.

In a further embodiment the invention relates to nucleic acid molecules encoding a polypeptide of the invention, as well as to vectors comprising such a nucleic acid and to host cells comprising such a vector.

In a further embodiment the invention relates to the use of molecules inhibiting neuropilin-1 function for the manufacture of a medicament for the treatment or prevention of invasion and/or metastasis of naturally occurring cancer cells; e.g. of mesodermal origin, wherein invasiveness and/or metastatic potential of said cancer cells depends on neuropilin-1 function.

In a further embodiment the invention relates to a method of treating or preventing invasion and/or metastasis in a patient, wherein the invasiveness and/or metastatic potential of said cancer cells depends on neuropilin-1 function.

In a further embodiment the invention relates to a method to determine the dependency of the invasiveness and/or adhesiveness of a naturally occurring cancer cell on the functionality of neuropilin-1.

In a further embodiment the invention relates to a method for the identification of a ligand useful for inhibiting the invasiveness and/or adhesiveness of a naturally occurring cancer cell, particularly the identification of such ligands that bind to the extracellular domain of neuropilin-1.

### DETAILED DESCRIPTION OF THE INVENTION

In order that the invention described herein may be more fully understood, the following detailed description is provided. As used herein, the following definitions shall apply unless otherwise indicated.

A "polypeptide" as used herein is a molecule comprising more than 10, preferably more than 20, most preferably more than 30, and less than 10000, more preferably less than 2500, most preferably less than 1000 aminoacids. Also polypeptides with substantial amino acid sequence identity and polypeptides, which contain a low percentage of modified or non-natural amino acids are encompassed.

The terms "antibody" and "immunoglobulin", as used herein refer to any immunological binding agent, including polyclonal and monoclonal antibodies. Depending on the type of constant domain in the heavy chains, antibodies are assigned to one of five major classes: IgA, IgD, IgE, IgG, and IgM. Several of these are further divided into subclasses or isotypes, such as IgG1, IgG2, IgG3, IgG4, and the like. The heavy-chain constant domains that correspond to the different classes of immunoglobulins are termed alpha, delta, epsilon, gamma and mu, respectively. The subunit structures and three-dimensional configurations of different classes of immunoglobulins are well known.

Antibodies may be also selected from modified immunoglobulins, for example chemically or recombinantly produced antibodies, CDR grafted antibodies or humanized antibodies, site directed mutagenized antibodies that exhibit substantial amino acid sequence identity in their CDR regions, particularly in their CDR3 region, to the corresponding antibody fragments of the invention and retain substantially the same affinity for neuropilin-1 binding as the corresponding antibody fragments.

The CDRs (complementary determining region) of an antibody are the parts of these molecules that determine their specificity and make contact with specific ligands. The CDRs are the most variable parts of the molecule and contribute to the diversity of these molecules. They are structurally defined in a human IgG as amino acids 24 to 41 (CDR-L1), 50 to 57 (CDR-L2) and 90 to 101 (CDR-L3) of the light chain and amino acids 26 to 38 (CDR-H1), 51 to 70 (CDR-L2) and 100 to 125 (CDR-H3) of the heavy chain (see Kabat et al. (1987) 4th edn US Department of Health and Human Services, Public Health Service, NIH, Bethesda). The CDR regions of an antibody fragment can easily be determined by somebody skilled in the art by aligning the antibody fragment with said human IgG, e.g. using a program of the NCBI that allows to "Blast", and thereby align, two sequences with one another, and identifying the amino acids of the antibody fragment corresponding to the CDRs of a human IgG.

Substantial amino acid sequence identity as used herein means that at least 70%, preferably at least 75%, 80%, 85%, 90%, more preferably all but 5, still more preferably all but 3 and even more preferably all but 1 of the amino acids of two aligned amino acid sequences, particularly of aligned CDRs, are identical.

The term "antibody fragment" is used to refer to any fragment of an antibody-like molecule that has an antigen binding region, and this term includes antibody fragments such as scFv, dsFv, Fab', Fab, F(ab')₂, Fv, single domain antibodies (DABs), diabodies, and the like. The techniques for preparing and using various antibody-based constructs and fragments are well known in the art (see Kabat et al. (1991) J. Immunol. 147, 1709-19), specifically incorporated herein by reference.

"scFv" antibody fragments comprise the VH and VL domains of an antibody, wherein these domains are present in a single polypeptide chain. Generally, the scFv polypeptide further comprises a polypeptide linker between the VH and VL domains that enables the scFv to form the desired structure for antigen binding.

A "Fv" fragment is the smallest antibody fragment that retains an intact antigen binding site.

A "dsFv" is a disulfide stabilized Fv.

A "Fab" fragment, is an antigen binding fragment, containing complete light chains paired with the VH and CH1 domains of the heavy chain.

A "Fab"' fragment, is a reduced F(ab')₂ fragment.

A "F(ab')₂" fragment, is a bivalent fragment comprising two Fab fragments linked by a disulfide bridge at the hinge region.

A "single domain antibody (DAB)" is an antibody with only one (instead of two) protein chain derived from only one of the domains of the antibody structure. Dabs exploit the finding that, for some antibodies, half of the antibody molecule binds to its target antigen almost as well as the whole molecule (Davies et al. (1996) Protein Eng. 9: 531-537.

"Diabodies" are bivalent or bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (Holliger et al. (1993) Proc. Natl. Acad. Sci. USA, 90, 6444-6448).

The terms "label" or "labeled" refers to a detectable marker or the incorporation of such, respectively, e.g., by incorporation of a fluorophore-, chromophore- or radio-labeled amino acid or attachment of a fluorophore-, chromophore- or radiolabel to a polypeptide or attachment of moieties that can be detected by a labeled second molecule containing a fluorescent marker or enzymatic activity that can be detected by an optical or a colorimetric method. An example for such a two-step detection system is the well known biotin-avidin system. Various methods of labeling polypeptides and glycoproteins are known in the art and may be used (for example see Lobl et al. (1988) Anal. Biochem., 170, 502-511).

An "epitope" includes any protein determinant capable of specific binding to an immunoglobulin or an antibody fragment. Epitopic determinants usually consist of chemically active surface groupings of molecules such as exposed amino acids, aminosugars, or other carbohydrate side chains and usually have specific three-dimensional structural characteristics, as well as specific charge characteristics.

A "naturally occurring cancer cell" as used herein is a cell that has not been transfected, transduced or otherwise genetically engineered in the laboratory. Such a cell does not comprise artificial DNA sequences, e.g. of vectors, or DNA sequences being found only in other species, but not usually in the species from which the naturally occurring cancer cell was derived. However, a naturally occurring cancer cell may comprise sequences that are not usually found in the species from which it was derived, if those sequences have arisen due to the processes of mutation and selection that took place within the individuum from which the naturally occurring cancer cell was derived, and/or during continued culture of the naturally occurring cancer cell.

Selected cancer cell-types as used herein consist of tumor cells derived from , e.g. the mesoderm, preferably derived from the group of neoplasms consisting of soft tissue tumors and sarcomas, fibromatous neoplasms, myxomatous neoplasms, lipomatous neoplasms, myomatous neoplasms, complex mixed and stromal neoplasms, synovial-like neoplasms, mesothelial neoplasms, lymphatic vessel tumors, osseous and chondromatous neoplasms, giant cell tumors, miscellaneous bone tumors, odontogenic tumors, hodgkin's and non-Hodgkins's lymphoma, plasma cell tumors, mast cell tumors, immunoproliferative diseases and leukemias, in particular tumor cells derived from the group of neoplasms consisting of soft tissue tumors and sarcomas, fibromatous neoplasms, myxomatous neoplasms, lipomatous neoplasms, myomatous neoplasms, complex mixed and stromal neoplasms, synovial-like neoplasms, mesothelial neoplasms, lymphatic vessel tumors, osseous and chondromatous neoplasms, giant cell tumors, miscellaneous bone tumors, odontogenic tumors, in particular tumor cells derived from sarcomas, in particular derived from sarcomas of the bone or sarcomas of soft tissue. Neoplasmas can also be derived from brain, central nervous system, lungs, stomach, lower intestine, liver, kidneys and the pancreas.

In one embodiment sarcomas can be all sarcomas with the exception of angiomas and haemangiomas.

"Treating metastatic tumors" or "treating micro-metastases", as used herein means that the metastasis of the tumor is stabilized, prevented, delayed, or inhibited by the molecule of the invention, either as a single medicament or in combination with other medicaments. Stable disease or "No Change" (NC) is a description for the course of the disease with either no change of the metastases or a reduction of less than 50% or an increase of less than 25 % over at least 4 weeks. Prevention can be, for example, that no new metastases are detected after the treatment is initiated. This can lead to a two- to three-fold median and/or a %-year survival rate of treated patients compared with untreated patients. A delay can signify a period of at least 8 weeks, 3 months, 6 months or even one year in which no new metastases are detected after the treatment is initiated. Inhibition can mean that the average size or the total number of new metastases is at least 30%, 40%, 50%, 60%, 70%, 80% or even 90% lower in a group treated with the molecule of the invention in comparison with an untreated group. Number, size and prevalence of metastases can be detected by a skilled practitioner in the field of oncology following generally accepted practice and diagnostic procedures for the detection of metastases, for example as outlined in Harrisons Principles of Internal Medicine 15^{th} ed 2001 Mc Graw Hill.

"Metastatic tumors" as used herein includes both tumors at the primary site capable of metastasizing and metastasized tumors at a secondary site. Such metastatic tumors can be of any organ or tissue origin, like brain, central nervous system, lungs, stomach, lower intestine, liver, kidneys or the pancreas, etc., in particular tissue of mesodermal origin such as bone, spleen, thyroid, endometrial, ovarian, or lymphoid tissue.

A micro-metastase is an accumulation of tumor cells with a size smaller than 2 mm, which can usually only be detected by histological methods.

"Invasiveness" as used herein is the ability of a cell to migrate through a layer of other cells or to migrate through the extracellular matrix. Invasiveness can be assessed by the Matrigel assay described in Example 8 or Example 9. Invasion is measured as cells that reach the lower surface of the filter during a certain incubation period. When more than 40% of cells within 6h to 12h reach the other side of the filter and form colonies in an invasion assay like in Example 8 or Example 9, the naturally occurring cancer cell is defined as invasive. The control cells instead form only up to 5% colonies in the same time frame and are defined as non-invasive.

"Adhesiveness" as used herein is the ability of a cell to reattach after they have been removed from the matrix on which it had been grown, resuspended as a solution of single cells (not in direct contact with other cells of the solution), and replated on a matrix to which adhesion is possible. A cell is defined as adhesive if in an assay as described in Example 11 or Example 12, more than 40% of the cells adhere within a time of 30-120 min. Instead, only up to 5% of the control cells adhere within the same time frame.

Metastatic potential as used herein is the ability of a tumor cell to form a new tumor at a site distant from the primary tumor of which the tumor cell was derived (a metastase). Metastatic potential can be measured by injecting, e.g. 1x10⁶ , cells into the lateral tail vein of athymic nude mice and determining the number of tumor nodules in the lung, e.g. 2 months post injection, e.g. as described in the section "Tumor cell injections" on page 2346 of Huang et al (1996) Oncogene 13, 2339-2347, or the sections "Animals and production of tumors" and "Histochemical analysis for calcified matrix" on page 1882 of Radinsky et al. (1994) Oncogene 9: 1877-1883. A cell line to produce more than 3, preferably more than 8, more preferably more than 20 tumor nodules in the lung in this assay is considered metastatic.

Therapeutically effective amounts are amounts which eliminate or reduce the patient's tumor burden, or which prevent, delay or inhibit metastasis. The dosage will depend on many parameters, including the nature of the tumor, patient history, patient condition, the possible co-use of cytotoxic agents, and methods of administration. Methods of administration include injection (e.g., parenteral, subcutaneous, intravenous, intraperitoreal, etc), for which the molecule inhibiting neuropilin-1 function is provided in a nontoxic pharmaceutically acceptable carrier. In general, suitable carriers and diluents are selected so as not to significantly impair biological activity of the binding agent (e.g., binding specificity, affinity or stability), such as water, saline, Ringer's solution, dextrose solution, 5% human serum albumin, fixed oils, ethyloleate, or liposomes.). Acceptable carriers can include biocompatible, inert or bio-absorbable salts, buffering agents, oligo- or polysaccharides, polymers, viscoelastic compound such as hyaluronic acid, viscosity-improving agents, preservatives, and the like. In addition, the pharmaceutical composition or formulation may also include other carriers, adjuvants, or nontoxic, non-therapeutic, non-immunogenic stabilizers and the like. Typical dosages may range from about 0.01 to about 20 mg/kg, or more particularly from about 1 to about 10 mg/kg.

Therapeutic methods employing molecules inhibiting neuropilin-1 function may be combined with chemotherapy, surgery, and radiation therapy, depending on type of the tumor, patient condition, other health issues, and a variety of factors. The molecules inhibiting neuropilin-1 function may also be used as the single effective medicament of a therapeutic composition.

A "molecule inhibiting neuropilin-1 function", is a molecule resulting in inhibition of the biological activity of neuropilin-1. This inhibition of the biological activity of neuropilin-1 can be assessed by measuring one or more indicators of neuropilin-1's biological activity, such as neuropilin-1 dependent invasiveness or neuropilin-1 dependent adhesion. These indicators of neuropilin-1's biological activity can be assessed by one or more of several *in vitro* or *in vivo* assays (see, Examples 8 or 9 and Examples 11 or 12). Preferably, the ability of a molecule to inhibit neuropilin-1 activity is assessed by inhibition of neuropilin-1-induced invasiveness or adhesion of invasive human sarcoma cells, particularly the cells used in Examples 8 and 9 or Examples 11 and 12.

A "molecule inhibiting neuropilin-1 function" of the invention is not a molecule which is a general inhibitor of protein function, like a protease, like a denaturing agent, e.g. urea or guanidinium hydrochloride, like heavy metal atoms or like small molecules (e.g. aldehydes or isocyanates) reacting covalently and non-specifically with biomolecules (lipids, proteins, sugars). A molecule inhibiting neuropilin-1 function is characterized by its ability to inhibit neuropilin-1 function at a concentration at which it does not inhibit the function of the insulin receptor (e.g. as determined in an anti-Phosphotyrosine Western Blot Assay, see e.g. B. Cariou et al. (2002) J Biol Chem., 277, 4845-52) and the Acetylcholin receptor (e.g. as determined by measuring the Ca influx, see M. Montiel et al. (2001) Biochem Pharmacol. 63, 337-42.) and the B-CAM cell surface glycoprotein (e.g. by determining binding of hemoglobin A red blood cells (AA RBCs) to immobilized laminin as described in the section "Flow chamber assays" on page 2551 of Udani et al. (1998) J. Clin. Invest. 101, 2550-2558). Only a molecule inhibiting neuropilin-1 function but at the same concentration not significantly affecting the function of the other three receptors mentioned is a molecule inhibiting neuropilin-1 function as used in this patent. Inhibition is understood to be at least a 15%, preferably a 20%, more preferably a 25%, 30%, 40%, 50% or even a 60% decrease in function, as defined by neuropilin-1 function in an invasion and/or adhesion assay as mentioned above, when compared to a negative control with the same experimental conditions, but without the molecule of the invention. A molecule is defined as not significantly affecting the function of the other three receptors if the decrease in function affected by the molecule of the invention is less than 10%, more preferably less than 5%.

Additionally, in the case of a molecule of the invention which inhibits gene expression of neuropilin-1, such a molecule decreases neuropilin-1 expression by more than 50%, preferably by more than 80%, still more preferably by more than 90%, most preferably by more than 95% when measured in a quantitative western blot normalized to the level of beta tubulin present, when present in an experiment at a concentration of 10 nM to 100 µM, preferably at around 1 µM, in which the amount of neuropilin-1 is compared between two otherwise identical samples, wherein in one sample the molecule of the invention was allowed to inhibit neuropilin-1 expression. In the same experiment the molecule of the invention does not decrease the amount of the beta tubulin present per cell by more than 20%, and said molecule does not decrease the relative level of the insulin receptor and the B-CAM cell surface glycoprotein by more than 20%.

Additionally, in the case of a polypeptide of the invention, particularly an antibody or antibody fragment of the invention, the polypeptide of the invention is considered to inhibit the biological function of neuropilin-1 if it reduces the invasiveness and/or adhesiveness of naturally invasive cancer cells in an experiment as in Example 8 or 9 or Example 11 or 12 by more than 15%, preferably more than 20%, more preferably a 25%, 30%, 40%, 50% or even a 60% decrease in neuropilin-1 function as defined above, when said antibody fragment is present at a concentration of 1 nM to 50 µM, preferably around 20 µM.

Additionally, in the case of a small chemical compound of the invention, said compound is considered to inhibit the biological function of neuropilin-1 if it reduces the invasiveness and/or adhesiveness of naturally invasive cancer cells in an experiment as in Example 8 or 9 and Example 11 or 12 by more than 15%, preferably more than 20%, more preferably a 25%, 30%, 40%, 50% or even a 60% decrease in neuropilin-1 function as defined above, when present at a concentration of 10 nM to 100 µM, preferably at around 1 µM, while not affecting cell morphology, cell cycle progression (determined by analyzing the DNA content of a cell population by propidium iodide staining and FACS analysis), and not increasing the percentage of the cells of the culture that show signs of apoptosis (determined by measuring the percentage of cells showing DNA fragmentation, e.g. by a so called tunnel-assay). A small chemical compound as used in this invention is a molecule with a molecular weight between 50 Da and 10000 Da, preferably between 100 Da and 4000 Da, more preferably between 150 Da and 2000 Da, or a physiologically acceptable salt thereof.

"Identifying" as used herein means the identification of a biomolecule having desired properties from a mixture of biomolecules comprising related but non-identical biomolecules with slightly different properties.

A "ligand" as used herein is a molecule displayable by an amplifiable ligand-displaying unit. A ligand is that part of an ALDU through which the ALDU can bind to a target. Preferably it is a polypeptide as defined above, an RNA-oligonucleotide or a DNA-oligonucleotide an oligonucleotide comprising more than 20 base units but less than 10,000, preferably less than 1,000 base units. A ligand can bind to an extracellular region of an antigen. This binding may have specificity in the sense that the ligand binds to one antigen with high affinity but to a moderately related antigen with lower, for example 10- or 50- or 200-fold lower affinity. Moderately related antigens are antigens with up to 30 % amino acid identity in the extracellular regions.

A ligand "binding specifically to a neuropilin-1" as mentioned herein can be a ligand which binds to neuropilin-1 under the buffer conditions given in Examples 2 and 3. The dissociation constant between the ligand and neuropilin-1 can be measured, e.g. by use of the so-called BIACORE System (see, for example, Fivash et al. Curr Opin Biotechnol. (1998) 9, 97-101) and "binding specifically" can then be understood to mean that the dissociation constant between the ligand and neuropilin-1 is lower than 10 µM, preferably lower than 1 µM, more preferably lower than 500, 400, 300, 200, 100, 50, 20 nM, most preferably from 0,1 nM to 20 nM if measured under standard conditions, for example at 20°C, ambient pressure and in a suitable buffer, e.g. 20 mM Tris, 100 mM NaCl, 0,1 mM EDTA at an overall pH of 7.0. Further, this molecule does not bind to neuropilin-2, which is another member of the neuropilin family and shares a 47% homology to neuropilin-1. Thus, the dissociation constant between the ligand and neuropilin-2 is higher than 100 µM, preferably higher than 1 mM, or is alternatively at least 50-fold, preferably at least 200-fold, 1000-fold or 5000-fold worse (higher) than the dissociation constant between ligand and neuropilin-1.

The term "at least one" as used here means "one and more than one", particularly one, two, three, four and five.

The present invention relates to polypeptides, which can specifically bind to the extracellular region of neuropilin-1 and can inhibit neuropilin-1 function in invasion and/or metastasis. Those polypeptides comprise a sequence selected from the group consisting of SEQ ID NO. 1 and SEQ ID NO. 2.

In a preferred embodiment the polypeptide of the invention is an antibody fragment, in particular a scFv, dsFv, Fab', Fab, F(ab')2, Fv, single domain antibody or diabody, more particularly a scFv, dsFv, Fv, single domain antibody or diabody, still more particularly a scFv, single domain antibody or diabody and even more preferably a scFv.

In another preferred embodiment the polypeptide of the invention is an antibody, in one preferred embodiment an antibody derived from a scFv antibody fragment, in another preferred embodiment a polyclonal or monoclonal antibody, particularly a human monoclonal antibody.

Anti-human neuropilin-1 binding antibodies may be selected from modified immunoglobulins, for example chemically or recombinantly produced antibodies or humanized antibodies, site directed mutagenized antibodies, that exhibit substantial amino acid sequence identity in their CDR regions, particularly in their CDR3 region, to the corresponding antibody fragments of the invention and retain substantially the same affinity for neuropilin-1 binding as the corresponding antibody fragments.

In another preferred embodiment the polypeptide of the invention is a human antibody selected from the group consisting of IgA, IgD, IgE, IgG, and IgM, in particular IgG and IgM, more particularly IgG1, IgG2a, IgG2b, IgG3, IgG4.

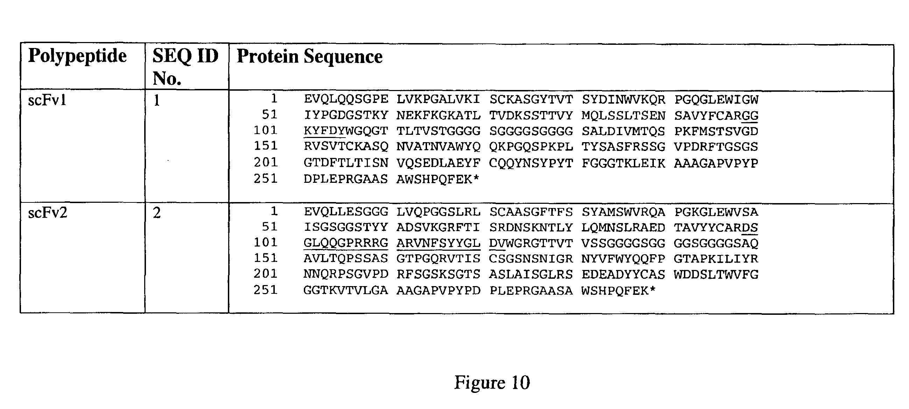

In another preferred embodiment the CRD3 region of the antibody or the antibody fragment is identical to one of the CDR3 regions shown in Figure 10.

In another preferred embodiment of the invention a polypeptide of the invention, particularly an antibody fragment or an antibody of the invention, is labeled with a detectable label. Particularly, examples for detectable labels are radioisotopes, chromophores, fluorophores, enzymes or radioisotopes. The detectable label can, for example, be selected from this group.

In another embodiment, the polypeptide of the invention can be covalently or non-covalently conjugated and/or coupled to or with, respectively, another protein, a solid matrix (e.g. like a bead), with itself to form multimers, a cytotoxic agent further enhancing the toxicity to targeted cells, a cytostatic agent, a prodrug, or an effector molecule, which is able to modify the cell expressing neuropilin-1 or to recruit immune cells. All these conjugates are "bioconjugates" of the invention.

A list of cytotoxic agents include, but is not limited to, daunorubicin, taxol, adriamycin, methotrexate, 5 FU, vinblastin, actinomycin D, etoposide, cisplatin, doxorubicin, genistein, andribosome inhibitors (e.g., trichosantin), or various bacterialtoxins (e.g., Pseudomonas exotoxin; Staphylococcus aureus protein A).

Bioconjugates comprising the polypeptides of the invention, particularly the antibody fragment or antibody of the invention, together with said cytotoxic moieties are made using a variety of bifunctional protein coupling agents. Some examples of such reagents are N-succinimidyl 3-(2-pyridyldithio)-propionate (SPDP), bifunctional derivatives of imidoesters such a dimethyl adipimidate HCl, active esters such as disuccinimidyl suberate, aldehydes such as glutaraldehyde, bisazido compounds such as bis-(R-azidobenzoyl)hexanediamine, bisdiazonium derivatives such as bis-(R-diazoniumbenzoyl)ethylenediamine, diisocyanates such as tolylene 2,6-diisocyanate, and bis-activated fluorine compounds such as 1,5-difluoro-2,4-dinitrobenzene. Methods useful for the production of bioconjugates are described in detail in March's Advanced Organic Chemistry: Reactions, Mechanisms and Structure, 5th Edition, Wiley-Interscience; or Bioconjugate Techniques, Ed. Greg Hermanson, Academic Press.

The expression of a metastasis-associated neuropilin-1 antigen can be detected by using a bioconjugate or a polypeptide of the invention, particularly an antibody or an antibody fragment of the invention. A sample is taken from the subject, e.g., a biopsy specimen taken from tissue suspected of having a metastatic tumor. Generally, the sample is treated before an assay is performed. Assays, which can be employed include ELISA, RIA, EIA, Western Blot analysis, immunohistological staining and the like. Depending upon the assay used, the antigens or the antibodies can be labeled by an enzyme, a fluorophore or a radioisotope. (See, e.g., Coligan et al. (1994) Current Protocols in Immunology, John Wiley & Sons Inc., New York, New York; and Frye et al. (1987) Oncogene 4, 1153-1157.)

Therefore, one embodiment of the invention relates to the use of at least one polypeptide of the invention and/or at least one labeled polypeptide of the invention and/or at least one bioconjugate of the invention for the detection of neuropilin-1. For example one polypeptide of the invention or one labeled polypeptide of the invention or one bioconjugate of the invention can be used for the detection of neuropilin-1, or one labeled polypeptide together with one bioconjugate or two or three polypeptides or two labeled polypeptides can be used.

In another embodiment, the present invention encompasses a diagnostic kit. Such a kit comprises at least one bioconjugate and/or at least one labeled polypeptide of the invention and/or at least one polypeptide of the invention, particularly an antibody fragment or an antibody of the invention, or a labeled version of these, and consists additionally of the reagents and materials necessary to carry out a standard competition or sandwich assay. Said diagnostic kit may be used for the determination of the invasive potential of biological samples, in particular of certain cancer cell types. A kit will further typically comprise a container.

By using the polypeptide of the invention, particularly the antibody fragment or antibody of the invention, it is further possible to produce anti-idiotypic antibodies, which can be used to screen antibodies to identify whether the antibody has the same binding specificity as a human monoclonal antibody of the invention and can also be used for active immunization (Herlyn et al. (1986) Science, 232, 100). Such anti-idiotypic antibodies can be produced using well-known hybridoma techniques (Kohler et al. (1975) Nature, 256:495). An anti-idiotypic antibody is an antibody, which recognizes unique determinants present on the antibody of interest. These determinants are located in the hypervariable region of the antibody. It is this region, which binds to a given epitope and, thus, is responsible for the specificity of the antibody. An anti-idiotypic antibody can be prepared by immunizing an animal with the polypeptide, particularly the antibody fragment or antibody, of interest. The immunized animal will recognize and respond to the idiotypic determinants of the immunizing antibody and produce an antibody to these idiotypic determinants. By using anti-idiotypic antibodies, it is possible to identify other hybridomas expressing monoclonal antibodies having the same epitopic specificity.

It is also possible to use the anti-idiotype technology to produce monoclonal antibodies, which mimic an epitope. For example, an anti-idiotypic monoclonal antibody made to a first monoclonal antibody will have a binding domain in the hypervariable region, which is the "image" of the epitope bound by the first antibody. Thus, the anti-idiotypic monoclonal antibody can be used for immunization, since the anti-idiotype monoclonal antibody binding domain effectively acts as an antigen.

In another embodiment the modified polypeptide or the bioconjugate of the invention, binds to human neuropilin-1 and reduces the invasiveness and/or adhesiveness of invasive human sarcoma cells by 15-70%, or preferably by 15-30%, 20-40% or even at least 50%, when tested in an invasion assay (see Example 8 or 9 and Example 11 or 12).

In another embodiment, the antibody fragment of the invention specifically recognizes one or more epitopes of neuropilin-1, or epitopes of conserved variants of neuropilin-1, or peptide fragments of the neuropilin-1.

In another embodiment, the invention relates to the use of a molecule of the invention, particularly selected from the group consisting of a small chemical compound of the invention, a molecule inhibiting gene expression of neuropilin-1, a bioconjugate or a polypeptide of the invention, more particularly an antibody fragment or an antibody of the invention as a medicament.

In another embodiment, the present invention relates to a pharmaceutical composition comprising effective amounts of at least one, particularly one, molecule of the invention, particularly at least a bioconjugate of the invention or at least one of a molecule inhibiting gene expression of neuropilin-1, more particularly wherein the molecule is an antisense oligonucleotide against neuropilin-1, an interfering dsRNA (siRNA) or siRNA-like hairpin RNA against neuropilin-1 or a vector leading to cellular presence of siRNA against neuropilin-1 or siRNA-like hairpin RNA against neuropilin-i, or alternatively wherein the molecule is an antibody fragment or antibody of the invention, in combination with a pharmaceutically acceptable carrier and/or a diluent. The pharmaceutical composition can be used for the treatment of conditions related to the over-expression or ectopic expression of human neuropilin-1, especially the treatment of metastatic tumors, especially of metastatic tumors derived from the group selected of cancer cell-types of pages 8 and 9.

In another embodiment of the invention, pharmaceutical compositions are provided comprising a pharmaceutically acceptable carrier and a therapeutically effective amount of at least one molecule inhibiting neuropilin-1 function, particularly wherein the molecule inhibits gene expression of neuropilin-1, more particularly wherein the molecule is an antisense oligonucleotide against neuropilin-1, an interfering dsRNA (siRNA) or siRNA-like hairpin RNA against neuropilin-1 or a vector leading to cellular presence of siRNA against neuropilin-1 or siRNA-like hairpin RNA against neuropilin-1. Alternatively the molecule inhibiting neuropilin-1 function can particularly be a molecule which can bind to the extracellular region of neuropilin-1, more particularly wherein the molecule is a small chemical compound or a modified polypeptide or the bioconjugate of the invention, still more preferably wherein the molecule is a modified scFv of the invention or a modified antibody derived from such a scFv of the invention.

In another embodiment, the invention relates to a method of treating or preventing metastasis in a patient comprising the administration of a molecule inhibiting neuropilin-1 function, in a pharmaceutical acceptable composition in an amount effective to treat, i.e. inhibit, delay or prevent, metastasis of neuropilin-1 mediated invasion and/or adhesion, particularly wherein the molecule inhibits gene expression of neuropilin-1, more particularly wherein the molecule is an antisense oligonucleotide against neuropilin-1, an interfering dsRNA (siRNA) or siRNA-like hairpin RNA against neuropilin-1 or a vector leading to cellular presence of siRNA against neuropilin-1 or siRNA-like hairpin RNA against neuropilin-1. Alternatively the molecule inhibiting neuropilin-1 function can particularly be a molecule which can bind to the extracellular region of neuropilin-1, which can be identified be the method of identifying a ligand binding specifically to the extracellular region of neuropilin-1 described below, more particularly wherein the molecule is a small chemical compound or an antibody or an antibody fragment or a modified polypeptide of the invention or a bioconjugate of the invention, still more preferably wherein the molecule is a modified scFv of the invention or a modified antibody derived from such a scFv of the invention. This method of treating or preventing metastasis can be effective to reduce or inhibit the invasion and/or adhesion of cancer cells derived from mesodermal cells, in particular cancer cells derived from sarcomas, in particular cancer cells derived from sarcomas of the bone and/or soft tissue. The cancer cells can be selected from the group of cancer cells described on page 8 and 9.

The present invention further relates to a method to produce the polypeptide of the invention by recombinant techniques. These techniques are well known in the art (Skerra et al. (1993), Curr. Opin. Immunol. 5, 256-62; Chadd et al. (2001), Curr. Opin. Biotechnol. 12, 188-94).

For example, nucleic acid sequences encoding a polypeptide of the invention, particularly an antibody fragment or an antibody (e.g., a gene encoding an antibody fragment of Figure 10 or an antibody thereof) can be isolated and cloned into one or more polynucleotide expression vectors, and the vector can be transformed into a suitable host cell line for expression of a recombinant polypeptide of the invention. Expression of the gene encoding the polypeptide of the invention provides for increased yield of the polypeptide, and also allows for routine modification of the polypeptide by introducing amino acid substitutions, deletions, additions and other modifications, for example humanizing modifications (Rapley (1995) Mol. Biotechnol. 3: 139-154) in both the variable and constant regions of the antibody fragment or of the antibody of the invention without critical loss of binding specificity or neuropilin-1 blocking function (Skerra et al. (1993) Curr. Opin. Immunol. 5, 256-262).

The present invention therefore relates to an above mentioned isolated nucleic acid molecule encoding any one of the polypeptides of the invention, particularly an antibody fragment of the invention, more particularly a scFv, dsFv, Fv, single domain antibody or diabody of the invention, still more particularly a scFv, single domain antibody, diabody of the invention or an antibody derived from such a scFv of the invention, and even more preferably a scFv of the invention or an antibody derived from such a scFv of the invention.

In a preferred embodiment the present invention relates to a nucleic acid molecule encoding a polypeptide comprising a sequence selected from the group consisting of SEQ ID NO. 1 and SEQ ID NO. 2.

The present invention further relates to a vector comprising a nucleic acid of the invention. Particularly the vector is a plasmid, a phagemid, or a cosmid.

For example, the nucleic acid molecule of the invention can be cloned in a suitable fashion into procaryotic or eucaryotic expression vectors (Sambrook et al., "Molecular cloning: a laboratory manual" Second edition, Cold Spring Harbor Laboratory Press (1989)). Such expression vectors comprise at least one promotor, at least one signal for translation initiation, at least one nucleic acid sequence of the invention and - in the case of procaryotic expression vectors - a signal for translation termination, while in the case of eucaryotic expression vectors preferably additional signals for transcriptional termination and for polyadenylation.

Examples for prokaryotic expression vectors are, for expression in *Escherichia coli,* e.g. expression vectors based on promotors recognized by T7 RNA polymerase, as described in US 4,952,496, for eucaryotic expression vectors for expression in Saccharomuces serevisiae, e.g., the vectors p426Met25 or 526GAL1 (Mummberg et al. (1994) Nucl. Acids Res., 22, 5767-5768), for the expression in insect cells, e.g., Baculovirus-vectors as e.g. described in EP-B1-0 127 839 or EP-B1-0 549 721, and for the expression in mammalian cells, e.g., the vectors Rc/CMV and Rc/RSV or SV40-vectors, which are commonly known and commercially available.

The molecular biological methods for the production of these expression vectors, as well as the methods of transfecting host cells and culturing such transfected host cells as well as the conditions for producing and obtaining the polypeptides of the invention from said transformed host cells are well known to the skilled person.

The present invention further relates to a host cell comprising a nucleic acid of the invention and/or a vector of the invention, particularly wherein the host cell is a microorganism like yeast or other fungi, like *Escherichia coli, Bacillus subtilis* or other bacteria. The host cell can also be a cell of higher eucaryotic origin, like an insect cell, preferably a virus infected insect cell, more preferably a baculovirus infected insect cell, or like a mammalian cell like HeLa, COS, MDCK 293-EBNA1, NSO or a hybridoma cell.

The present invention relates further to a method for the production of a polypeptide of the invention, particularly an antibody fragment of the invention, comprising culturing a microorganism transformed with a recombinant vector comprising DNA encoding a polypeptide of the invention, particularly an antibody fragment of the invention, and recovering said polypeptide of the invention, particularly an antibody fragment of the invention or a fusion protein containing it, from the medium.

The present invention shows that blocking neuropilin-1 function may inhibit invasiveness and/or adhesiveness of certain cancer cells derived from group selected of cancer cell-types of pages 8 and 9, and particularly inhibits invasiveness and or adhesiveness of cancer cells, e.g., derived from human sarcoma cells, lymphoma cells and mesothelial neoplasms, more particularly human sarcoma cells.

One embodiment of the invention is therefore the use of at least one, particularly one, molecule inhibiting neuropilin-1 function in the manufacture of a medicament for the treatment or prevention of invasion and/or metastasis of naturally occurring cancer cells, wherein invasiveness and/or metastatic potential of said cancer cells depends on neuropilin-1 function, particularly of such tumor cells which are derived from mesodermal cells or cancer cells derived from the group selected of the cancer cell-types of pages 8 and 9.

In one particular embodiment of the invention the molecule inhibits gene expression of neuropilin-1. Particularly, the molecule inhibiting gene expression of neuropilin-1 can be an antisense oligonucleotide targeted against neuropilin-1, an interfering double stranded RNA, commonly known as siRNA (small interfering RNA) targeted against neuropilin-1, a siRNA-like hairpin RNAs targeted against neuropilin-1 function as inhibitors of gene expression, or a vector leading to cellular presence of siRNA targeted against neuropilin-1 or siRNA-like hairpin RNAs targeted against neuropilin-1.

Antisense oligonucleotides are well known in the art as means to reduce gene expression of a specific gene (see, for example, Probst J.C. (2000) Methods 22(3): 271-281; Heasman J. (2002) Dev. Biol. 243(2): 209-214; Castanotto et al. (2000) Methods Enzymol. 313: 401-420 and Jen and Gewirtz (2000) Stem Cells. 18(5): 307-319).

Recently it has been found that short, double stranded RNAs potently and specifically inhibit expression of a mRNA with a complementary exonic sequence. This phenomenon has been termed RNA interference (RNAi). These double stranded RNAs have been termed siRNAs, for small interfering RNAs, have a paired region of at least 18, preferably at least 19 nucleotides (this paired, double stranded region targets them against an mRNA with a complementary exonic sequence), and have a length of 20 nucleotides to 25 nucleotides, preferably of 21 nucleotides to 23 nucleotides (Elbashir et al. (2001) Nature 411, 428-429). These siRNAs can be synthesized chemically by methods well known in the art and are commercially available (see e.g. suppliers given in Elbashir et al. above and also in Elbashir et al., (2002) Methods 26, 199-213) or by T7 transcription off a suitable DNA template (see Yu et al. (2002) PNAS 99, 6047-6052). They can be delivered to a wide range of cell types, in which inhibition of gene expression of a certain gene is desired, by, e.g., synthesizing the two RNA strands, annealing them and transfecting them (see Elbashir et al. and Yu et al., above). Detailed protocols for the application of siRNAs are described in Elbashir et al., (2002) Methods 26, 199-213.

Another way of effecting RNA interference is to transfect a plasmid that leads to the cellular production of siRNAs or siRNA like hairpin RNAs, which are also functional in RNAi. This can be done by plasmids which carry both, the sense and the antisense strand of the siRNA under the control of a mammalian, preferrably human or mouse, U6 promotor, which leads to the transcription of both strands in a transfected cell, some of which anneal to form functional double stranded siRNAs within the transfected cell (Miyagishi and Taira (2002) Nature Biotech. 19, 497-500). Alternatively, the RNA species transcribed from the U6 promotor can be siRNA-like hairpin RNAs which consist of a 19-base pair siRNA stem with the two strands joined by a structured loop and a U₁₋₄ 3' overhang at the end of the antisense strand (see Paul et al. (2002) Nature Biotech. 19, 505-508). Alternatively the RNA species transcribed from the U6 promotor can be short hairpin RNAs, which are transcribed as small temporal RNAs, short hairpin precursors of about 70 nucleotides, and processed into active siRNAs within the cell in which they are transcribed (see Paddison et al. (2002) Genes and Development 16, 948-958). All these methods based on transfection of plasmids have in common that they lead to the cellular production of siRNAs, which leads to the inhibition of the gene with an exonic region complementary to the at least 18 nucleotide long base paired region of the siRNA. It is clear that also future ways to affect the cellular presence of siRNAs will lead to the desired effect of inhibiting neuropilin-1 function and are within the scope of the invention.

In another preferred embodiment the molecule inhibiting neuropilin-1 inhibits the function of expressed neuropilin-1.

Expressed neuropilin-1 is to be understood in this context as neuropilin-1 protein already present on naturally occurring cancer cells before any kind of treatment is initiated.

These molecules are particularly molecules, which bind to the extracellular region of neuropilin-1.

The extracellular segment of neuropilin-1 is defined as that part of the neuropilin-1 protein outside of the cellular membrane, consisting of five domains a1, a2, b1, b2 and c. Domains, which are specifically involved in cell adhesion are b1 and b2. It should be appreciated that neuropilin-1 is a glycoprotein, so not only the mentioned amino acids, but also the sugar modifications on them are considered as being part of the extracellular segment of neuropilin-1.

More particularly this molecule is selected from the group consisting of a small chemical compound, an antibody against neuropilin-1, an antibody fragment against neuropilin-1, a polypeptide of the invention, an anti-idiotypic antibody of the invention and/or a bioconjugate of the invention, especially wherein the molecule is a polypeptide and/or a bioconjugate of the invention.

In another preferred embodiment the naturally occurring cancer cells, which depend on neuropilin-1 function for invasiveness, adhesiveness and/or metastatic potential can be any cancer cells wherein the cancer cells are cells derived from the group of neoplasms consisting of adenocarcinomas, sarcomas, morbus hodgkin, non-hodgkin lymphoma of high and low malignancy, multiple myeloma, malignant tumors of the brain, head and neck tumors, carcinoma of the thyroid, mesothelioma, leukemia, carcinoma of the esophagus, stomach and pancreas carcinoma, primary carcinomas of the liver, carcinoma of bilary duct and bladder, colorectal carcinoma, basel cell carcinoma, malignant melanoma, osteosarcoma, malignant gliomas, Ewing carcinoma, soft tissue sarcoma, Kaposi sarcoma, nephroblastoma, neuroblastoma, carcinoma of the kidney, testicular carcinoma, carcinoma of the urinary bladder, malignant tumors of the ovaries, carcinoma of the endometrium of the cervix, tumors of the adrenal gland, particularly from the group of neoplasms consisting of malignant fibrous histiocytoma, liposarcoma, fibrosarcoma, synovial sarcoma, osteosarcoma (parosteal osteosarcoma, periosteal osteosarcoma, small-cell osteosarcoma), chondrosarcoma, Ewing's sarcoma, giant-cell tumor of bone, osteogenic sarcoma, leiomyosarcoma, rhabdomyosarcoma, mesothelioma, lymphangiosarcoma, myxosarcoma, endotheliosarcoma, chordoma, Kaposi's sarcoma and lymphangioendotheliosarcoma.

The invention further pertains to the neuropilin-1 antigen as a druggable target. Another aspect of the present invention pertains to antibody fragments that bind to human neuropilin-1 with high neutralizing capacity.

In another embodiment of the invention, at least one polypeptide of the invention and/or a bioconjugate of the invention are used for identifying additional molecules that specifically bind human neuropilin-1, particularly in screening assays. These methods entail contacting a reference anti-neuropilin-1 antibody fragment with a target species comprising the neuropilin-1 domain in the presence of a putative competitor test-binding agent. This step of contacting is conducted under conditions suitable for complex formation between the reference antibody fragment and the target species in the absence of the test-binding agent. Complex formation between the reference antibody fragment and the target species in the presence of the test-binding agent is detected as an indicator of specific binding activity of the test-binding agent to neuropilin-1. This screening method is useful for high throughput screening of, e.g., other antibody libraries or antibody fragment libraries, antisense oligonucleotide libraries or peptide and small molecule libraries to identify and characterize additional "molecules binding specifically to neuropilin-1". Competition is determined by an assay in which the antibody fragment, or other binding agent under test substantially inhibits specific binding of the reference antibody fragment to the target species containing the neuropilin-1 domain. This can be determined for example by measuring binding of the reference antibody fragment to a target species comprising neuropilin-1 domain in the presence and absence of a putative competitor, i.e. a "molecule binding specifically to neuropilin-1" under conditions suitable for complex formation. Numerous types of competitive binding assays are known and routinely practicable within the invention, as described for example in U.S. Pat. No. 4,376,110. Typically, such assays involve the use of a target species containing the neuropilin-1 domain (e.g., purified neuropilin-1 or a cell line expressing the neuropilin-1 antigen), an unlabeled "molecule binding specifically to neuropilin-1", and a labeled reference antibody fragment or other binding agent. Competitive inhibition is measured by determining the amount of label bound to the target species in the presence of the "molecule binding specifically to neuropilin-1". Usually the "molecule binding specifically to neuropilin-1" is present in excess. "Molecules binding specifically to neuropilin-1" identified by these competition assays ("competitive binding agents") include antibodies, antibody fragments, peptides, antisense oligonucleotides, small molecules and other binding agents that bind to an epitope or binding site bound by the reference antibody fragment, as well as a "molecule binding specifically to neuropilin-1" that bind to an epitope or binding site sufficiently proximal to an epitope bound by the reference antibody fragment. Preferably, competitive binding agents of the invention will, when present in excess, inhibit specific binding of a reference antibody fragment to a selected target species by at least 10%, preferably by at least 25%, more preferably by at least 50%, and even more preferably by at least 75%-90% or greater.

In addition to a polypeptide of the invention, particularly a modified antibody fragment or a modified antibody of the invention, natural or artificial ligands, peptides, anti-sense, or other small molecules capable of specifically targeting human neuropilin-1 may be employed. Drugs can be designed to bind or otherwise interact and inhibit human neuropilin-1 based upon the present invention. In this regard, rational drug design techniques such as X-ray crystallography, computer-aided (or assisted) molecular modeling (CAMM), quantitative or qualitative structure-activity relationship (QSAR), and similar technologies can be utilized to focus drug discovery efforts. Rational design allows prediction of molecules, which can interact with proteins or specific parts thereof. Such molecule structures can be synthesized chemically and/or expressed in biological systems. Small molecules may be produced by synthesizing organic compounds according to methods that are well known in the art. A plurality of peptides, semi-peptidic compounds or non-peptidic, and organic compounds may be synthesized and then screened in order to find compounds, which bind to neuropilin-1 with high neutralizing capacity. Particularly compounds that inhibit neuropilin-1 related invasion. See generally Scott and Smith, "Searching for Peptide Ligands with an Epitope Library", Science (1990), 249, 386-90 and Devlin et al., "Random Peptide Libraries: A Source of Specific Protein Binding Molecules", Science, (1990), 249, 40407.

The present invention also provides methods of using the modified antibody or modified antibody fragments to inhibit human neuropilin-1 activity or to detect human neuropilin-1 in cancer cells, preferably sarcoma cells, either in vitro or in vivo. In a preferred embodiment, treating cells expressing the antigen with one or more modified antibody fragments causes or leads to a reduction or inhibition of the invasive or adhesive abilities of human cancer cells, particularly of human sarcoma cells.

The migration of tumor cells into tissue is an important step in metastasis. The processes of adhesion and invasion can be studied in the transendothelial model (See, Woodward et al. (2002) Invest Ophthalmol Vis Sci 43, 1708-14 and Vachula et al. (1992) Invasion Metastasis 12, 66-81). The transendothelial model provides a useful ex vivo, e.g. an *in vitro,* system for the investigations of cellular interactions during the invasion process.

The present invention therefore further provides an ex vivo, e.g. an *in vitro* method to determine the dependency of the invasiveness of a naturally occurring invasive cancer cell on the functionality of neuropilin-1. This method comprises the steps of:
a. contacting the cells with a molecule inhibiting neuropilin-1 function;
b. contacting the cancer cell with a gel-like matrix, under conditions suitable for the growth of said cancer cells; and
c. determining the migration of said cancer cells through the gel-forming matrix.

The term "gel-like matrix" as used herein is understood to be a semi-solid substance with a water content of at least 90%, which allows cultivation of cancer cells in contact with the matrix and allows migration of invasive cancer cells through a slab of said "gel-like matrix" of 0,1 mm to 1 mm, preferably 0,3mm thickness, but not migration of non-invasive cells. Examples for such a "gel-like matrix" are substances resembling the extracellular matrix in protein and carbohydrate composition, particularly the commercially available "Matrigel". Particularly the "gel-like matrix" comprises one of the proteins selected from the group consisting of the proteins collagen type IV, fibronectin and laminin. More particularly the gel-like matrix comprises the proteins collagen type IV, fibronectin and laminin. More preferably the gel-like matrix comprises the proteins collagen type IV, laminin, entactin, nidogen and heparan sulfate proteoglycans or collagen type IV, fibronectin, laminin, nidogen, entactin, and vitronectin.

In a preferred embodiment the interfering molecule of step a) is a polypeptide that specifically binds to an extracellular epitope of neuropilin-1, particularly a modified polypeptide of the invention, more particularly a modified antibody or a modified antibody fragment of the invention, still more particularly a modified antibody fragment, even more particularly a modified scFv, dsFv, Fv, single domain antibody or diabody, especially a modified scFv, single domain antibody or diabody and even more preferably a modified scFv.

In another preferred embodiment step a) of said method comprises the use of an antisense oligonucleotide against neuropilin-1, siRNA or siRNA-like hairpin RNA against neuropilin-1 or a vector leading to cellular presence of siRNA against neuropilin-1 or siRNA-like hairpin RNA against neuropilin-1. Particularly preferred is the use of siRNA or siRNA-like hairpin RNA against neuropilin-1 or a vector leading to cellular presence of siRNA against neuropilin-1 or siRNA-like hairpin RNA against neuropilin-1.

The present invention therefore further provides an ex vivo, e.g. an *in vitro* method to determine the dependency of the adhesiveness of a naturally occurring invasive cancer cell on the functionality of neuropilin-1. This method comprises the steps of:
a. contacting the cells with a molecule inhibiting neuropilin-1 function;
b. contacting the cancer cell with a layer of ECM proteins, under conditions suitable for the growth of said cancer cells; and
c. determining the adhesion of said cancer cells to the layer of ECM proteins.

The term ""layer of ECM proteins" as used herein is understood to be a semi-dry layer of a protein solution, which allows cultivation of cancer cells in contact with the layer and allows attachment of invasive cancer cells to said layer. The thickness of said ""layer of ECM proteins" is between 0,1 mm to 1 mm, preferably 0,3mm thick. Examples for "ECM proteins are substances of the extracellular matrix. Particularly "ECM proteins are selected from the group consisting of the proteins collagens, entactin, nidogen, vitronectin, fibronectin and laminins. More particularly ECM proteins are selected from collagen S type I, collagen type IV, fibronectin and laminin.

In a preferred embodiment the interfering molecule of step a) is a polypeptide that specifically binds to an extracellular epitope of neuropilin-1, particularly a modified polypeptide of the invention, more particularly a modified antibody or a modified antibody fragment of the invention, still more particularly a modified antibody fragment, even more particularly a modified scFv, dsFv, Fv, single domain antibody or diabody, especially a modified scFv, single domain antibody or diabody and even more preferably a modified scFv.

In another preferred embodiment step a) of said method comprises the use of an antisense oligonucleotide against neuropilin-1, siRNA or siRNA-like hairpin RNA against neuropilin-1 or a vector leading to cellular presence of siRNA against neuropilin-1 or siRNA-like hairpin RNA against neuropilin-1. Particularly preferred is the use of siRNA or siRNA-like hairpin RNA against neuropilin-1 or a vector leading to cellular presence of siRNA against neuropilin-1 or siRNA-like hairpin RNA against neuropilin-1.

For the remainder of the document the following definitions shall apply as well, unless otherwise indicated.

As used herein "amplifiable ligand-displaying units" (ALDUs) are molecules or collections of molecules with dual function: they can bind to a target cell via a ligand; and they carry physically associated with the ligand an information about its identity, which allows individual units to be identified, and amplified. Examples of ALDUs are oligonucleotides, particularly RNAs, useful for the process of selection-amplification (SELEX), phages of phage displa libraries, viruses of viral display libraries, ribosomes of ribosome-display techniques, but also individualized beads carrying an identifiable molecule or ligand, particularly chemical entities, e.g. small molecules bound to beads, which are recognizable by inert chemical tags. Preferred are such ALDUs, which comprise a nucleic acid as the identifiable component. Such ALDUs can be either amplified *in vitro,* e.g., by nucleic-acid amplification methods like RT-PCR, PCR, LCR and the like, or they can be amplified in vivo, for example an individual phage can infect a bacterium and yield, after several cycles of infection, millions of virtually identical progeny. A library of ALDUs is a collection of similar, but in general non-identical ALDUs, for example phages of a phage library that display different scFvs in the context of an otherwise identical phage surface.

A ligand "derived from an ALDU" as mentioned herein is a ligand, which had been displayed by such an ALDU, which had been selected by binding to the surface of a target cell. As an example, if the ALDU is a phage of a phage library displaying scFvs the "ligand derived from an ALDU" in this case is a polypeptidee, here a scFv. As another example, in the case of ribosomal display the ligand "derived from an ALDU" is the polypeptide, which was presented by the complex comprising the ribosome, the RNA and the polypeptide.

As used herein, the term "associated with the surface of a cell" means that a biomolecule is either part of the surface of a cell, e.g. a constituent of the surface, or is binding to another biomolecule which is a constituent of the surface of a cell. For example, a transmembrane polypeptide, like a transmembrane receptor of an eukaryotic cell can be seen as a constituent of the surface if at least one part of it transverses the lipid bilayer of the cell membrane and is in direct contact with the lipids of the cell membrane. An example for a polypeptide that is binding to another biomolecule, which is a constituent of the surface of a target, is, for example, the β2-microglobuline of the class 1 major histocompatibility complex. In this case the β2-microglobuline binds non-covalently to the a chain of the MHC - a transmembrane polypeptide -, and is therefore also regarded as being a molecule associated with the surface.

As used herein "contacting" of two components means allowing the two components to physically associate with one another and bind to one another.

"Separating" as used herein means the physical separation of two or more components, for example a cell with an ALDU bound thereto can be separated from a free ALDU by centrifugation, wherein the cell with the ALDU bound thereto is pelleted and the free ALDU is still in the supernatant.

"Amplifying" as used herein is any process that increases the number of ALDUs or ligands derived from ALDUs by at least a factor of two, preferably by a factor of 10, 100, 1.000, 10.000, 100.000, 1.000.000, 10.000.000 or even a billion. For example a single phage can be amplified by infecting a bacterium and the infected bacterium then produces several mostly identical copies of the infecting phage, or a DNA-molecule can be amplified by the process of PCR to yield 10⁴ or more mostly identical daughter DNA-molecules.

"Identifying" as used herein means locating or recognizing an individual component, for example an ALDU, with special properties and isolating said individual component.

"Determining the chemical identity of a ligand" as used herein means determining the structural composition of a ligand. For example, if the ALDU library is a phage library displaying scFvs, then "determining the chemical identity of a ligand" would mean to determine the sequence of the scFv polypeptide, for example by sequencing the region encoding the scFv of the phage from which it was derived.

"A screening assay" as used herein is aimed at detecting an individual, e.g. an ALDU, with special properties among other similar individuals with slightly different properties. In order to qualify for a screening assay at least 3, 4, 5, 6, 7, 8, 9, 10, 20, 30, 40, 50 or even 100 individuals must be tested in a certain functional assay to find an individual/individuals with the desired function detected among them.

The invention relates to a method of identifying a ligand binding specifically to the extracellular region of neuropilin-1, wherein said ligand is capable of affecting a biological function, like adhesiveness and invasiveness, of neuropilin-1, comprising the steps of
a) contacting a library of amplifiable ligand-displaying units (ALDU) with a cancer cell, e.g. a sarcoma cell;
b) separating said cancer cell and the ALDUs bound thereto from ALDUs not bound to said cancer cell;
c) amplifying the ALDUs bound to said cancer cell;
d) identifying an ALDU or a ligand derived from an ALDU after step c), which affects said biological activity of neuropilin-1 by a functional screening assay;
e) identifying an ALDU or a ligand derived from an ALDU after step d) capable of binding to neuropilin-1
and optionally f) determining the chemical identity of the ligand. The preferred order of the steps is a, b, c, d, e and optionally f.

The method advantageously combines a screening step based on binding to the surface of a sarcoma cell with a screening step based on a functional assay. A library of amplifiable ligand-displaying units (ALDU) is brought in contact with a sarcoma cell in such a way that those ALDUs of the library with specificity for a surface antigen of the sarcoma cell can bind to the sarcoma cell. For example phages of a phage library displaying antibodies or antibody fragments can be allowed to bind to cultured sarcoma cells or, as another example, RNA of an RNA-oligonucleotides library can be allowed to bind to a sarcoma cell.

In another step, the sarcoma cell and the ALDUs bound thereto are separated from unbound ALDUs, in order to select for those ALDUs with specificity for the sarcoma cell. The separation can be achieved, e.g., by removing the solution, in which the contacting step has been performed from cultured sarcoma cells grown adhesively in a culture flask and together with the solution the unbound ALDUs; or, as another example, sarcoma cells with RNAs of a SELEX library bound thereto can be pelleted by centrifugation, while the unbound RNAs remain in the supernatant as part of the solution, in which the contacting step had been performed.

As another step the ALDUs bound to the target are amplified, in order to obtain high enough concentrations of the ALDUs so that they are useful for being applied in a functional screen. This amplifying step makes use of the ALDUs' intrinsic property to be amplifiable. For example phages displaying, e.g., an antibody or antibody fragment, which have been separated from the non-bound phages in step b), but are still bound to the sarcoma cell, can be amplified by first recovering the bound phages, for example by eluting the phages from the sarcoma cells and then using the recovered phage eluate to infect bacteria, e.g. *Escherichia coli.* The amplification of the phages in *Escherichia coli* then leads to a significant increase in total phage number, but in such a way, that the concentration of those individual phages, which were capable of binding to the sarcoma cells, is dramatically increased.

However, the phages need not necessarily be removed from the target cell, as a phage still bound to a target cell can infect added bacteria and thus be amplified.

As another example, the mRNA of a ribosomal-display library, which is associated by its attachment to a ribosome displaying the polypeptide corresponding to the mRNA, with the surface of a sarcoma cell, can be directly used for RT-PCR, without the need of removing the intact ALDU from the target before amplifying the target. Thus, a step in which the ALDU bound to a target is recovered from the target before amplifying the ALDU is an optional step.

Amplifying the ALDU can also consist in determining the chemical identity of the ligand of such ALDUs, which have bound to the sarcoma cell and then amplifying the ligands. That is to say, e.g., that in the case of a phage library, displaying an antibody or an antibody fragment, DNA encoding those ligands can be cloned from phages, which had bound to the sarcoma cell, e.g., by PCR, and the ligand, e.g., the antibody or antibody fragment, can then be produced recombinantly to yield sufficient amounts of the ligand to be useful for a functional screening assay. In such a way, not the ALDU itself is amplified, but the ligand displayed by the ALDU. This, however, is within the scope of the invention, as the aim of the amplification step is the generation of a ligand concentration high enough to be useful for a functional screening assay and this can be achieved by either amplifying the ligand or the ALDU displaying it.

In another step, an ALDU or a ligand derived from an ALDU, which had bound to a sarcoma cell is detected based on its effects on a biological function of neuropilin-1 to be tested in a functional screening assay. For such a functional screening assay, the ALDUs or ligands derived from them are advantageously individualized, such that a signal or a pattern from the functional screening assay is relatable to an individual ALDU or an individual type of ligand. This can be done, e.g., by filling separate wells of a multi-well plate with individual ligands each and then performing an assay for a desired biological function of neuropilin-1 in those individual wells.

In another example phages, which had bound to a sarcoma cell, and had been separated from unbound phages, can be individualized by infecting bacteria and plating the infected bacteria in, e.g., soft agar, so that individual plaques, which represent clones of individual phages, form. Those individual plaques can then be tested for their effects in a biological screening assay for neuopilin-1 function. In such an assay, an individual plaque with an effect in the functional screening assay for neuropilin-1 function is identifiable. The ligand displayed by the phages of the plaque can then be identified, e.g. by sequencing the DNA of the phages of the plaque, which represent a clone of a single phage, initially selected by its ability to bind to the sarcoma cell. Thus, in those cases where an ALDU itself is used in the functional screening assay, the identity of the ligand displayed by the ALDU can be determined, if desired.

In a preferred embodiment the ligand is capable of inhibiting a biological function of neuropilin-1. For example, the biological function of a neuropilin-1 can be invasion, adhesion, or angiogenesis. Adhesion and invasion have been explained as adhesiveness and invasiveness before.

"Angiogenesis" is the process where cells induce blood vessel formation in their proximity. Angiogenesis can accompany the growth of malignant tissue and can therefore be a property of malignant cells. That is to say that malignant cells can have the property of inducing angiogenesis.

Preferably, the biological function inhibitable by a ligand, e.g. an antibody or antibody fragment, identified by the method of the invention is the adhesiveness and invasiveness of a sarcoma cell.

In a preferred embodiment of the invention, the ligand is a polypeptide, in particular an antibody or antibody fragment. For example, if the ligand is an antibody it can be selected from the group consisting of IgA, IgG, IgD, IgE and IgM, and particularly from the group consisting of human IgG and IgM. If the ligand is an antibody fragment it can be selected from the group consisting of scFv, dsFv, Fab', Fab, F(ab')₂, Fv, single domain antibodies and diabodies.

In another preferred embodiment of the invention the ligand is selected from the group consisting of protein scaffolds with a tertiary structure, random polypeptides, small molecules, RNA-aptamers and DNA-aptamers. Protein scaffolds with tertiary structure are ligands obtainable by using a gene encoding a polypeptide with a defined tertiary structure, wherein one or more regions of the gene encoding the polypeptide can be replaced by randomized oligonucleotides or randomized oligonucleotides can be inserted into the gene encoding the polypeptide without changing the tertiary structure of the encoded polypeptide. Examples for these ligands are e.g. Anticalin, Affibody, cystein knot or protein scaffolds with an immnoglobulin fold. Anticalin is described in Beste et al (1999) Proc Natl Acad Sci USA. 96, 1898-903 and ALDUs displaying Anticalin are described in Beste et al, see supra. An Affibody is a disulfide bond-independent three-helix bundle scaffold Z, derived from domain B of Staphylococcal protein A and ALDUs displaying Affibodies are described in Hansson et al (1999) Immunotechnology 4, 237-52. A Cystein knot is a protein scaffold involving interlocking disulfide bonds as exemplified by the structure of conotoxins and nerve growth factor. A cystein knot is described in McDonald et al (1991) Nature 354, 411-4 and ALDUs displaying Cystein knots are described in Smith et al. J Mol Biol. 1998, 277, 317-32. ). Protein Scaffolds with the immunoglobulin fold are antibodies and antibody fragments and other polypeptides having the tertiary structure of the immunoglobulin superfamily and ALDUs displaying protein scaffolds with the immunoglobulin fold are disclosed in McCafferty et al. (1990) Nature 348, 552-4. ALDUs displaying random polypeptides are disclosed in Scott et al. (1990) Science, 249, 386-90. RNA-aptamers are structural mimics composed of ribonucleic acid and are disclosed in Tuerk and Gold (1990) Science 249, 505-10, and Tuerk et al., (1992) Proc. Natl. Acad. Sci. USA 89, 6988-92. DNA-aptamers are structural mimics composed of deoxyribonucleic acid and ALDUs displaying DNA-aptamers are disclosed in Bock et al (1992) Nature 355, 564-6.

This makes clear that the nature of the ligand displayed on the ALDU is not critical, but the ability of the ALDU to be identifiable and amplifiable.

However, as explained before, amplification of the ALDU can be achieved by increasing the number of an individual ALDU, but also by increasing the number of a ligand displayed by an individual ALDU. Since this can in principle also be achieved by an individual bead displaying a small chemical in combinatorial chemical libraries (see e.g., Ohlmeyer et al. (1993) Proc. Natl. Acad. Sci USA 90, 10922-26; Liu et al. J. Am. Chem. Soc. (2002) 124, 7678-80; Liang et al Science (1996) 274, 1520-2) such bead-format libraries are also libraries of ALDUs in a way. Small molecules can be synthesized on microsphere beads, which can be indexed with inert chemical tags. During each step of the synthetic scheme a tag molecule that encodes the step number and the chemical reagent used in that step is attached to the bead. This array of tags can then be used as a binary code to record the reaction history of each bead. The identified compound can then easily be synthesized in a bigger scale.

However, preferably the ALDU is a biological ALDU that is the information about its identity is stored as a nucleic acid, e.g. RNA or DNA. In a preferred embodiment the ALDU is selected from the group consisting of viruses useful for viral display, phages useful for phage display, bacteria useful for bacterial display, ribosomes useful for ribosome display, yeasts useful for yeast display and oligonucleotides useful for selection and amplification. Viruses useful for viral display can be, e.g., plant viruses, viruses of fungi or animal viruses, in particular viruses that can be obtained from cell culture, are amenable to genetic engineering techniques and can display a foreign ligand on their surface. Viruses useful for viral display on retroviruses are disclosed in Russell et al., (1993) Nucleic Acids Res. 21, 1081-5.

Viruses useful for display by plant viruses, e.g. the Cowpea mosaic virus are disclosed in Brendan et al., (1999) Microbiology 145, 211-20.

Phages useful for phage display can be all phages that can be obtained from culture, are amenable to genetic engineering techniques and are capable of displaying a foreign ligand on their surface. Phage display has been disclosed in Smith et al. (1985) Science, 228, 1315-17, phage display of an antibody has been disclosed in WO 91/17271.

Bacteria useful for bacterial display are bacteria, which can be kept in culture, are amenable to genetic engineering techniques and are able to display a ligand on their surface. Examples for bacteria useful for bacterial display are disclosed in Dougherty et al., (1999) Protein Eng. 12,613-21; and Westerlund-Wickstrom B. (2000) Int. J. Meth. Microbiol. 290, 223-30.

Ribosomes useful for ribosomal display have been disclosed in Shaffizel et al., (1999) J. Immunol. Methods 231, 119-35; and Willson et al., (2001) Proc. Natl. Acad. Sci. USA 98, 3750-5.

Oligonucleotides useful for selection/amplification (Selex) have been disclosed in Tuerk and Gold (1990) Science 249, 505-10, and Tuerk et al., (1992) Proc. Natl. Acad. Sci. USA 89, 6988-92.

However, also lower eukaryotes which can be cultured and are amenable for genetic engineering techniques and able to display a foreign ligand on their surface are useful as ALDUs, e.g. genetically modified yeast, as disclosed in Boder and Wittrup (2000) Methods Enzymol. 328,430-44.

In another preferred embodiment the ALDU library is selected from the group consisting of immune or naive libraries, in particular wherein said naive library is a synthetic, semi-synthetic or natural library. This relates particularly to the case wherein the ligand displayed on the ALDU is an antibody or an antibody fragment. In those cases, one can distinguish between immune or naive ALDU libraries. In the case of immune libraries the library members, e.g. antibody fragment genes, can be obtained from lymphocytes, e.g. B lymphocytes, of immunized animals. In the case of naive libraries the library members have not gone through the process of an active immune response. Depending on the way of its production, the naive library can be, e.g., a semi-synthetic library, in which library members are obtained by use of germline V-genes as starting material. The regions coding for one or several V-gene CDRs, encoded e.g. by CDR1 and CDR2 can be replaced by randomized oligonucleotides, or randomized oligonucleotides can be added to one or several CDRs, either at the beginning, as an insert into the CDR, or at the end of the CDR, and regions coding for one or several of the non V-gene encoded CDRs e.g. CDR3 can be added to the V-gene as randomized oligonucleotides.

Semi-synthetic libraries are disclosed in Williamson et al (1993) Proc. Natl. Acad. Sci. U S A. 90,4141-5.

An antibody-based naive libraries can also be synthetic libraries, e.g. in which library members are obtained by use of non-germline V-genes as starting material. The regions coding for one or several V-gene CDRs encoded e.g. by CDR1 and

CDR2 can be replaced by randomized oligonucleotides, or randomized oligonucleotides can be added to one or several CDRs, either at the beginning, as an insert into the CDR, or at the end of the CDR, and regions coding for one or several of the non V-gene encoded CDRs e.g. CDR3 can be added to the V-gene as randomized oligonucleotides (Griffiths et al (1994) EMBO J. 13, 3245-60; Knappik et al. J. Mol. Biol. 296, 57-86).

A naive library can be a natural library wherein the library members e.g. antibody fragment genes, can be obtained from lymphocytes, e.g. B-lymphocytes, of non-immunized donors. An example of a natural library is disclosed in Griffiths et al. see supra.

In another preferred embodiment of the invention, the ALDUs are derived from viruses and phages, in particular from Cowpea mosaic virus, retroviruses, lambda phage, T7 phage, T4 phage and filamentous phages, more particularly derived from Ff phages. An ALDU library derived from Cowpea mosaic virus is disclosed in Brendan et al. (1999) Microbiology 145, 211-20. An example of an ALDU library derived from a retrovirus is disclosed in Russell et al. (1993) Nucleic Acids Res. 21, 1081-5.

An example of an ALDU library derived from lambda phage is disclosed in Maruyama et al. Proc. Natl. Acad. Sci. U S A. (1994) 91, 8273-7. An example of an ALDU library derived from T7 phage is disclosed in Danner et al. Proc. Natl. Acad. Sci. U S A. (2001) 98, 12954-9. An example of an ALDU library derived from T4 phage is disclosed in Efimov et al. Virus Genes (1995) 10, 173-7. Often filamentous phages have been used as the basis for ALDU libraries (Phage Display of Peptides and Proteins: a laboratory Manual edited by Brian K. Kay, Jill Winter, John McCafferty.Academic Press 1996). The Ff phages of *Escherichia coli* have proven particularly useful as phages capable of displaying random and foreign ligands on their surface (Smith, Science (1985) 228, 1315-7). In those cases the ligand, e.g. a random polypeptide, is often displayed as a fusion protein with a phage protein, wherein the phage protein is derived from a phage surface protein, e.g. selected from the group consisting of pIII, pVIII and pVI. Phage display based on fusion proteins with pIII are disclosed in Smith, Science. (1985) 228, 1315-7. Phage display based on fusion proteins with pVIII are disclosed in Gram et al Proc. Natl. Acad. Sci. U S A (1992) 89, 3576-80. Phage display based on fusion proteins with pVI are disclosed in Jespers et al. Biotechnology (N Y). (1995) 378-82.

In another preferred embodiment step d) of the method of identifying a ligand of the invention comprises detecting an ALDU or a ligand derived from an ALDU, which affects the biological function of neuropilin-1 by a functional screening assay, wherein the functional screening assay applied for detecting the ALDU or a ligand derived from an ALDU is selected from the group consisting of an invasion assay, an adhesion assay or an angiogenesis assay. Preferred are an invasion assay and an adhesion assay.

An invasion assay measures the invasiveness of a cell. An invasion assay is, e.g., the assay performed in Example 8 and 9 of this application.

An adhesion assay measures the adhesiveness of a target, e.g. a cell, to something else, e.g. another cell, a virus, or a complex biological mixture like, e.g., a population of vehicles derived from a cell or, e.g., the basal lamina. An example of an adhesion assay is given in Example 11 and 12 of the present application.

An angiogenesis assay measures the ability of a cell to induce blood vessel formation, a process, which usually accompanies the growth of malignant tissue. An angiogenesis assay is disclosed in Kanda et al (2002) J. Natl. Cancer Inst. 94, 1311-9.

In another preferred embodiment the method of identifying a ligand binding specifically to the extracellular region of neuropilin-1 also comprises the step of incorporating the ligand identified into a therapeutic, prophylactic or diagnostic composition. This can, e.g., be done by mixing the identified ligand with a pharmaceutically acceptable carrier known in the art, wherein the ligand is present in an amount, which is therapeutically effective.

In another preferred embodiment the invention relates to a method for the production of a pharmaceutical composition, comprising the method of identifying a ligand binding specifically to neuropilin-1 and further the step of mixing the identified ligand, or a modified or a labeled version thereof, with a pharmaceutical acceptable carrier known in the art. A modified ligand is a ligand with a covalent modification. Examples of modified ligands are labeled ligands.

In a preferred embodiment of the invention, the separation step b) of the method of identifying a ligand of the invention is achieved by centrifugation, filtration, density centrifugation, FACS or immobilization onto solid support. For example an ALDU bound to a sarcoma cell can be separated from unbound ALDUs making use of the fact that the complex of an ALDU bound to a target has different biochemical properties compared to an unbound ALDU. Those changed biochemical properties can be size, specific binding, charge density, density or the like. Filtration can separate the bound ALDUs from the unbound ALDUs, e.g. if the size of the filter is such that ALDUs bound to the sarcoma cell are retained by the pores of the filter, while the unbound ALDUs can pass through the pores of the filter. Filtration is therefore useful if the target is large, like a sarcoma cell, and the ALDU is small, e.g. a phage, a virus, a nucleic acid or a ribosome displaying a ligand.

Density centrifugation separates biomolecules according to their density. Density centrifugation can be applied if the density of an ALDU bound to a target is different from that of an unbound ALDU. For example, the density centrifugation can be applied if an ALDU is dense, e.g. a ribosome displaying a ligand, and the target is not as dense, like a sarcoma cell or vehicles derived from the membrane of a sarcoma cell.

FACS can separate the complex of ALDU bound to a sarcoma cell from unbound ALDUs based on fluorescence of the target. FACS can be performed by labeling the sarcoma cell with a fluorescent dye and then passing the labeled sarcoma cell in suspending medium through a narrow dropping nozzle so that each sarcoma cell is in a small, separate droplet. A laser-based detector system is often used to excite the fluorescent dye and droplets with e.g. fluorescence-positive sarcoma cells are given an electric charge. Charged and uncharged droplets can then be separated as they pass charge plates and can be collected in two different tubes. In this way small droplets containing ALDUs that are bound to a sarcoma cell are separated from bulk solution containing unbound ALDUs.

Immobilization of the sarcoma cell onto solid support can be performed in order to separate ALDUs bound to the immobilized sarcoma cell from unbound ALDUs. Immobilization of the sarcoma cell onto solid support is often performed by using surface activated solid support or specific binding of the surface of the solid support with the sarcoma cell using e.g. biomolecules with specific binding properties towards the sarcoma cell. In order to separate ALDUs that are bound to the immobilized sarcoma cell from unbound ALDUs, the immobilized sarcoma cell is separated from the bulk solution containing unbound ALDUs.

Separation of ALDUs bound to the immobilized sarcoma cells from the bulk solution containing unbound ALDUs can be performed by removing the bulk solution containing the unbound ALDUs from the immobilized sarcoma cells. For example, if the sarcoma cell is immobilized onto e.g. the well of a plastic micro titer plate via covalent interactions then the bulk solution containing the unbound ALDUs can be removed by removing the solution and washing the wells with washing solution.

Separation of ALDUs bound to the immobilized sarcoma cell from the bulk solution containing unbound ALDUs can be performed by filtration if the size of the filter is such that the sarcoma cell is retained by the pores of the filter, while the unbound ALDUs can pass the filter.

In another preferred embodiment the amplification step c) of the method of identifying a ligand of the invention is achieved by either eluting the ALDU bound to a sarcoma cell, or by lysing a sarcoma cell under conditions where the ALDU remains amplifiable, and subsequent amplification. ALDUs bound to a sarcoma cells can be eluted from the sarcoma cells first, e.g. by use of solutions with low pH and/or high salt, as demonstrated in Example 2, and subsequently amplified, e.g. such that the eluted phages of Example 2, are used to infect *Escherichia coli* in order to produce large numbers of progeny phages. However, the elution step from the target, while it may be convenient, is not necessary, as, e.g., in the case of phage libraries even the phages still bound to a sarcoma cell, are capable of infecting *Escherichia coli* and thus capable of producing large numbers of identical progeny, and thus are amplifiable even when still bound to the sarcoma cell.

In one embodiment of the invention the ALDUs or the ligands derived from an ALDU which are used for the functional screening assay of step d) of the method of identifying a ligand according to the invention are chemically modified to increase their biological activity or endow the ALDU or the ligand with biological activity. As an example, one way to increase the biological activity of an ALDU or a ligand or to endow the ALDU or the ligand with biological activity is to use the ALDU or the ligand in combination with CALI (Chromophore-Assisted Laser Inactivation).

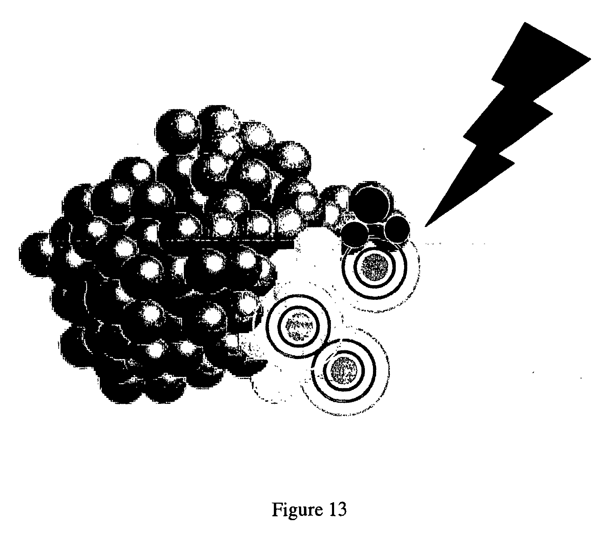

The principle of CALI is based on the local initiation of a photochemical reaction that leads to the generation of short-lived reactive species, which in turn selectively modify the target molecule and cause its functional inactivation. Highly specific but non-inhibitory ligands (e.g. antibodies, antibody fragments, small molecules) are labeled with a suitable chromophore (e.g. malachite green, fluorescein, methylene blue, eosin). After complex formation between the target molecule (e.g. proteins) and the ligand, the complex is irradiated with laser light of an appropriate wavelength to excite the chromophore. The excitation triggers a photochemical reaction that initiates the generation of short-lived reactive species (e.g. hydroxyl radicals or highly reactive oxygen species). These reactive species modify the protein within a small radius around their site of generation. The distance that a reactive species can travel is very short due to its short lifetime. Therefore, the modifications of amino acid residues within the protein occur in close proximity to the binding site of the ligand. The damaging effect is restricted to a radius of 15-40Å, which is well below the average distance of two proteins within a cell, which is at about 80Å, (assuming an average cytosolic protein concentration of 300mg/ml and an average protein size of 50kDa) ensuring a high spatial resolution of the process. This principle is shown in Figure 13. As an example, a modified antibody against a given protein often can selectively inhibit the function of that particular protein after CALI, even if this antibody did not show an inhibiting function without CALI. In cases where the binding site of the ligand is close or within an important functional domain of the protein, these induced modifications lead to permanent inactivation of the protein. The functional inactivation of the protein is measured in an appropriate readout assay and evaluated in the context of disease relevant physiological functions like cell invasion, cell adhesion, cell signaling or apoptosis.

Inactivation of proteins with CALI was shown to be very specific to the respective protein. Linden et al showed that β-galactosidase could be efficiently inactivated with a malachite-green labeled anti-β-galactosidase antibody even in the presence of alkaline phosphatase in the same solution. β-galactosidase was inactivated by 95 % after 10 min of laser irradiation whereas alkaline was not effected at all (Linden et al. (1992) Biophys. J. 61, 956-962). Jay also demonstrated that a dye-labeled antibody bound to a single epitope of a protein was sufficient to inactivate acetylcholinesterase (Jay (1988) Proc. Natl. Acad. Sci. USA, 85, 5454-58).

Henning et al. described that CALI was successfully used against a diverse array of proteins (Henning et al. Innovations in Pharmaceutical Technology (2002) 62-72). These protein include membrane proteins (eg. α-, β-, ε-chains of T cell receptor, β1 integrins, ephrin A5 or FAS receptor), signal transduction molecules (eg. calicineurin, cyclophillin A or PKC), cytoskeletal proteins (eg. actin, ezrin or kinesin) or transcription factors. Henning et al. further described that CALI can be used for identification of novel proteins as drug targets and at the same time the elucidation of their function in the biological context of interest (Henning et al. (2002) Current Drug Discovery May, 17-19).

Several application examples of CALI show that CALI is able to convert specific but non-inhibitory ligands into blocking reagents. Therefore, these ligands can be used to modulate the action of inhibitory ligands. CALI can also be used to further enhance the inhibitory effect of a ligand that has already an inhibitory effect by itself. Example 9 and Example 12 are examples where CALI is integrated in an invasion and an adhesion assay, respectively.

In another embodiment of the invention, the ALDU or the ligand derived from an ALDU and used for the functional screening assay of step d) of the method of identifying a ligand of the invention are unmodified.

The chemical modification of the ALDU or the ligand derived from an ALDU can be the addition of chromophores. A chromophore is that part of a molecule that possesses high optical activity due to mobile electrons that interact with light. Some examples of chromophores are, e.g., fluorescein derivatives, rhodamine derivatives, coumarin derivatives, porphyrin derivatives, phthalocyanine derivatives, naphthalocyanines derivatives eosin derivatives, triphenylmethane derivatives or acridine derivatives. A list of chromophores useful for chemically modifying biomolecules is disclosed in The Sigma Aldrich Handbooks of Stains and Dyes, Ed., F.J. Green (1990) ISBN No. 0-941633-22-5.

In another preferred embodiment of the invention, the method of identifying a ligand of the invention comprises as the identification step d) a spatial separation of the different ALDUs of a pool of ALDUs and then the screening of the spatially separated ALDUs for their effect on a biological function of neuropilin-1 in such a way that the result obtained can be assigned to an individual ALDU. Spacial separation of the different ALDUs of a pool of ALDUs can be done, e.g., by filling separate wells of a multi-well plate with individual ligands each and then use those multi-well plates in a functional screening assay. If, e.g., in one well the biological function of neuropilin-1 is inhibited, then a ligand inhibiting said biological function of neuropilin-1 is identified, as the experimenter knows the identity of each ligand he put into each individual well.

In another preferred embodiment the method of identifying a ligand according to the invention can comprise the step of determining the identity of the ligand, wherein this determination step is achieved by sequencing the DNA of an identified ALDU, taking a PCR fingerprint of the nucleic acid of an identified ALDU or also, in the case of a ligand derived from an ALDU, by mass spectrometry of such a ligand. PCR fingerprinting of, e.g., phages is disclosed in (Marks et al. J. Mol. Biol. (1991) 222, 581-97). Mass spectrometrical analysis of a ligand, e.g. a polypeptide, is disclosed in Shevchenko et al. (1996) Anal. Chem. 68, 850-58 or Spengler et al (1992) Rapid. Commun. Mass. Spectrom. 6, 105-8.

The method of identifying a ligand according to the invention may further comprise at least one additional step of screening ALDUs, e.g. wherein the screening is based on biochemical properties of the ALDUs. Such an additional screening step may comprise a method selected from the group consisting of flow cytometry, ELISA, immunoprecipitation, binding assays, immunohistochemical experiments, affinity studies, immunoblots and protein arrays. Biochemical properties can be determined by the size, the shape, the density, the charge density, the hydrophobicity, or the binding specificity of an ALDU or the ligand derived from an ALDU. The biochemical properties of the ALDU or the ligand derived from an ALDU form the basis of the applicability of the above-mentioned methods for screening the ALDUs. Flow cytometry is usually performed by labeling cells with a fluorescent dye and then passing those labeled cells in suspending medium through a narrow dropping nozzle so that each cell is in a small, separate droplet. A laser-based detector system is often used to excite the fluorescent dye and droplets with e.g. fluorescence-positive cells are registered.

Immunoprecipitation is a procedure by which a ligand, e.g. an antibody or antibody fragment, that reacts specifically with an antigen, e.g. an antigen associated with the surface of a eukaryotic cell, is used to remove said antigen from a solution, e.g. a cell lysate. The immunoprecipitate consisting of the ligand bound to the antigen can then be examined by utilizing biochemical methods like, e.g. 2D gel electrophoresis. In particular the identity of the antigen can then be determined by use of, e.g., mass spectrometry and it can be determined whether this ligand really binds to neuropilin-1.

In another preferred embodiment of the invention, the method of identifying a ligand of the invention can further comprise a substractive selection step. A substractive selection step is a step, which removes ALDUs with an undesired property. For example a substractive selection step can be affected by removing the ALDUs capable of binding to a control cell, if the property of binding to a control cell is undesired. By way of example, if one wants to select for ALDUs specific for cancer cells one could first select those ALDUs which bind to cancer cells, elute the bound ALDUs, e.g. phages, and then remove those ALDUs which are capable of binding to non-cancer-cells, by for example contacting the pool of eluted ALDUs with non-cancer-cells and removing those ALDUs bound to the non-cancer-cells. The ALDUs remaining in the supernatant are then ALDUs specific for cancer cells and can then be used in functional screening assays according to the method of identifying a ligand of the invention.

After step d) the pool of ALDUs, e.g. phages, is enriched in ALDUs binding to neuropilin-1. Those ALDUs binding to neuropilin-1 can finally be identified by numerous methods known in the art. ALDUs, e.g. phages, can be separated to form individual clones and the clones of the ALDUs, e.g. phages, can be probed with labeled neuropilin-1 protein, or a labeled part of the neuropilin-1 protein, e.g. an at least seven amino acid long peptide of the extracellular region of neuropilin-1. Clones binding to such a probe are identified as neuropilin-1-binders. ALDUs, e.g. phages, can also be affinity purified on purified neuropilin-1 protein or on recombinant neuropilin-1

Alternatively, the open reading frame encoding the ligand of the ALDU, e.g. an antibody or antibody fragment, can be recloned from the whole enriched pool into an expression vector, the ligand, e.g. an antibody or the antibody fragment can then be expressed in clones of another host cell, and the clone of the host cell carrying the expression vector comprising a nucleic acid encoding for the ligand, e.g. an antibody or the antibody fragment binding specifically to neuropilin-1 can be identified, e.g. by the method described above for the identification of relevant phage clones, e.g. by a method of the Examples, or by affinity purification on recombinant neuropilin-1.

A particular advantage of this method is that a ligand, e.g. an antibody or an antibody fragment specific for the accessible part of the extracellular region of neuropilin-1 is obtained, since the initial selection step is performed on intact cells, which present the accessible part of the extracellular region of neuropilin-1 for binding of the phages.

In a preferred embodiment of the invention, the above method comprises instead of steps e) and f) the further steps of:
e) contacting ALDUs, e.g. isolated phages with recombinant neuropilin-1;
f) washing said neuropilin-1 with a buffered detergent and/or high salt solution; and
g) eluting ALDUs, e.g. phages bound to neuropilin-1; and
h) determining the identity of the ligand, e.g. an antibody or antibody fragment represented by said eluted ALDU, e.g. a phage.

In certain embodiments, the ligand, e.g. antibody fragments expressed on the ALDU, e.g. phages comprise an antibody or an antibody fragment selected from the group consisting of scFv, dsFv, Fab', Fab, F(ab')₂, Fv, single domain antibodies (DABS) and diabodies, more particularly selected from the group consisting of scFv, dsFv, Fv, single domain antibody or diabody, still more particularly selected from the group consisting of scFv, single domain antibody or diabody and even more preferably a scFv.

The "detergent" used in steps c) and f) can be a detergent solution, preferably buffered, and can be Tween in a concentration of 0.001- 0.5%, particularly 0.01-0.1 %. "High salt" in step f) is a high salt solution, preferably buffered, and has an ionic strength of 10mM-1M, particularly 20-500mM, more particularly 50-350mM, even more preferably 80-250mM. Typical useful anions are, for example, chloride, citrate, phosphate, hydrogen phosphate or borate. Typical useful cations are, for example, sodium, potassium, lithium, calcium or magnesium.

The buffered solution in the above paragraph typically has a pH of 7-8. For example, DMEM or PBS, particularly with 1-20%, more particularly 5-15%, even more preferably about 10% FCS, can be used as buffers.

Isolation of cells with phages bound to them is effected by gentle centrifugation at g values from 200 to 300 for 3 to 20 minutes, particularly 5 to 10 minutes. Elution of bound phages, both to cells and to immobilized neuropilin-1, is effected by a wash with 2-100mM, particularly 4-50mM, more particularly 5-20mM, even more preferably around 10mM glycine at a pH of from 0 to 2.5, particularly from 1 to 2.5, more particularly from 1.5 to 2.5.

The neuropilin-1 inhibitory activity of these antibody fragments, particularly of these scFvs may be assayed as described above, in vivo or in a cell culture experiment. Cell culture assays include assays that determine the inhibitory effect of the antibody fragments of this invention in an invasion or adhesion assay as described in Examples 8 or 9 and Examples 11 or 12. Results of the invasion assay are provided in Fig 2. Results of the adhesion assay are provided in Figs. 3 and 4.

The following examples, including experiments conducted and results achieved, are provided for illustrative purposes only and are not to be construed as limiting upon the present invention.

### DESCRIPTION OF THE DRAWINGS

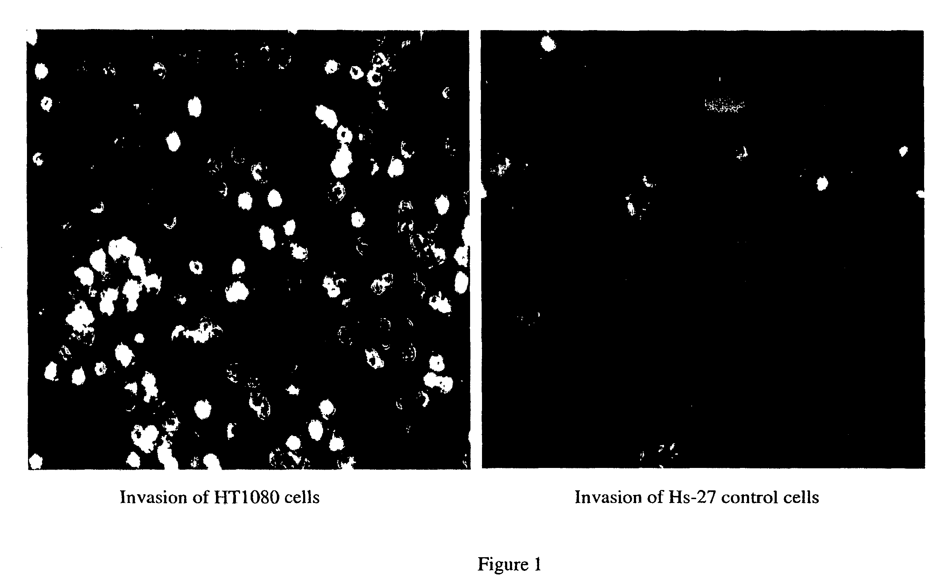

- Figure **1**: shows the invasion of stained HT1080 cells through a 8µm filter. Fluorescence was quantified after six hours incubation at 37°C. Data presented are the mean of n = 3 wells +/- SD.

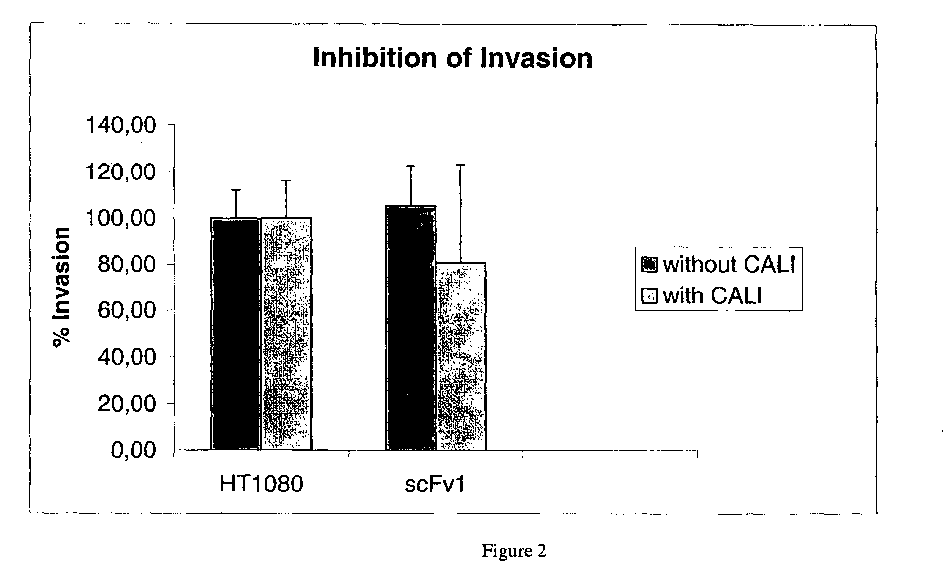

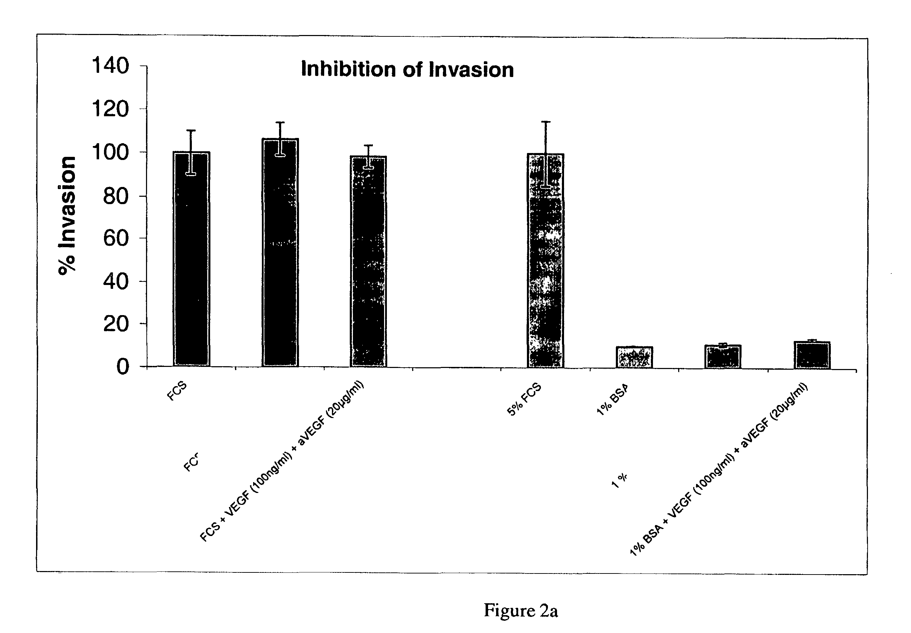

- Figure **2**: shows the inhibitory effect of scFv1 on the invasion of HT1080 cells. Invasion was determined in a chemotaxis assay with a Matrigel coated cell migration chamber. The invasion was determined after laser irradiation (with CALI) and without laser irradiation (without CALI). The invasion of HT1080 cells in the absence of any inhibitory molecule was used as a control (left bars).
- Figure **2a**: shows the influence of VEGF on the invasiveness of HT1080 cells. In vasion was determined in a chemotaxis assay with a Matrigel coated cell migration chamber.

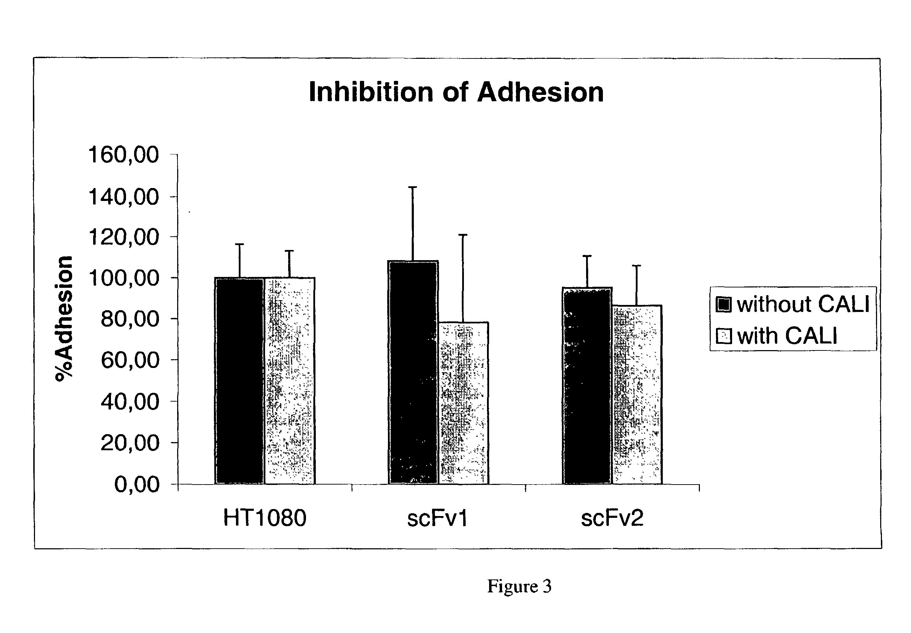

- Figure **3**: shows the inhibitory effect of scFv1 and scFv2 on the adhesion of HT1080 cells. Adhesion to collagen S type I was determined after laser irradiation (with CALI) and without laser irradiation (without CALI). The adhesion of HT1080 cells to collagen S type I in the absence of any inhibitory molecule was used as a control (left bars).

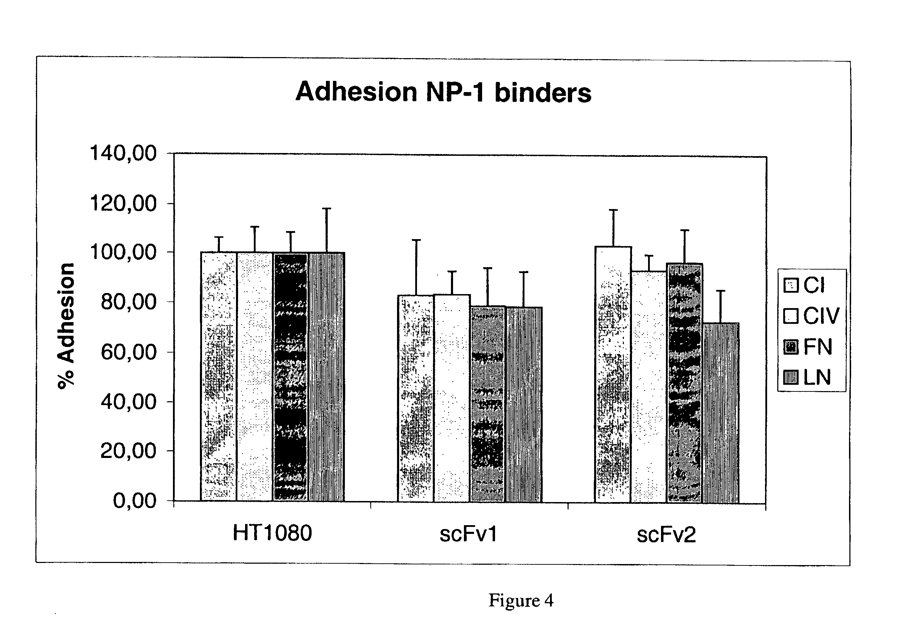

- Figure **4**: shows the inhibitory effect of scFv1 and scFv2 on the adhesion of HT1080 cells to different matrix proteins. The adhesion of HT1080 cells to the different matrix proteins in the absence of any inhibitory molecule was used as a control (left bars).

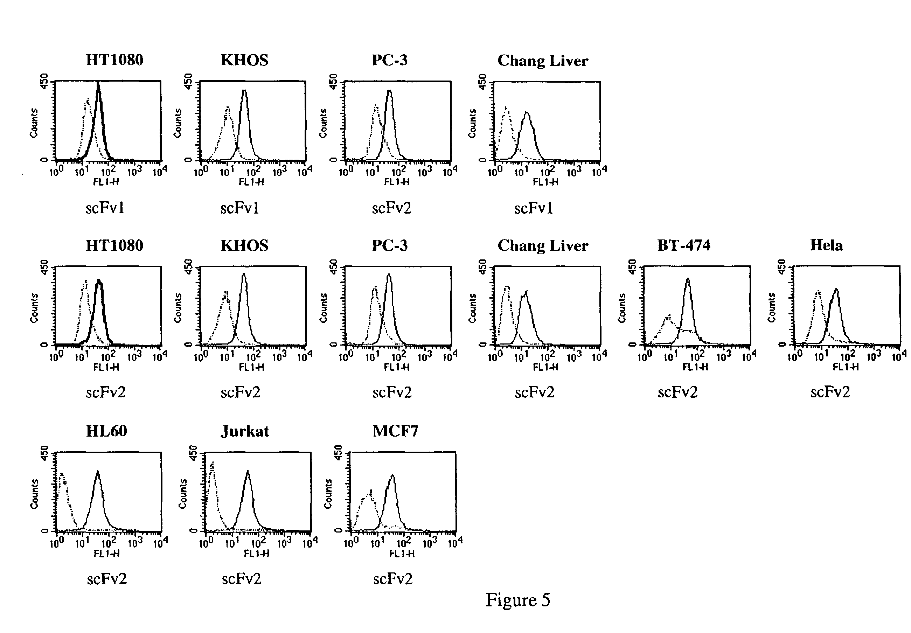

- Figure **5**: shows results of FACS analysis of scFv1 and scFv2 with different cells lines (bold line) and HS-27 cells (dotted line) as control.

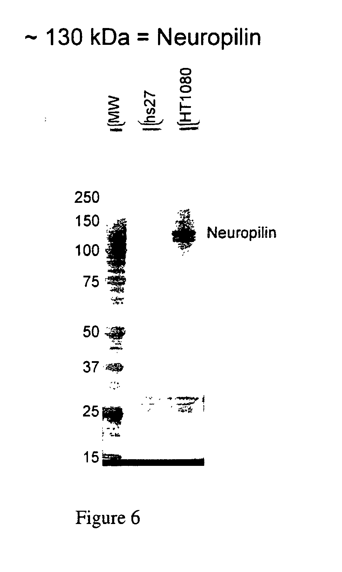

- Figure **6**: shows the result of the immunoprecipitation experiments. scFv1 was tested on HT1080 cells and compared to HS-27 cells as a control. The immuno-complexes were separated by SDS-PAGE and silver stained.

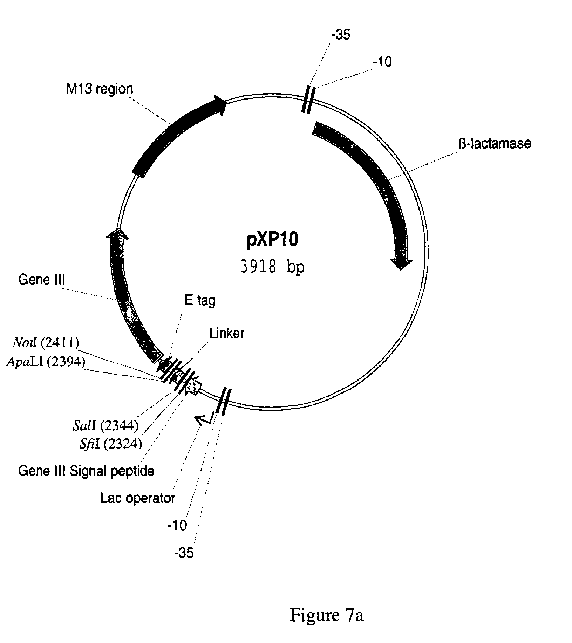

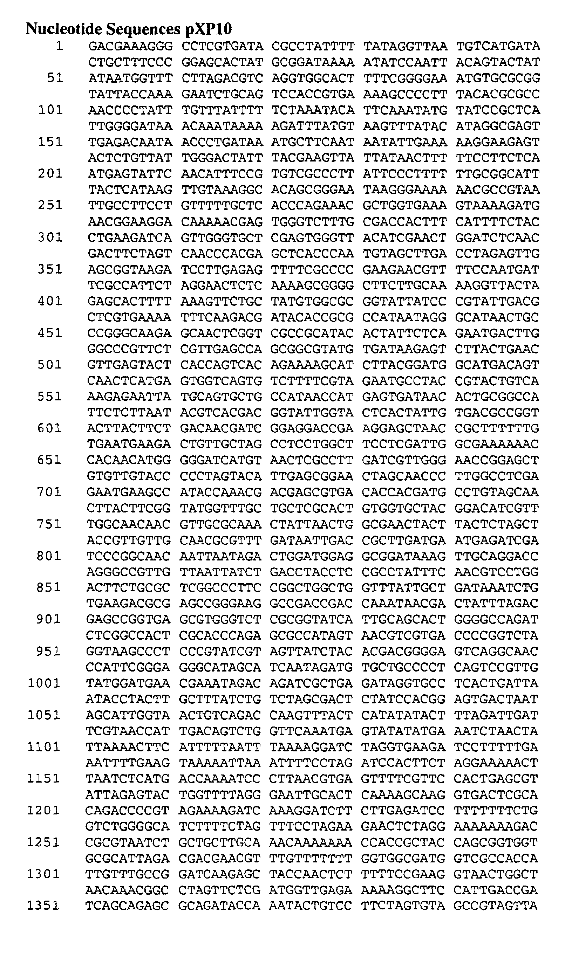

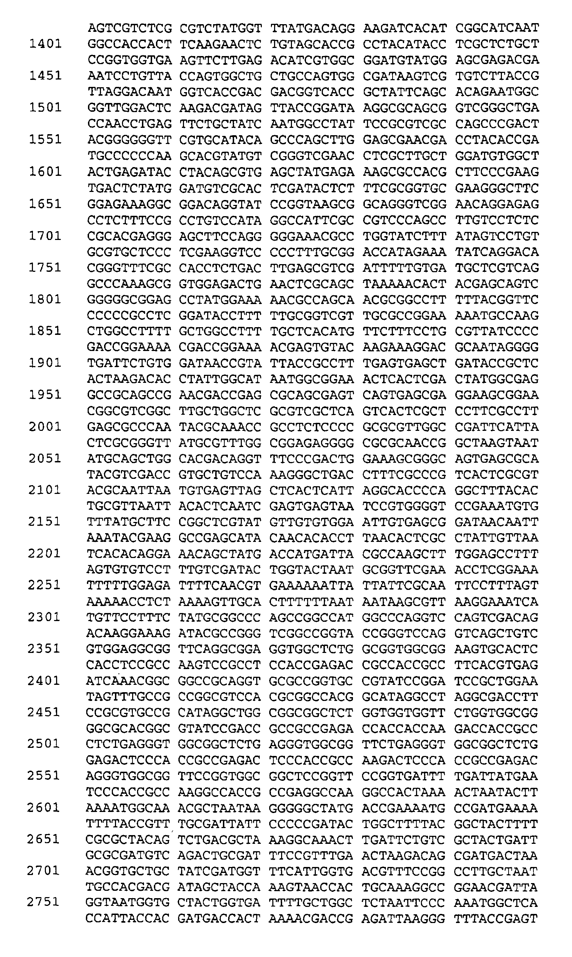

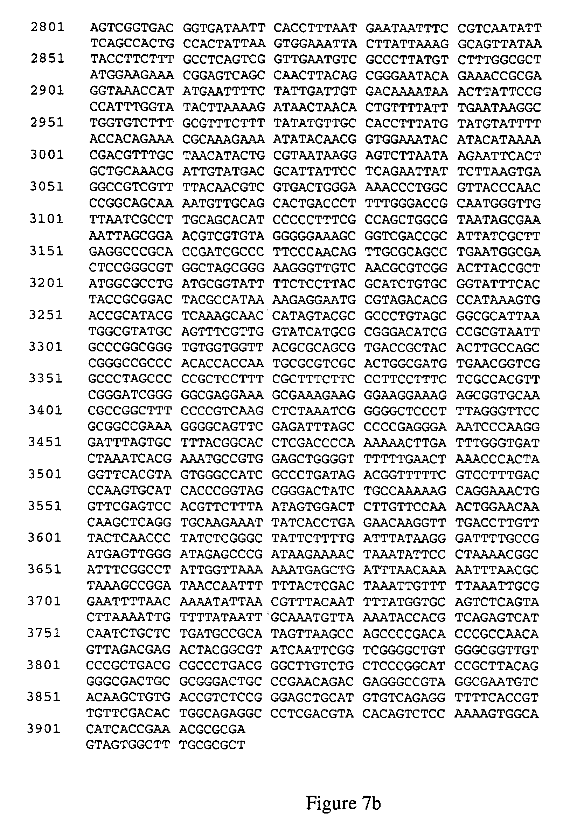

- Figure **7a** and **7b**: show the vector map and sequence of the scFv display vector pXP10.

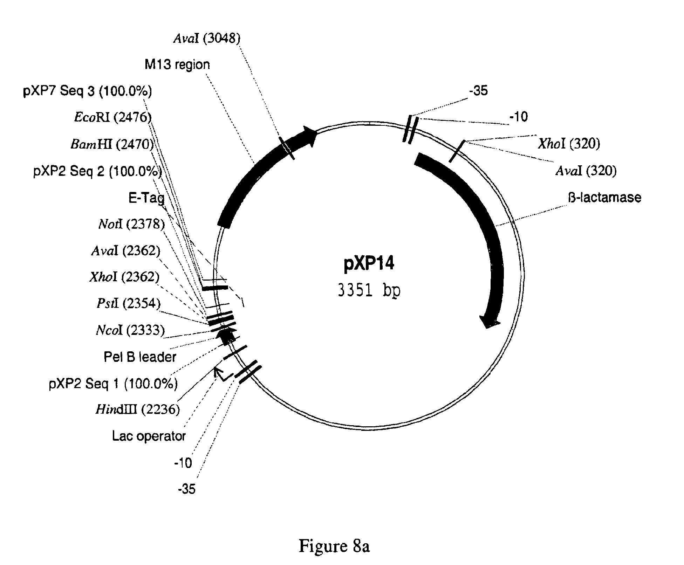

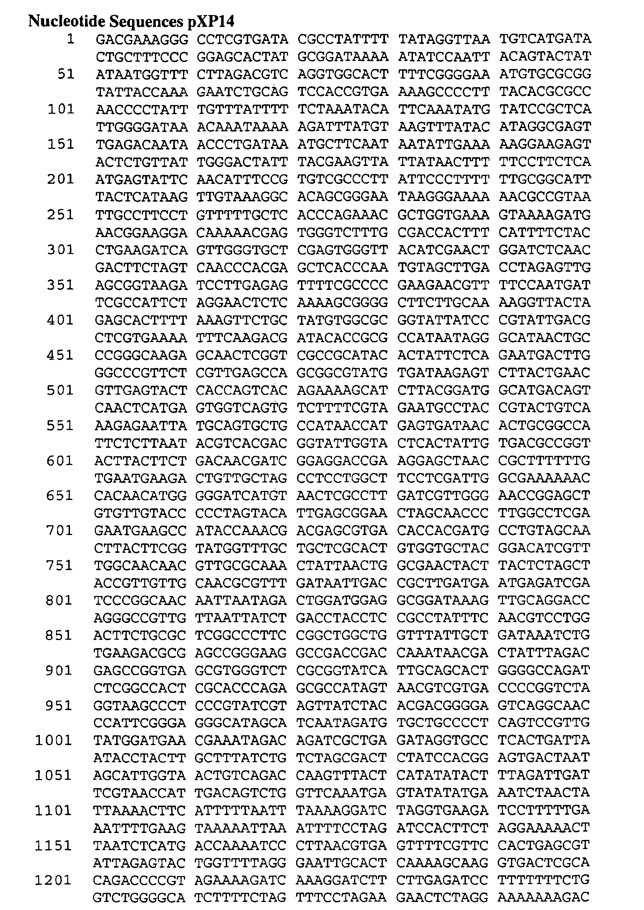

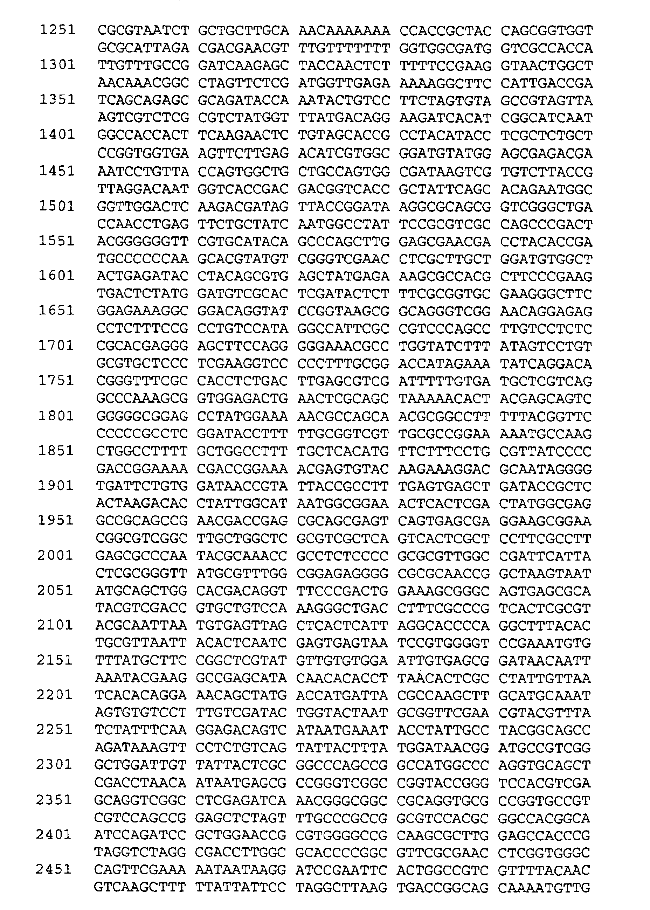

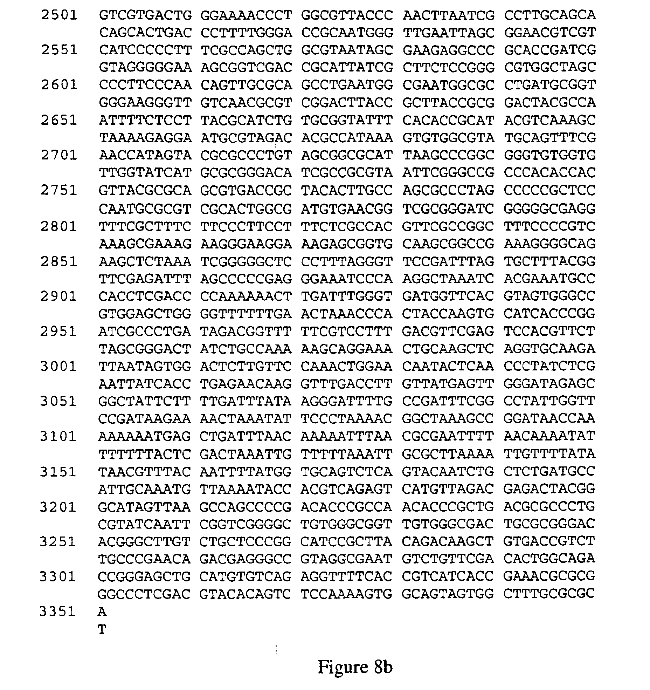

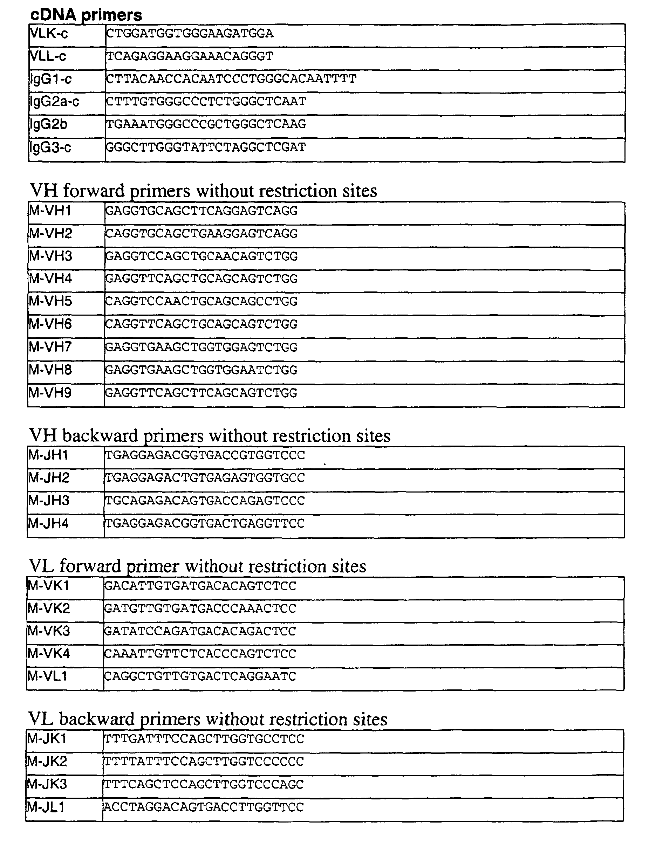

- Figure **8a** and **8b**: show the vector map and sequence of the scFv expression vector pXP14.

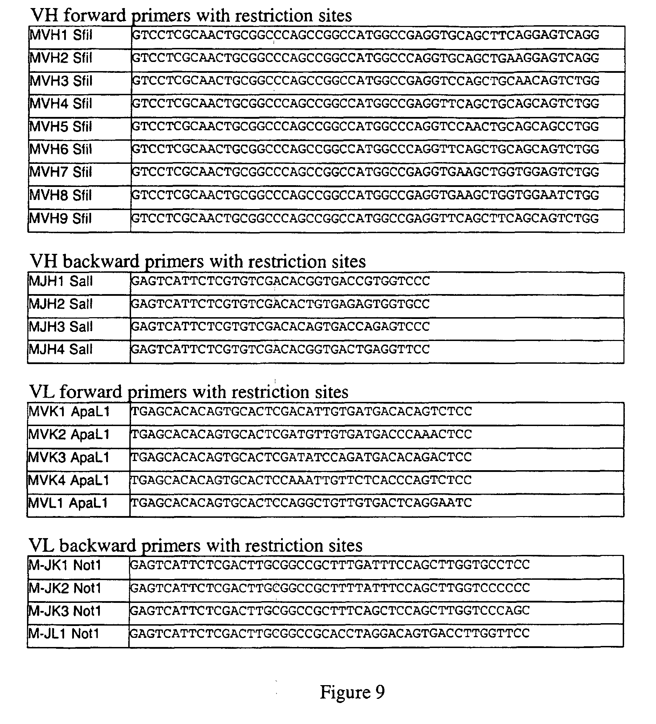

- Figure **9**: shows the sequences for the construction primers for a mouse library.
- Figure **10**: shows the amino acid sequences of scFv1 and scFv2.

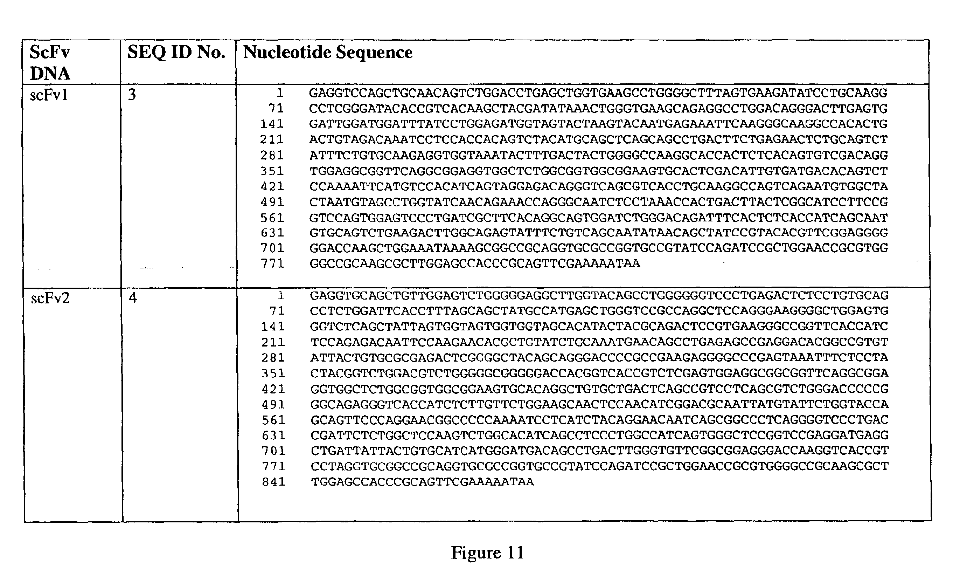

- Figure **11**: shows the nucleotide sequences coding for the polypeptides scFv1 and scFv2.

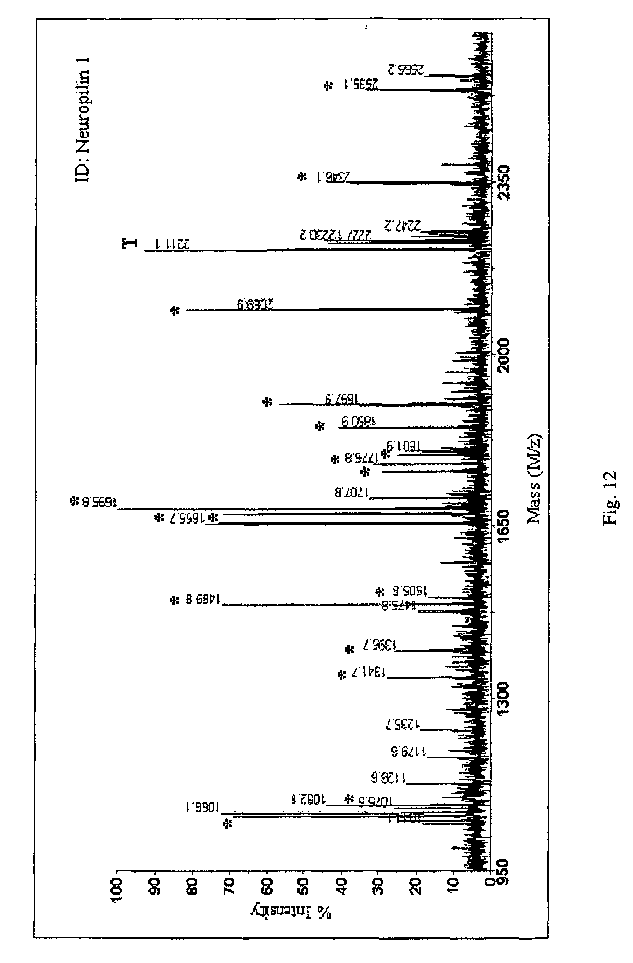

- Figure **12**: shows a MALDI-MS spectrum of the peptide mixture obtained from the band with an approximate size of 130 kDa immunoprecipitated with scFv 1. Two trypsin auto digestion peaks, indicated as T, were used for internal calibration. A total of 17 peaks, marked with asterisks, matched neuropilin-1 (SwissProt, 014786), with a mass deviation of less than 10 ppm. The matched peptides cover 22 % (206/923 residues) of the protein.
- Figure **13**: shows the principle of Chromophore-Assisted Laser Inactivation (CALI).

### EXAMPLES

### Example 1: Construction of an immune library

Two BALB/c mice were each immunized intradermally with 2 x 10⁷ paraformaldehyde fixed HT1080 cells (human fibrosarcoma cell line; ATCC, CCL-121). Following the first immunization, the injections were repeated thwice in a period of 39 days, the mice sacrificed and the spleens isolated and frozen in liquid nitrogen. The immunizations were performed by Charles River, Germany GmbH, Kißlegg.

Total RNA was isolated using the RNeasy Midi Kit (QIAGEN #75142) as described by the manufacturer using half of each spleen preparation. The RNA concentration and purity was determined by a denaturing formaldehyde gel and photometric measurement.

cDNA was synthesized using 8.9µg of freshly prepared RNA and 10pmol of a primer mix (IgG1-c, IgG2a-c, IgG2b-c, IgG3-c, VLL-c, VLK-c) using the Superscript™ II Kit (GibcoBRL Life Technologies #18064-014) These primers anneal to the RNA encoding the IgG heavy-chain genes and the light chain genes of the kappa and lambda family. VH genes were PCR amplified from 1µl of cDNA using 36 individual combinations of 9 forward primers (MVH1, M+VH2, MVH3, MVH4, MVH5, MVH6, MVH7, MVH8, MVH9) and 4 backward primers (M-JH1, M-JH2, M-JH3, M-JH4) without restriction sites. VL genes were PCR amplified with one primer mix (M-VK1, M-VK2, M-VK3 M-VK4, M-VL1, M-JK1, M-JK2, M-JK3, M-JL1) without restriction sites. PCR products were gel-purified (QIAquick Gel Extraction Kit, #28706) and re-amplified using individual combinations of 9 forward primers (MVH1 SfiI, MVH2 SfiI, MVH3 SfiI, MVH4 SfiI, MVHS SfiI, MVH6 SfiI, MVH7 SfiI, MVH8 SfiI, MVH9 SfiI) and 4 backward primers (M-JH1 SalI, M-JH2 SalI, M-JH3 SalI, M-JH4 SalI) with restriction sites for VH and one primer mix (M-VK1 ApaLI, M-VK2 ApaLI, M-VK3 ApaLI, M-VK4 ApaLI M-VL1 ApaLI, M-JK1 NotI, M-JK2 NotI, M-JK3 NotI, M-JL1 NotI) with restriction sites for VL. PCR products were gel-purified (QIAquick Gel Extraction Kit, #28706) and cloned into the phage display vector pXP10 using the restriction sites S*fi*I*ISalI* for VH and *ApaLI*/*NotI* for VL. The ligation mix was transfected into *E.coli* TG-1 by electroporation resulting in a library size of 10⁷ independent clones.

### Example 2: Selection and Screening of scFv (Selection on fixed cells)

Single chain Fv were selected from a phage display library generated from mice immunized with fixed HT1080 cells. The library was generated using the phage display vector pXP10.

HT1080 cells were harvested with 0.05% EDTA, fixed with paraformaldehyde, diluted to 1×10⁷cells/ml in PBS and immobilized onto wells of a 96 well UV cross-link plate (Corning Costar). The wells of the UV cross-link plate were blocked with 5% Skim Milk Powder (#70166, Fluka) in PBS (MPBS). 10¹² cfu (colony forming units) of phage library/10⁶ cells were pre-blocked for 1 hour at 25°C with MPBS and subsequently incubated for 1.5 hour at room temperature (RT) with the cells. The wells of the UV cross-link plate were washed six times with PBS + 0.05% Tween-20 followed by six washes with PBS. Bound phage were eluted by the addition of 10 mM Glycine pH 2.2, and neutralized with 1M Tris/HCl pH 7.4. Typically, between 10³ and 10⁶ cfu were eluted in the 1^{st} round of selection, thus the diversity of the enriched repertoire is decreased compared to the original repertoire. The eluate containing the enriched repertoire was amplified by infecting exponentially growing *E. coli* TG1. Phagemid containing *E. coli* were selected and propagated by overnight growth at 30°C on LB agar plates supplemented with 100µg/ml ampicillin and 1% glucose. Following this step, the enriched repertoire can either be amplified as a polyclonal pool and used for further rounds of selection in an iterative manner until convergence to desired properties is achieved or be spatially separated and screened on a single clone level. Phage particles for the next round of selection were produced by super-infecting exponentially growing cultures of the previous round of selection with helper phage VCS-M13 (Stratagene, La Jolla, CA) and growing the cultures overnight at 20°C in 2xTY supplemented with 100 µg/ml ampicillin and 50 µg/ml kanamycin. Selection ready phage were precipitated with 0.5 M NaCl/4% PEG-6000 from the cleared bacterial supernatant and re-suspended in PBS. One round of selection was performed followed by screening on a single clone level.

### Example 2.1: Selection and screening of scFv (Selection on cells in suspension)

Single chain Fv were selected from a large non-immune phage displayed repertoire of human origin containing 10¹¹ independent clones, provided by Cambridge Antibody Technology Ltd., Cambridge, UK.

For selection, HT1080 cells (human fibrosarcoma cell line; ATCC, CCL-121) were harvested with 0.05% EDTA and diluted to 1x10⁷cells/ml in DMEM + 10% FCS. Two times 10¹² cfu of phage library/10⁷ cells were pre-blocked for 1 hour at 25°C with DMEM + 10% FCS and subsequently incubated with end-over-end rotation for 1.5 hour at 25°C with the cells in Eppendorf tubes pre-blocked with DMEM + 10% FCS. Three times 10⁷cells were used for the first round of selection and 1x10⁷ cells were used for the 2^{nd} round of selection, respectively. The cells were washed by centrifugation at 220xg for five minutes, followed by removal of the supernatant and re-suspension in wash buffer. Five washes with DMEM + 10% FCS + 0.05% Tween-20 as wash buffer and five washes with DMEM + 10% FCS as wash buffer were performed. Bound phages were eluted by the addition of 10 mM glycine pH 2.2, neutralized with 1M Tris/HCl pH 7.4. Typically, between 10³ and 10⁶ cfu were eluted in the 1^{st} round of selection, thus the diversity of the enriched repertoire is decreased compared to the original repertoire. The eluate containing the enriched repertoire was amplified by infecting exponentially growing *E. coli* TG1. Phagemid containing *E. coli* were selected and propagated by overnight growth at 30°C on LB agar plates supplemented with 100µg/ml ampicillin and 1% glucose. Following this step, the enriched repertoire can either be amplified as a polyclonal pool and used for further rounds of selection in an iterative manner even until convergence to desired properties is achieved or be spatially separated and screened for a desired function on a single clone level. Phage particles for the next round of selection were produced by super-infecting exponentially growing cultures of the previous round of selection with helper phage VCS-M13 (Stratagene, La Jolla, CA) and growing the cultures overnight at 20°C in 2xTY supplemented with 100 µg/ml ampicillin and 50 µg/ml kanamycin. Selection ready phage were precipitated with 0.5 M NaCl/4% PEG-6000 from the cleared bacterial supernatant and re-suspended in PBS. In this example two rounds of selection were performed followed by screening on a single clone level.

### Example 3: Selection and Screening of scFv (Screening on fixed cells)

For screening, the genes encoding the selected scFv, contained in the phage display vector, were re-cloned to the expression vector pXP14. This vector directs the expression of a scFv in fusion with a Streptag and E-tag and does not contain a filamentous phage gene-3. Expression vector containing *E. coli* TG1 from single colonies were grown in individual wells of a microtiter plate so that each well contains only one scFv clone. The bacteria were grown at 30°C in 2xTY supplemented with 100 µg/ml ampicillin and 0.1% glucose in 96-well microtiter plates (#9297, TPP) until an OD₆₀₀ of 0.7. Expression was induced with IPTG at a final concentration of 0.5 mM and continued at 25°C overnight. Single chain Fv containing cleared lysates were prepared by addition of hen-egg lysozyme (#L-6876, Sigma) to a final concentration of 50 µg/ml for 1 hour at 25°C and centrifugation for 15 minutes at 3000 x g. Prior to the screening ELISA, the cleared lysates were blocked by the addition of an equal volume of DMEM + 10% FCS for 1 hour. For the screening ELISA, HT1080 cells were harvested with 0.05% EDTA, fixed with paraformaldehyde, diluted to 1x10⁷cells/ml in PBS and immobilized onto wells of a 96 well UV cross-link plate (Coming Costar). The wells of the UV cross-link plate were blocked with MPBS and the scFv containing blocked cleared lysates added for 1.5 hours at 25°C. The plates were washed 2x with PBS + 0.1% Tween-20 and 1x with PBS, incubated with HRP conjugated α-E-tag (#27-9413-01, Pharmacia Biotech; diluted 1:5000 in MPBS with 0.1 % Tween-20) for 1 hour, washed 3x with PBS + 0.1% Tween-20 and 3x with PBS, developed with POD (#1 484 281, Roche) and signals read at 370 nm. Positive clones were retested against HT1080 cells and control human fibroblasts Hs-27 (ATCC CRL-1634) using the ELISA screening procedure described above.

In a typical screen, 2760 (30x92) clones were screened for binding to HT1080 cells with 5 % positives defined as clones giving a background subtracted signal > 0.1. 155 positive clones were retested for specific binding to HT1080 cells compared to the Hs-27 control cells with 28 % positives defined as clones giving a background subtracted signal on HT1080 of twice the value of the signal on Hs-27 control cells. scFv1 was identified by applying this selection and screening method.

### Example 3.1: Selection and screening of scFv (Screening on adherent cells)

For screening, the genes encoding the selected scFv, contained in the phage display vector, were re-cloned to the expression vector pXP14. This vector directs the expression of a scFv in fusion with a Streptag and E-tag and does not contain a filamentous phage gene-3. Expression vector containing *E. coli* TG1 from single colonies were grown in individual wells of a microtiter plate so that each well contains only one scFv clone. The bacteria were grown at 30°C in 2xTY supplemented with 100 µg/ml ampicillin and 0.1% glucose in 96-well microtiter plates (#9297, TPP) until an OD₆₀₀ of 0.7. Expression was induced with IPTG at a final concentration of 0.5 mM and continued at 25°C overnight. Single chain Fv containing cleared lysates were prepared by addition of hen-egg lysozyme (#L-6876, Sigma) to a final concentration of 50 µg/ml for 1 hour at 25°C and centrifugation for 15 minutes at 3000xg. Prior to the screening ELISA, the cleared lysates were blocked by the addition of an equal volume of DMEM + 10% FCS for 1 hour. For the screening ELISA, HT1080 cells were seeded in a 96-well microtiter plate (#9296, TTP) at a density of 3x10⁴ cells/well in DMEM + 10% FCS overnight at 37°C. The wells were blocked with DMEM + 10% FCS for 1 hour at 37°C and the scFv containing blocked cleared lysates added for 1.5 hours at 25°C. The plates were washed 2x with PBS + 0.1% Tween-20 and 1x with PBS, incubated with HRP conjugated α-E-tag (#27-9413-01, Pharmacia Biotech; diluted 1:5000 in 5% Skim Milk Powder (#70166, Fluka) in PBS with 0.1 % Tween-20) for 1 hour, washed 3x with PBS + 0.1% Tween-20 and 3x with PBS, developed with POD (#1 484 281, Roche) and signals read at 370 nm. Positive clones were retested against HT1080 cells and control human fibroblasts Hs-27 (ATCC CRL-1634) using the ELISA screening procedure described above.

In a typical screen, 1472 (16x92) clones were screened for binding to HT1080 cells with 16 % positives defined as clones giving a background subtracted signal > 0.1. 238 positive clones were retested for specific binding to HT1080 cells compared to the Hs-27 control cells with 28 % positives defined as clones giving a background subtracted signal on HT1080 of twice the value of the signal on Hs-27 control cells. scFv2 was identified by applying this selection and screening method.

### Example 4: Sequencing and large scale expression

Sequencing of scFv1 and scFv2 genes was performed by Sequiserve GmbH, Vaterstetten, Germany using the primer pXP2 Seq2 (5'-CCCCACGCGGTTCCAGC-3') and pXP2 Seq1 (5TACCTATTGCCTACGGC-3'). The amino acid sequences are shown in Figure 10 and nucleotide sequences are shown in Figure 11.

Unique clones identified by sequencing were streaked out from glycerol stocks onto LB/Amp(100µg/ml)/1% Glucose Agar plates and incubated o/n at 30°C. 10 ml LB/Amp/Glu (1%) media were inoculated with a single colony and grown o/n at 30°C and 200 rpm shaking. The next morning the overnight cultures were placed on ice until inoculation of 1L 2xTY media supplemented with 100µg/ml Ampicillin and 0.1% Glucose in 2L Erlenmeyer-flasks. The cultures were grown at 25°C shaking until an OD₆₀₀0.5 - 0.6 was reached and then induced with IPTG 0.1 mM final concentration. Fresh Ampicillin was added to 50 µg/ml and incubation was proceeded at 22°C o/n shaking. In the morning the cultures were centrifuged at 5000 x g for 15 minutes at 4°C, supernatants discarded and the pellets resuspended carefully on ice with a pipette in 10 ml pre-cooled PBS-0.5 M Na buffer containing protease inhibitors complete (#1697498, Roche). After re-suspension was completed, bacterial suspensions were transferred to 20 ml oakridge centrifuge tubes and hen-egg lysozyme (#L-6876, Sigma) added to a final concentration of 50 µg/ml for 1 hour on ice. The lysed bacteria were centrifuged at 20000 x g for 15 minutes at 4°C and the supernatants (lysate) transferred to a 15 ml plastic tube. For affinity purification the lysates were loaded with 1 ml/min onto 1ml StrepTactin (# 2-1505-010, IBA) columns equilibrated with 10 column volumes (CV) PBS-0.5 M Na buffer via a parallel protein purification system (self-made). After a 10 CV wash with PBS the elution was done with 5 CV PBS/5mM Desthiobiotin (#D-1411, Sigma) and 1 ml fractions collected. The fractions were measured at UV_{280,} protein containing fractions were pooled and concentrated with Amicon Ultra Centrifugal Filter Devices 10.000 MWCO (#UFC801024, Millipore) at 4700 x g. The concentrated scFv were checked on 12% Bis-Tris SDS-PAGE gels stained with Coomassie Blue for purity and frozen in aliquots with 20 % glycerol at -80°C.

### Example 5: FACS analysis for tumor cell specific binding

To test the ability of purified anti HT1080 single chain Fv to bind specifically to target cells, we performed a fluorescence-activated cell sorter (FACS) analysis using HT1080 cells (ATCC CCL-121), KHOS cells (ATCC CRL-1544), PC-3 cells (ATCC CRL-1435), BT-474 cells (ATCC HTB-20), Hela cells (DSMZ ACC-57), HL60 cells (DSMZ ACC-3), Jurkat cells (DSMZ ACC-282), MCF7 cells (DSMZ ACC-115) and chang liver cells (DSMZ ACC-57 contain impurities of Hela cells) (for all 10⁶ cells/ml) and Hs-27 cells (10⁶ cells/ml) as control cell line (see Figure 5). Cells were incubated with 10µg/ml of pure scFv in Cell Wash (BD (Becton, Dickinson and Company) #349524) for 20 min at 4°C, washed, and bound scFvs were detected with a secondary FITC labeled anti E-tag mab (Amersham #27-9412-01). Samples were washed and analyzed on a Becton Dickinson FACSscan. Figure 5 shows the log fluorescence intensity (FL1-H; x-axis) versus the relative cell numbers (counts; y-axis) for cells reacting with scFv1. The thin line represents the control cell line (HS-27) and the bold line the tumor cell lines or chang liver cells. scFv1 and scFv2 specifically stain the tumor cell lines with up to 10 fold higher signals compared to the control cell line.

### Example 6: Competition analysis by FACS

To test the ability of the purified anti neuropilin-1 scFv to block common neuropilin-1 epitopes on the target cells, single cell suspensions of HT1080 are harvested with 0,5mM EDTA/PBS. Approximately 1 x 10⁶ cells are incubated in CellWash "(BD, #349524) with 10µg/ml scFv for one hour at 4°C. After washing with Cell Wash 10µg/ml FITC labeled scFv is added and incubated for 20 min at 4°C. Signals of bound FITC labeled scFvs with and without pre incubation of other neuropilin-1 binders are analyzed on a Becton Dickinson FACSscan. "

### Example 7: Labeling of antibody fragments with FITC

scFv were labeled with fluorescein isothiocyante (FITC) (Molecular Probes, Eugene, USA #F1906) by the following method: Aliquots of a 10mg/ml solution of FITC in dimethyl sulfoxide were added to 100µg of scFv1 dissolved in PBS/0.5M NaHCO₃, pH 9.5 in a ratio of 30:1 (FITC:scFv1). The sample was incubated for two hours at room temperature with agitation, free FITC was separated using desalting columns (2 Micro Spin G-25, Pharmacia 27-5325-01). The ratio of labeling was determined via mass spectrometry and via UV/VIS spectroscopy, whereby the protein concentration was calculated at 280nm and the FITC concentration at 494nm.

### Example 8: Invasion assay for identification of inhibitory antibody fragments

The ChemoTx® system (Neuro Probe Inc.#106-8, Gaithersburg) is used as a disposable chemotaxis/cell migration chamber in a 96 well format with an 8µm filter Track etched Polycarbonate pore size, 5.7 mm diameter/site.

13,3µl of 0.3mg/ml Matrigel (Matrigel is a solubilized basement membrane preparation extracted from the Engelbreth-Holm-Swarm (EHS) mouse sarcoma, a tumor rich in extracellular matrix proteins. Its major component is laminin, followed by collagen IV, heparan sulfate proteoglycan, entactin and nidogen. It also contains TGF-β fibroblast growth factor, tissue plasminogen activator, and other growth factors which occur naturally in the EHS tumor) (Becton Dickenson, BD #356234) diluted in Dulbeccos PBS (Gibco #14040-091) is applied on the membrane filter of the 96-well plate on row B-H and on row A 1,2 µg/site of collagen S type I (Roche #10982929) is diluted in 0,05 M HCl (Sigma #945-50) and is incubated over night at 20°C in a desiccator for gelation. HT1080 cells are grown to 70-80% confluence in DMEM supplemented with GlutamaxI (862mg/l (Gibco #31966-021) with 10% FCS (Gibco #10270106). The cells are washed 2x with DMEM/GlutamaxI/0.1 % BSA (Sigma #A-7030) then labeled *in situ* with Bis-benzimide H 33342 (Sigma #B-2261) are diluted 1:100 in DMEM/GlutamaxI/0.1 % BSA for 15 min at 37°C, 7,5% CO₂. Cells are washed 2x with DMEM/GlutamaxI/0.1 % BSA and are loaded with DMEM/GlutamaxI/0.1 % BSA for 15 min at 37°C, 7,5% CO₂ for recovering. After washing 2x with PBS w/o Ca²⁺, Mg²⁺ (Gibco, 10010-015), the cells are detached with 0.5mM EDTA (Sigma #E8008), are collected with Dulbeccos PBS/0.1% BSA/10mM Hepes (Gibco #15630-056), are washed 2x with Dulbeccos PBS/0.1% BSA/10mM Hepes, are suspended in Dulbeccos PBS/0.1% BSA/10mM Hepes and are diluted to 6,7 x 10⁶ cells/ml with Dulbeccos PBS/0.1% BSA/10mM Hepes. 6,7 x 10⁶ cells/ml are incubated 1:1 with 40µg/ml of a control scFv as a negative control for inhibition of invasion and with HT1080 specific scFv for 1h on ice. After dilution to 6,7 x 10⁵ cells/ml with DMEM/GlutamaxI/0.1 % BSA, HT1080 cells and HT1080 cell/scFv dilutions are pipetted in triplicate onto the chemotaxis chamber (row B-H) at a density of 3,4 x 10⁴ cells/well and are incubated for 6 h at 37°C, 7,5% CO₂. DMEM/GlutamaxI with 5% FCS is used as a chemo attractant in the lower chamber. A standard curve from 1x10⁴ to 4x10⁴ cells/site is performed on collagen S type I coated row A of the chemotaxis chamber. DMEM/GlutamaxI/0.1% BSA is used in the lower chamber (cells are not migrating). After scraping the non-migrating cells from the top of the membrane (except the Standard curve on row A) fluorescence of cells, which had migrated through the membrane (not migrated in case of the Standard curve), is measured on the Fluostar Galaxy (bMG) microplate reader using excitation/emission wavelengths of 370/460nm.

### Example 8.1: Influence of VEGF on the invasion of HT1080 cells

The invasion assay set-up was identical to the set-up described in Example 8. The invasion membrane (Neuroprobe #106-8, Gaithersburg, MD) was coated overnight with Matrigel (0,3mg/ml; BD #356234, Bedford, MA). 80 % confluent HT1080 cells were stained with Bis-benzimide H33342 and then detached with 0,05% EDTA. Cells (7x10⁶/ml, final 35.000/well) were pre-incubated for 1h at 4°C with:
a. 0, 25, 50 or 100 ng/ml recombinant human VEGF (R&D Systems #293-VE, Minneapolis) in the presence or absence of 20 µg/ml anti-human VEGF antibody (R&D Systems #AF-293-NA). 5% FCS (not heat-inactivated) in DMEM was used as chemo-attractant in the well below the membrane;
b. in the presence or absence of 20 µg/ml anti-human VEGF antibody, with 5% FCS and 25, 50 or 100 ng/ml VEGF as chemo-attractant;
c. 0, 25, 50 or 100 ng/ml VEGF in the presence or absence of 20µg/ml anti-human VEGF antibody, with 1% BSA (Sigma #A7030, ST. Louis, MO) in DMEM in the well below the membrane;
d. in the presence or absence of 20 µg/ml anti-human VEGF antibody, using 25, 50 or 100 ng/ml VEGF in DMEM containing 1% BSA as chemo-attractant.

50µl of the preincubated cell suspensions (a-d) were pipetted on top of the membrane and the invasion plate was incubated for 6h at 37°C, 7,5% CO₂. The readout of the invasion assay was identical to the readout described in Example 8. No significant increase of invasion was seen when the cells were pre-incubated with different concentrations of recombinant VEGF (a). Also, the addition of anti-human VEGF antibody had no influence on the invasion of HT1080 cells (a), compared to the result of Experiment 8. The addition of recombinant VEGF to the chemo-attractant FCS did not result in an increase of invasion of HT1080 cells (b). A variation of the VEGF concentration did not show an effect either. The addition of anti-human VEGF antibody had also no effect on this result (b). The preincubation with VEGF did not result in an invasion of HT1080 cells when BSA was used as a chemo-attractant (c). The use of VEGF as a chemo-attractant in DMEM and 1% BSA did also not stimulate the invasion of HT1080 cells (d), whereas 5% FCS as a chemo-attractant induced invasion (fourth bar from the left in Figure 2a).

### Example 9: Invasion assay for target identification with CALI

This Example is in general identical to Example 8, except for the use of FITC-labeled scFv (see, Example 7 for labeling) and the integration of the CALI process within the invasion assay.

The ChemoTx® system (Neuro Probe Inc.#106-8, Gaithersburg) was used as a disposable chemotaxis/cell migration chamber in a 96 well format with an 8µm filter Track etched Polycarbonate pore size, 5.7 mm diameter/site.

13,3µl of 0.3mg/ml Matrigel (see Example 8) diluted in Dulbeccos PBS (Gibco #14040-091) was applied on the membrane filter of the 96-well plate on row B-H and on row A 1,2 µg/site of collagen S type I (Roche #10982929) was diluted in 0,05 M HCl (Sigma #945-50) and was incubated over night at 20°C in a desiccator for gelation. HT1080 cells were grown to 70-80% confluence in DMEM supplemented with GlutamaxI (862mg/I (Gibco #31966-021) with 10% FCS (Gibco #10270106). The cells were washed 2x with DMEM/GlutamaxI/0.1 % BSA (Sigma #A-7030) then labeled *in situ* with Bisbenzimide H 33342 (Sigma #B-2261) and were diluted 1:100 in DMEM/GlutamaxI/0.1 % BSA for 15 min at 37°C, 7,5% CO₂. Cells were washed 2x with DMEM/GlutamaxI/0.1 % BSA and loaded with DMEM/GlutamaxI/0.1 % BSA for 15 min at 37°C, 7,5% CO₂ for recovering. After washing 2x with PBS w/o Ca²⁺, Mg²⁺ (Gibco, 10010-015), the cells were detached with 0.5mM EDTA (Sigma #E8008), collected with Dulbeccos PBS/0.1% BSA/10mM Hepes (Gibco #15630-056), washed 2x with Dulbeccos PBS/0.1% BSA/10mM Hepes, suspended in Dulbeccos PBS/0.1% BSA/10mM Hepes and diluted to 6,7 x 10⁶ cells/ml with Dulbeccos PBS/0.1% BSA/10mM Hepes. 6,7 x 10⁶ cells/ml were incubated 1:1 with 40µg/ml of FITC-labelled anti-betal integrin monoclonal antibody (JB1, Chemicon #MAB1963) as a control for inhibition of invasion after CALI and with HT1080 specific FITC labelled scFv for 1h on ice. 1,3x10⁵ HT1080 cells/well or HT1080 cell/scFv or Ab dilution were pipetted in triplicate in two 96-well plate, black, ultra thin clear flat bottom special optics (Costar #3615). One plate was kept on ice in the dark while the other plate was irradiated on an ice block with continuous wave laser at 488nm (0.5W, 30 sec). After dilution to 6,7 x 10⁵ cells/ml with DMEM/GlutamaxI/0.1 % BSA, HT1080 cells and HT1080 cell/scFv dilutions were pipetted in triplicate (non irradiated triplicate beside irradiated triplicate) onto the chemotaxis chamber (row B-H) at a density of 3,4 x 10⁴ cells/well and incubated for 6 h at 37°C, 7,5% CO₂. DMEM/GlutamaxI with 5% FCS was used as a chemo attractant in the lower chamber. A standard curve from 1x10⁴ to 4x10⁴ cells/site was performed on collagen S type I coated row A of the chemotaxis chamber. DMEM/GlutamaxI/0.1% BSA was used in the lower chamber (cells were not migrating). After scraping the non-migrating cells from the top of the membrane (except the Standard curve on row A) fluorescence of cells, which had migrated through the membrane (not migrated in case of the Standard curve), was measured on the Fluostar Galaxy (bMG) microplate reader using excitation/emission wavelengths of 370/460nm. In a general experiment, a value of 45000 corresponded to 100% migrated cells.

The invasion phenotype of HT1080 cells was assessed by comparing their relative ability to invade tumor extracellular matrix (Matrigel) using the Transwell culture system described above. scFv1 showed after CALI an inhibitory effect of 25% on the invasion of the HT1080 cells. The result of the invasion assay with and without CALI (see Example 8) is shown in Figure 2. Figure 2 shows that CALI converts scFv1 in an inhibitory antibody fragment.

### Example 10: MTS viability assay

Viable cells were detected by measuring the conversion of the tetrazolium dye MTS (MTS, Celltiter A_{queous} one, Promega #G4000) to formazan. HT1080 cells and HT1080 cell/scFv dilutions (obtained from the dilutions prepared in the Invasion assay) were pipetted in triplicate at a density of 3,4 x 10⁴ cells/well and were plated in a 96-well plate (black, ultra thin clear flat bottom, special optics, Costar #3615) 10 µl MTS was added to each well and incubated for 1 hour at 37°C, 7,5% CO₂. Absorbance was measured at 492 nm with the Fluostar Galaxy (bMG) microplate reader. For all tested scFvs, no effect on viability of cells was seen (data not shown).

### Example 11: Cell-matrix adhesion assay for identification of inhibitory antibody fragments

21 wells of a 96-well flat bottom plate (Costar #3614) were coated with one matrix protein selected from collagen S type I 1µg/well (Roche #10982929), collagen type IV 1µg/well (Rockland 009-001-106), fibronectin 1µg/well (Sigma F2518) and laminin 1µg/well (Roche 1243217) in Dulbeccos PBS (Gibco #14040-091), respectively, at 4° C over night. At the same time 3 wells in row A were coated with 2% BSA (Sigma #A-7030)/Dulbeccos PBS for a blank value. Wells were washed twice with Dulbeccos PBS, and blocked with 2% BSA (Sigma #A-7030)/Dulbeccos PBS for 1h at 37°C and washed with Dulbeccos PBS. HT1080 were harvested, stained with 2,5mM (final concentration) Calcein AM (Molecular Probes C-3099), washed twice with PBS w/o CaCl₂ w/o MgCl₂ (Gibco 10010-015) and diluted to 1,5 x10⁵/ml in buffer (0,5% BSA (Sigma #A-7030) + 10mM Hepes + DMEM (Gibco 31966-02)). HT1080 cells were mixed with 10µg/ml scFv and incubated for 30 min on ice. The HT1080 cells alone and HT1080/scFv dilutions were pipetted in triplicate at a density of 1,5x10⁴ cells/well and incubated for one hour at 37°C, 7,5% CO₂. After two additional washing steps with Dulbeccos PBS, where non-adherent cells were washed away, a Standard curve from 3,7 x10³ to 1,5 x10⁴ stained cells/well diluted in Dulbeccos PBS was performed in triplicate in row A. Washed wells were filled with 100µl Dulbeccos PBS and the absorbance of attached cells and of the Standard curve was measured on the Fluostar Galaxy (bMG) microplate reader using excitation/emission wavelengths of 485/520nm. scFv1 showed an inhibitory effect of approximately 20% on the adhesion of HT1080 cells to collagen S type I, collagen type IV, fibronectin and laminin. scFv2 showed an inhibitory effect of 27 % on the adhesion of HT1080 cells to laminin and a relatively low inhibitory effect on the adhesion of HT1080 cells to collagen type IV and fibronectin. No effect was seen on the adhesion of HT1080 cells to collagen S type I. Results for scFv1 and scFv2 are shown in Figure 4.

### Example 12: Cell-matrix adhesion assay for target identification with CALI

96-well plates (TPP #9296) (cell culture treated) were coated in Row B-H with collagen S type I 1µg/well (Roche #10982929) in Dulbeccos PBS (Gibco #14040-091) and in Row A well 10-12 were coated with 2% BSA (Sigma #A-7030)/Dulbeccos PBS at 4°C over night. The plate was washed with Dulbeccos PBS, blocked Row B-H and Row A well 10-12 with 2% BSA/Dulbeccos PBS for 1h at 37°C and washed again with Dulbeccos PBS. HT1080 cells were grown to 70-80% confluence in DMEM supplemented with GlutamaxI (862mg/l (Gibco #31966-021) with 10% FCS (Gibco #10270106). The cells were washed 2x with DMEM/GlutamaxI/0.1 % BSA (Sigma #A-7030) then labeled *in situ* with Bis-benzimide H 33342 (Sigma #B-2261) were diluted 1:100 in DMEM/GlutamaxI/0.1 % BSA for 15 min at 37°C, 7,5% CO₂. Cells were washed 2x with DMEM/GtutamaxI/0.1 % BSA and loaded with DMEM/GlutamaxI/0.1 % BSA for 15 min at 37°C, 7,5% CO₂ for recovering. After washing 2x with PBS w/o Ca²⁺, Mg²⁺ (Gibco, 10010-015), the cells were detached with 0.5mM EDTA (Sigma #E8008), collected with Dulbeccos PBS/0.1% BSA/10mM Hepes (Gibco #15630-056), washed 2x with Dulbeccos PBS/0.1% BSA/10mM Hepes, suspended in Dulbeccos PBS/0.1% BSA/10mM Hepes and diluted to 6,7 x 10⁶ cells/ml with Dulbeccos PBS/0.1% BSA/10mM Hepes. 6,7 x 10⁶ cells/ml were incubated 1:1 with 40µg/ml of FITC-labeled anti-betal integrin monoclonal antibody (JB1, Chemicon #MAB1963) as a control for inhibition of adhesion after CALI and with HT1080 specific FITC labeled scFv for 1h on ice. 1,3x10⁵ HT1080 cells/well or HT1080 cell/scfv or Ab dilution were pipetted in triplicate in two 96-well plate, black, ultra thin clear flat bottom special optics (Costar #3615). One plate was kept on ice in the dark while the other plate was irradiated on an ice block with continuous wave laser at 488nm (0.5W, 30 sec). After dilution to 6,7 x 10⁵ cells/ml with DMEM/GlutamaxI/0.1 % BSA, HT1080 cells and HT1080 cell/scFv dilutions were pipetted in triplicate (non irraditated triplicate beside irradiated triplicate) onto the coated and blocked plate. In Row A well 10-12 6,7 x 10⁵ cells/ml with DMEM/GlutamaxI/0.1 % BSA were pipetted as a background control. Plate was incubated for 1h at 37°C, 7,5% CO₂ and washed 2x with Dulbeccos PBS, where non-adherent cells were washed away. In Row A well 1-9 a standard curve from 1x10⁴ to 4x10⁴ cells/well is performed, in all other wells 50 µl Dulbeccos PBS is pipetted. Fluorescence of cells, which had adhered to the Collagen S type I (not adhered in case of the Standard curve), was measured on the Fluostar Galaxy (bMG) microplate reader using excitation/emission wavelengths of 370/460nm. scFv1 showed after CALI an inhibitory effect of 30% on the adhesion of the HT1080 cells to collagen S type I. scFv2 showed after CALI an inhibitory effect of approximately 10% on the adhesion of HT1080 cells to collagen S type I. In one set of experiments scFv2 showed already an inhibitory effect on the adhesion of HT1080 cells to collagen S type I of 5% without CALI. The results of the adhesion assay with and without CALI are shown in Figure 3.

### Example 12: Immunoprecipitation

HT1080 and Hs-27 cells (10⁸) were lysed in 3ml 50 mM Tris-HCl, pH 8.0, 150 mM NaCl, 1% Triton X-100 (v/v) containing protease inhibitor cocktail (1 pill in 50ml buffer) (Boehringer Mannheim, Cat.-No. 1697498) and 100 µM Pefablock (Roth, Cat.-No. A154.1). Lysates were pre-incubated for 2h at 4°C with Streptactin Sepharose (IBA, # 2-1201-010) and the supernatants used for the immunoprecipitation reactions. HT1080 specific scFv (50 µg/1 mg cell extract) were added to the cleared lysates, samples rotated for 2h at 4°C, gently centrifuged at 700g to pellet the Streptactin Sepharose, the pellet was washed 4 times with 1ml volume of PBS + 0.1% Tween buffer per wash, before the complexes were isolated by elution from the Streptactin Sepharose pellet with 50 µl 10 mM D-desthiobiotin in PBS 0.1% Tween 20. The immuno-complexes were separated by SDS-PAGE and silver stained for MS analysis.

scFv 1 and scFv 2 pulled down a protein, detected as a band on SDS-PAGE by silver staining at a molecular weight of ~ 130 kDa. This band was only detected in the HT1080 cell extract and not in the Hs-27 cells (control cells), see Figure 6.

### Example 13: Protein identification via mass spectroscopy

The gel bands obtained from immunoprecipitations followed by SDS PAGE were subjected to a tryptic in-gel digest over night at 37 °C. Peptides were extracted using 5% formic acid and the resulting peptide mixture was desalted using ZipTip µC18 (Millipore) and eluted first with 2 µl of 30% ACN/0.1% TFA, then with 2 µl of 70% ACN/0.1% TFA. The two fractions were pooled and one microliter of the obtained peptide mixtures was mixed in a 1:1 ratio with a solution of α-cyano-4-hydroxycinnamic acid (3 mg/ml), co-crystallized on a Teflon-coated stainless steel target and analyzed on a MALDI-TOF instrument yielding peptide mass fingerprints (PMF) in a mass range of m/z 800-3000. The obtained PMF were used to search all entries for the species *Homo sapiens* in the NCBI and SwissProt databases. In all cases, only peptides matching a given protein with a mass deviation of less than 10 ppm were considered for identification.

The band with an approximate size of 130 kDa, obtained by using scFv1 or scFv2, yielded up to 17 peptide peaks (in different experiments with the same scFv), which matched neuropilin-1, with a maximum protein coverage of 22% (206/923 residues). 3 peptides, namely peptide fragments 659-672, 680-702 and 776-787 clearly support the identity of neuropilin-1. Neuropilin-1's splice variant, called soluble neuropilin-1, misses amino acids 645-923 when compared to neuropilin-1 with its 923 amino acids. Therefore, the obtained mass spectrum clearly proves that the full length version of NP-1 was immunoprecipitated by scFv1 and scFv2. A spectrum is shown in Figure 12.

### Example 15: Methods for epitope mapping

An epitope mapping may be carried out according to one of the following methods:

### Example 15.1: "Classical" epitope mapping

Defined fragments of the cDNA for the antigen of interest are expressed as recombinant (fusion)proteins and probed in various assays such as Westernblot or ELISA.

### Example 15.2: Phage display technology

The technique of epitope mapping using random peptide phage display libraries was developed to clone small random fragments of the cDNA for the antigen of interest into the phage protein pIII of filamentous phages and display them on the surface of the phage (Fack et al., (1997) J. Immunol. Methods 7, 43-52). Epitope-displaying phages can be captured with antibodies in a procedure called "biopanning". Sequencing of the inserts of the corresponding phages gives some information on the epitopes. This procedure is in principle capable to identify conformational epitopes.

### Example 15.3: Peptide scan technology

It is based on the synthesis of immobilized peptides on activated membranes using the Fmoc chemistry. Amino acid solutions are applied to the activated membrane leading to a peptide bond between the amino-group on the membrane (the membrane is activated with PEG) and the activated carboxy-group of the applied amino acid. After each cycle a specific washing procedure, acetylation, deprotection and monotoring of free amino-groups is performed. In contrast to the in vivo protein-synthesis membrane bound oligo-peptide chains are stepwise synthesized from C- to the N-terminus. Oligo-peptides containing natural as well as modified amino acids can be synthesized up to a length of 20 amino acids. Following synthesis the membranes are equilibrated and unspecific binding sites are blocked. After incubation with the antibody of interest and several washing steps the detection is performed using an HRP-conjugated secondary antibody in combination with the ECL-System. Membranes can be stripped, regenerated, and re-used up to 10 times depending on the antibody. Small overlapping oligo-peptides that ideally cover the complete amino acid sequence of the antigen of interest are synthesized on a solid support. This method allows the identification of linear epitopes on the amino acid level. It also allows rapid mutational studies.

### Example 16: Depletion of cellular neuropilin-1 protein by RNAi against neuropilin-1 mRNA

The following ribo-oligonucleotides can be purchased from, e.g. Proligo (Hamburg, Germany, www.proligo.com), Pierce Chemical (part of Perbio Science, Rockford, Illinois, www.perbio.com), or another supplier of RNA oligonucleotides:

Oligo 1A (5'-CAGCACCATACAATCAGAGTT-3'),

Oligo 2A (5'-CTCTGATTGTATGGTGCTGTT-3'),

Oligo 1B (5'- CAGCACCATTGAATCAGAGTT -3'),

Oligo 2B (5'- CTCTGATTCAATGGTGCTGTT -3'),

Oligo 3A (5'- TGGGATGTCAGAAAGAAACTT-3'),

Oligo 4A (5'- GTTTCTTTCTGACATCCCATT-3')

Oligo 3B (5'- TGGGATGTCTCAAAGAAACTT -3'),

Oligo 4B (5'- GTTTCTTTGAGACATCCCATT -3').

The oligonucleotides 1A and 2A anneal to form siRNA directed against the neuropilin-1 mRNA, the oligonucleotides 1B and 2B anneal to form a negative control for the pair above, as the underlined nucleotides are mismatches to the neuropilin-1 mRNA. The oligonucleotides 3A and 4A anneal to form siRNA directed against the neuropilin-1 mRNA, the oligonucleotides 3B and 4B anneal to form a negative control for the 3A/4A-pair, as the underlined nucleotides are mismatches to the neuropilin-1 mRNA. Both; the 1A/2A- and the 3A/4A-pair are directed against a C-terminal region only present in the membrane-bound full-length form of neuropilin-1, but not in the soluble form of neuropilin-1.

The oligonucleotide pairs 1A/2A, 1B/2B, 3A/4A, 3B /4A are annealed using Protocol 2 on page 203 of Elbashir et al., Methods (2002) 26: 199-213. When working with RNA special care is used to avoid RNAse contamination of the oligonucleotides. DEPC-treated water is used for preparing buffers. Gloves are always worn to avoid RNAse-contamination by contact with the skin. For detailed instruction on RNA-related work consult, e.g. Chapter 7.3 and 7.4 of the 2^{nd} edition of Sambrook et al. "Molecular Cloning: A Laboratory Manual" (1989), Cold Spring Harbor Laboratory Press.

HT1080 cells are grown to about 90% confluence in DMEM supplemented with Glutamax (Gibco #31966-021) with 10% FCS (Gibco #10270106). 24h before transfection with the annealed oligonuceotide pairs described above, the 90% confluent cells out of a 175-ml cell culture flask are harvested, e.g. trypsinized with 10ml trypsin-EDTA solution (Life Technologies), gently centrifuged and resuspended in 10ml DMEM. The cell suspension is diluted 1:10 with fresh DMEM medium with 10% FCS without antibiotics and transferred as 500µl aliquots into each well of a 24-well plate. 24h after seeding the cells a confluency of about 50% is reached.

Separate wells of these cells are then transfected with the annealed oligonucleotide pairs described above according to Protocol 5 on page 207 of Elbashir et al., Methods (2002) 26, 199-213.

After 2 days of incubation according to step 5 of protocol 5 mentioned above, the cells are harvested from the wells and the cells of each well are processed for Western blot, e.g. samples (corresponding to the same number of cells) from a well tranfected without oligonucleotides (untreated cells), a well transfected with the oligonucleotide pair 1A/2A, the pair 1B/2B (negative control 1/2), the pair 3A/4A and the pair 3B/4B (negative control 3/4) are loaded on an acrylamide gel, after the run the proteins are blotted onto, e.g. nitrocellulose, e.g. using a semi-dry blotting apparatus, and the nitrocellulose membranes are probed with anti-neuropilin-1 antibodies.

The amount of neuropilin-1 protein detected in at least one of the samples 1A/2A or 3A/4A is reduced by more than 75% in comparison to the untreated cells. A significant reduction of the level of neuropilin-1 protein is not detected in the negative controls 1B/2B and 3B/4B in comparison to the untreated cells.

### Example 17: Inhibition of adhesion or invasion by RNAi against neuropilin-1

HT1080 cells are grown to about 90% confluence in DMEM supplement with Glutamax (Gibco #31966-021) with 10% FCS (Gibco #10270106). 24h before transfection with the annealed oligonuceotide pairs described above, the 90% confluent cells out of a 175-ml cell culture flask are trypsinized with 10ml trypsin-EDTA solution (Life Technologies). The cell suspension is diluted 1:10 with fresh DMEM medium with 10% FCS without antibiotics and transferred as 500µl aliquots into each well of a 24-well plate. 24h after seeding the cells a confluency of about 50% is reached.

Separate wells of these cells are then transfected with the annealed oligonucleotide pairs described above according to Protocol 5 on page 207 of Elbashir et al., Methods (2002) 26, 199-213.

After 2 days of incubation according to step 5 of protocol 5 mentioned above, the cells are labeled *in situ* with Bisbenzimide H 33342 (Sigma #B-2261) for 15 min at 37°C, 7,5% CO₂. After washing, the cells are dissociated with 0.5mM EDTA/PBS (Sigma #E8008), washed and suspended in 0.1% BSA (Sigma #A7030)/DMEM. The cells are then diluted to 6,7 x 10⁵ cells/ml with 0.1% BSA/DMEM, HT1080 cells and added onto the chemotaxis chamber as described in Example 8 or on a 96 well plate as described in Example 11. Invasiveness is then determined as described in Example 8 and adhesiveness is determined as described in Example 11.

The adhesiveness or invasiveness measured in at least one of the samples 1A/2A or 3A/4A is reduced by more than 40% in comparison to the untreated cells. A significant reduction of invasiveness is not detected for the negative controls 1B/2B and 3B/4B in comparison to the untreated cells.

## Claims

1. A polypeptide comprising a sequence selected from the group consisting of SEQ ID NO: 1 and SEQ ID: 2.

2. The polypeptide of claim 1, wherein the polypeptide is an antibody or an antibody fragment.

3. The polypeptide of claim 2, wherein the CDR3 region of said antibody or antibody fragment is identical to one of the CDR3 regions shown in Figure 10.

4. The polypeptide of any of claims 1 to 3, wherein said polypeptide is labeled with a detectable label; in particular wherein said detectable label is selected from the group consisting of a radioisotope, an enzyme and a chromophore.

5. A bioconjugate comprising a polypeptide according to any of claims 1 to 5.

6. Use of a polypeptide according to any of claims 1 to 4 and/or a bioconjugate of claim 5 for the detection of neuropilin-1.

7. A diagnostic kit comprising a polypeptide according to any of claims 1 to 4 and/or a bioconjugate according to claim 5, and a container.

8. A pharmaceutical composition comprising a bioconjugate according to claim 5, and a pharmaceutical acceptable carrier.

9. An isolated nucleic acid molecule encoding a polypeptide according to any of claims 1 to 4.

10. A vector comprising a nucleic acid according to claim 9.

11. Use of a molecule inhibiting neuropilin-1-function in the manufacture of a medicament for the treatment or prevention of metastasis of naturally occurring tumor cells, wherein the metastatic potential depends on neuropilin-1-related invasion and/or adhesion, preferably wherein the tumor cells are tumor cells derived from mesodermal cells.

12. Use according to claim 11, wherein the tumor cells are tumor cells derived from the group of neoplasms consisting of soft tissue tumors and sarcomas, fibromatous neoplasms, myxomatous neoplasms, lipomatous neoplasms, myomatous neoplasms, complex mixed and stromal neoplasms, synovial-like neoplasms, mesothelial neoplasms, lymphatic vessel tumors, osseous and chondromatous neoplasms, giant cell tumors, miscellaneous bone tumors, odontogenic tumors, hodgkin's and non-Hodgkins's lymphoma, plasma cell tumors, mast cell tumors, immunoproliferative diseases and leukemias, in particular tumor cells derived from the group of neoplasms consisting of soft tissue tumors and sarcomas, fibromatous neoplasms, myxomatous neoplasms, lipomatous neoplasms, myomatous neoplasms, complex mixed and stromal neoplasms, synovial-like neoplasms, mesothelial neoplasms, lymphatic vessel tumors, osseous and chondromatous neoplasms, giant cell tumors, miscellaneous bone tumors, odontogenic tumors, in particular tumor cells derived from sarcomas, in particular derived from sarcomas of the bone or sarcomas of soft tissue.

13. The use of claims 11 or 12, wherein the molecule inhibits gene expression of neuropilin-1, in particular wherein the molecule is an antisense oligonucleotide, a siRNA or siRNA-like hairpin RNA, or a vector leading to the cellular presence of a siRNA or siRNA-like hairpin RNA.

14. The use of claims 11 or 12 wherein the molecule inhibits the function of expressed neuropilin-1; in particular wherein the molecule binds to the extracellular region of neuropilin-1; more particularly wherein the molecule is selected from the group consisting of a small chemical compound, an antibody, an antibody fragment, an anti-idiotypic antibody, a polypeptide according to any of claims 1 to 4 and a bioconjugate according to claim 5; especially wherein the molecule is a bioconjugate according to claim 5.

15. The use of any of claims 11 to 14, wherein the tumor cells are cells derived from the group of neoplasms consisting of malignant fibrous histiocytoma, liposarcoma, fibrosarcoma, synovial sarcoma, osteosarcoma (parosteal osteosarcoma, periosteal osteosarcoma, small-cell osteosarcoma), chondrosarcoma, Ewing's sarcoma, giant-cell tumor of bone, osteogenic sarcoma, leiomyosarcoma, rhabdomyosarcoma, mesothelioma, hemangiosarcoma, lymphangiosarcoma, myxosarcoma, endotheliosarcoma, chordoma, Kaposi's sarcoma and lymphangioendotheliosarcoma.

16. An *ex vivo* method of determining the dependency of the invasiveness of a naturally occurring invasive cancer cell on the functionality of neuropilin-1, comprising the steps of:
b) contacting the cancer cell with a molecule inhibiting neuropilin-1 function;
c) contacting said cancer cell with a gel-like matrix under conditions suitable for the growth of said cancer cells; and
d) determining the migration of said cancer cells through the gel-like matrix.

17. An *ex vivo* method of determining the dependency of the adhesiveness of a naturally occurring invasive cancer cell on the functionality of neuropilin-1, comprising the steps of:
a) contacting the cancer cell with a molecule inhibiting neuropilin-1 function;
b) contacting said cancer cell with a layer of ECM proteins under conditions suitable for the growth of said cancer cells; and
c) determining the adhesion of said cancer cells to the layer of ECM proteins.

18. The method of claims 16 or 17, wherein step a) comprises contacting the cell with a polypeptide that specifically binds to an extracellular epitope of neuropilin-1, or wherein step a) comprises the use of an antisense oligonucleotide, an siRNA or siRNA-like hairpin RNA, or a vector leading to the cellular presence of an siRNA or siRNA-like hairpin RNA.

19. The method of claims 16 or 18, wherein the gel-like matrix comprises the proteins collagen type IV, fibronectin and laminin.

20. The method of claims 17 or 18, wherein the layer of ECM proteins comprises one protein selected from the group consisting of collagen S type I, collagen VI, fibronectin, laminin, nidogen, entactin, and vitronectin.

21. A method of identifying a ligand binding specifically to the extracellular region of neuropilin-1, wherein said ligand is capable of inhibiting invasiveness and/or adhesiveness of sarcoma cells, comprising the steps of:
a) contacting a library of amplifiable ligand-displaying units (ALDUs) with invasive sarcoma cells;
b) separating said sarcoma cells and the ALDUs bound thereto from ALDUs not bound to said cells;
c) amplifying the ALDUs bound to said cells;
d) identifying an ALDU or a ligand derived from an ALDU after step c) which affects invasiveness and/or adhesiveness of sarcoma cells;
e) identifying an ALDU or a ligand derived from an ALDU after step d) capable of binding to neuropilin-1;
f) and optionally determining the identity of the ligand represented by said ALDU.

22. The method of claim 21, instead of steps e) and f) further comprising the steps of:
e) contacting the ALDUs of step c) with immobilized neuropilin-1;
f) separating said immobilized neuropilin-1 and the ALDUs bound thereto from ALDUs not bound to said immobilized neuropilin-1;
g) amplifying the ALDUs bound to said immobilized neuropilin-1;
h) and optionally determining the identity of the ligand represented by said ALDU.

23. Use of a polypeptide according to claims 1 to 4 and/or a bioconjugate according to claim 5 for identifying molecules that specifically bind human neuropilin-1.

24. A method of treating or preventing metastasis in a patient, said method comprising administering to said patient a bioconjugate according to claim 5 or a ligand identifiable by the method of claims 21 or 22 in an amount effective to inhibit metastasis of neuropilin-1 mediated invasion and/or adhesion.

25. The method according to claim 24, wherein said method is used to reduce or inhibit the invasion and/or adhesion of cancer cells derived from mesodermal cells, in particular cancer cells derived from sarcomas, in particular cancer cells derived from sarcomas of the bone and/or of soft tissue.

26. The method according to claim 25, wherein the cancer cells are cells derived from the group of neoplasms consisting of malignant fibrous histiocytoma, liposarcoma, fibrosarcoma, synovial sarcoma, osteosarcoma (parosteal osteosarcoma, periosteal osteosarcoma, small-cell osteosarcoma), chondrosarcoma, Ewing's sarcoma, giant-cell tumor of bone, osteogenic sarcoma, leiomyosarcoma, rhabdomyosarcoma, mesothelioma, hemangiosarcoma, lymphangiosarcoma, myxosarcoma, endotheliosarcoma, chordoma, Kaposi's sarcoma and lymphangioendotheliosarcoma.
